(19) Europäisches Patentamt
European Patent Office
Office européen des brevets

(11) **EP 0 708 656 B1**

(12) **EUROPEAN PATENT SPECIFICATION**

(45) Date of publication and mention
of the grant of the patent:
**31.07.2002 Bulletin 2002/31**

(51) Int Cl.[7]: **A61K 38/24**, A61K 38/00,
A61K 38/04, A61K 38/16,
A61K 39/395, A61P 5/00,
A61P 15/00, A61P 35/00,
A61P 37/00

(21) Application number: **94915447.0**

(22) Date of filing: **28.04.1994**

(86) International application number:
**PCT/US94/04832**

(87) International publication number:
**WO 94/25060 (10.11.1994 Gazette 1994/25)**

(54) **IMMUNOGENIC LHRH PEPTIDE CONSTRUCTS AND SYNTHETIC UNIVERSAL IMMUNE STIMULATORS FOR VACCINES**

IMMUNOGENE LHRH PEPTIDKONSTRUKION UND SYNTHETISCHE, UNIVERSALE IMMUNSTIMULATOREN ALS IMPFSTOFFE

PEPTIDES IMMUNOGENES DE LA LHRH ET STIMULATORS D'IMMUNITE UNIVERSELLE DE SYNTHESE POUR VACCINS

(84) Designated Contracting States:
**AT BE CH DE DK ES FR GB GR IE IT LI LU MC NL PT SE**

(30) Priority: **27.04.1993 US 57166**
**14.04.1994 US 229275**

(43) Date of publication of application:
**01.05.1996 Bulletin 1996/18**

(73) Proprietor: **United Biomedical, Inc.**
**Hauppauge, New York 11788 (US)**

(72) Inventors:
• **Ladd, Anna E.**
**Brooklyn, NY 11235 (US)**
• **Wang, Chang Yi**
**Cold Spring Harbor, NY 11724 (US)**
• **Zamb, Timothy**
**Stony Brook, NY 11790 (US)**

(74) Representative: **Kolb, Helga, Dr. Dipl.-Chem.**
**Hoffmann Eitle,**
**Patent- und Rechtsanwälte,**
**Arabellastrasse 4**
**81925 München (DE)**

(56) References cited:
EP-A- 0 301 850      EP-A- 0 343 460
EP-A- 0 403 312      EP-A- 0 427 347
EP-A- 0 429 816      WO-A-92/05192
WO-A-92/13883      WO-A-92/20370
WO-A-93/03764      US-A- 4 608 251
US-A- 4 613 586

• SAD E.A.: "Bypass of carrier-induced epitope-specific suppression using a T-helper epitope" IMMUNOLOGY, vol. 76, no. 4, August 1992, pages 599-603, XP002057846
• SAD S ET AL: "SYNTHETIC GONADOTROPHIN-RELEASING HORMONE (GNRH) VACCINES INCORPORATING GNRH AND SYNTHETIC T-HELPER EPITOPES" VACCINE, vol. 11, no. 11, 1993, pages 1145-1150, XP000654065
• RANKIN E.A.: "The integrin-binding domain of invasin is sufficient to allow bacterial entry into mammalian cells" INFECTION AND IMMUNITY, vol. 60, no. 9, September 1992, WASHINGTON US, pages 3909-3912, XP002068378
• ENNIS E.A.: "Very late antigen 4-dependent adhesion and costimulation of resting human T cells by the bacterial beta1 integrin ligand invasin" J.EXP.MED., vol. 177, no. 1, 1 January 1993, pages 207-212, XP002068379

- JOURNAL OF GENERAL VIROLOGY, Volume 72, Part 6, issued June 1991, PARTIDOS et al.: "Immune Response in Mice Following Immunization with Chimeric Synthetic Peptides Representing B and T cell Epitopes of Measles Virus Proteins", pages 1293-1299, see page 1298.
- AMERICAN JOURNAL OF REPRODUCTIVE IMMUNOLOGY, Volume 22, Nos. 1-2, issued January-February 1990, LADD et al.: "Active Immunization against LHRH:1. Effects of Conjugation Site and Dose", pages 56-63, see page 56.
- METHODS IN ENZYMOLOGY, Volume 178, issued 1989, CORNETTE et al.: "Identification of T-cell Epitopes and Use in Construction of Synthetic Vaccines", pages 611-634, see pages 630-633.
- VACCINE, Volume 7, issued February 1989, WIESMULLER et al.: "Novel Low-Molecular-Weight Synthetic Vaccine Against Foot-And-Mouth Disease Contaning A Potent B-Cell and Macrophage Activator", pages 29-33, see page 29.
- PROSTATE, Volume 14, No. 1, issued 1989, JAYASHANKAR et al.: "Semisynthetic anti-LHRH Vaccine Causing Atrophy of the Prostate", pages 3-11, see page 4.
- INFECTION AND IMMUNITY, Volume 59, No. 10, issued October 1991, LEONG et al.: "Mapping and Topographic Localization of Epitopes of the Yersinia pseudotuberculosis Invasin Protein", pages 3424-3433, see entire document.

**EP 0 708 656 B1**

## Description

This invention relates to immunogenic luteinizing hormone releasing hormone (LHRH) peptides that lead to functional suppression of LHRH levels in males or females. When male rats are immunized with these peptides, serum testosterone drops and androgen-dependent organs atrophy significantly. These peptides are useful for inducing infertility and for treating prostatic hyperplasia, androgen-dependent carcinoma, prostatic carcinoma and testicular carcinoma in males. In females, the peptides are useful for treating endometriosis, benign uterine tumors, recurrent functional ovarian cysts and (severe) premenstrual syndrome as well as prevention or treatment of estrogen-dependent breast cancer. The subject peptides contain a helper T cell epitope (Th epitope) and have LHRH at the C terminus. The helper T cell epitope aids in stimulating the immune response against LHRH. The peptides, optionally, contain an invasin domain which acts as a general immune stimulator.

In another aspect this invention relates to immunogenic synthetic peptides having an invasin domain, a helper T cell epitope and a peptide hapten and methods of using these peptides to treat disease or provide protective immunity. The peptide haptens of the invention is LHRH.

Prostate cancer is the third leading cause of death in men and the most common malignancy in men over the age of 70 years. The number of new prostate cancer cases has risen steadily over the past 20 years, with the expectation that more than 4 million men over the age of 75 may develop clinically detectable prostate cancer in the early 21st century [Perez *et al.* (1985) in Cancer Principles and Practice of Oncology, Vol. 9 (DeVita *et al.,* eds.) J.B. Lippincott Company, Philadelphia, PA, pp. 1023-48; Chodak *et al.* (1990) Current Concepts in Prostate Cancer Diagnosis and Management, 26th Annual Meeting, American Society of Clinical Oncology. Unfortunately, at the time of diagnosis about 40-50% of the patients with newly diagnosed prostate cancer will have advanced disease (stage D), with a median survival time of approximately 2.4 years [Torty (1988) Adv. Onc. 4:15]. Consequently, the therapies developed to combat this disease should demonstrate efficacy as rapidly as possible.

The classical treatment for advanced prostate cancer has been surgical orchiectomy, i.e. castration, developed by Huggins and others in the early 1940s [Huggins *et al.* (1941) Cancer Res. 1:293-297]. This procedure reduces serum testosterone by 95%, causes measurable tumor regression in approximately 45% of patients, and disease stabilization in an additional 40% of patients. At least temporary stabilization of advanced prostatic disease, including improvement of urinary tract symptoms and reduction of pain, occurs in about 70% of patients [Klein (1979) N. Engl. J. Med. 300:824-33]. While such treatments are effective, particularly when combined with estrogen therapy, the associated psychological trauma is unacceptable to some patients.

Over 95% of testosterone production originates in the testes. Testosterone production in the Leydig cells of the testes is controlled by pituitary secretion of luteinizing hormone (LH). The secretion of LH together with follicle stimulating hormone (FSH), in turn is controlled by the pulsatile release of LHRH from the hypothalamus [See, for example, Paulsen (1974) in Textbook of Endocrinology (Williams, ed.) Saunders, Philadelphia, PA, pp323-367]. Attempts to block LHRH, to reduce testosterone effect on androgen-dependent organs, e.g. prostate, or to block other parts of this pathway have provided therapeutic alternative treatments for prostate cancer, including treatment with estrogens or LHRH analogs. Unfortunately, therapeutic doses of estrogens can cause significant side effects such as cardiovascular mortality, gynecomastia, nausea, sodium retention, and impotence [Blackard (1975) Can. Chem. Rep. 59:225-7]. Treatment with LHRH analogs, such as Leuprolide or goserelin, causes eventual decline of serum testosterone; however, the associated initial rise of serum LH and FSH levels (450 and 250 per cent, respectively), leads to a painful condition known as the "flare up phenomena" in which a temporary increase in serum testosterone and other symptoms occur [Crawford *et al.* (1991) Urol. Clin. N.A. 18:55-63]. In addition LHRH analog therapy can cause gastrointestinal upset and hot flushes.

Active immunization against LHRH has long been known to exert multiple effects, including decreasing serum and pituitary LH and FSH, reducing serum testosterone, suppressing spermatogenesis and causing reversible atrophy of the gonads and accessory sex organs. [See, for example, Fraser *et al.* (1974) J. Endocrinol. 63:399-405; Giri *et al.* (1991) Exp. Molec. Pathol. 54:255-264; Ladd *et al.* (1989) J. Reprod. Immunol. 15:85-101; and references cited therein].

Immune intervention of the androgen hormone cascade can also be used in the treatment of endometriosis in women. This disease is the second leading cause of infertility in females after infection-induced infertility. The ectopic development and maintenance of endometrial tissues outside the uterine musculature is mediated by estrogen. Since LHRH regulates the production of FSH by the anterior pituitary which in turn regulates the production of estrogen by the ovaries, blocking the action of LHRH is another therapy for this disease. Thus by analogy to prostate cancer, estrogen-driven tumors of the breast should also be responsive to LHRH immunotherapy.

In addition to providing treatment for a number of important diseases in both men and women, regulation of the androgen hormone cascade through immunologic intervention provides a means of regulating fertility in both sexes. Since LHRH controls both testosterone production, which regulates the development of sperm, and estrogen production, which causes the ripening of ova, immunological blocking of LHRH action results in reversible infertility. Moreover, LHRH-based immunotherapy provides a means for reversible contraception in male and female companion animals (e.g. dogs, cats, horses and rabbits) as well as mitigating undesirable androgen-driven behavior such as heat, territorial marking and aggression. Lastly, immunological castration (e.g. antibody-based inhibition of LHRH action) has application in the meat animal industry. Males are not processed into prime cuts of meat because of the offensive aroma and taste associated with their flesh as a result of circulating testosterone (e.g. boar taint). Since mechanical castration of male food animals is no longer considered humane, immunological castration provides an acceptable alternative to this practice.

Several immunogenic forms of LHRH have been tested. For example, LHRH has been combined with adjuvants or conjugated with protein to enhance immunopotency. However, these adjuvants have been unsuitable for human use, and protein carriers are too expensive for large scale use. Further, effective immunization with LHRH depends on the conjugation site between LHRH and the carrier. Conjugation of the carrier protein (diphtheria toxin or tetanus toxoid) to the amino terminus of LHRH provided a more effective vaccine for immunization and contraception relative to formulations having the carrier protein at other conjugation sites on LHRH [Ladd *et al.* (1990) Am. J. Reprod. Immunol. 22:56-63].

Moreover, protein linkage to LHRH is problematic because the majority of immune responses are directed to the carrier rather than to LHRH (the mass of the toxin molecule(s) is much greater than that of LHRH). This phenomenon leads to carrier-induced immune suppression. Because the majority of cancer or endometriosis patients have been previously immunized with diphtheria and tetanus vaccines as part of mandatory immunization programs, antibody and/or suppressor T cell responses directed to tetanus or diphtheria toxin components of the vaccines can interfere with the subsequent immune responses to toxin-linked LHRH immunogens.

Accordingly, an immune enhancer that is suitable for human use, inexpensive and capable of stimulating an early and strong immune response to LHRH has been sought. Likewise this immune enhancer should avoid carrier-induced suppression. Hence, it has been found that peptides containing particular structural arrangements of a Th epitope alone or linked to an invasin domain (as an immune enhancer) and LHRH (as immunogen) are effective in stimulating the production of antibodies against LHRH.

The present invention relates to peptides, preferably synthetic peptides, which are capable of inducing antibodies against LHRH that lead to the suppression of LHRH levels in males or females. The subject peptides are useful for inducing infertility and for treating prostatic hyperplasia, androgen-dependent carcinoma, prostatic carcinoma, testicular carcinoma, endometriosis, benign uterine tumors, recurrent functional ovarian cysts (severe) premenstrual syndrome or for prevention or treating estrogen-dependent breast cancer. Peptides of this invention have a Th epitope and carboxyl-terminal LHRH, or a peptide analog of LHRH. These peptides are effective as immunogens and therapeutics. The peptides of this invention are capable of reducing serum testosterone to levels comparable to those obtained by orchiectomy (castration) and of causing reversible atrophy of the testes, prostate and other androgen- or estrogen-dependent sex organs. Optionally, the peptides have an invasin domain as an immune stimulator.

Another aspect of this invention provides a vaccine composition comprising an immunologically effective amount of a peptide in accordance with this invention and one or more pharmaceutically acceptable carriers. Such vaccine compositions are useful in the induction of infertility or the treatment of prostatic hyperplasia, androgen-dependent carcinoma, prostatic carcinoma, testicular carcinoma, endometriosis, benign uterine tumors, recurrent functional ovarian cysts and/or (severe) premenstrual syndrome as well as for prevention or treatment of estrogen-dependent breast cancer.

A further aspect of the invention relates to a method for suppressing activity of circulating LHRH levels in a mammal by administering one or more of the subject peptides to the mammal for a time and under conditions sufficient to induce functional antibodies directed against said LHRH. Suppression of LHRH activity is useful to treat prostatic hyperplasia, androgen-dependent carcinoma, prostatic carcinoma, testicular carcinoma, endometriosis, benign uterine tumors, recurrent functional ovarian cysts or. (severe) premenstrual syndrome, or to prevent or treat estrogen-dependent breast cancer. More particularly, the invention provides a method for inducing infertility in a mammal by administering the subject vaccine compositions to the mammal for a time and under conditions to produce an infertile state in the mammal. Similarly, this invention relates to a method for treating androgen-dependent carcinoma by administering the subject vaccine compositions to the mammal for a time and under conditions to effect regression or prevent growth of the carcinoma.

Yet another aspect of the invention relates to an immunogenic synthetic peptide of about 30 to about 90 amino acids which contains an immunostimulatory invasin domain, a helper T cell (Th) epitope and a peptide hapten. LHRH, Further these peptides have one or more amino terminal $(A)_n$ groups, where A is an amino acid, $\alpha$-$NH_2$, and n is from 1 to about 10. The three elements of the subject peptides can be separated by a $(B)_o$ spacer group, where B is independently any amino acid and o is from 0 to about 10.

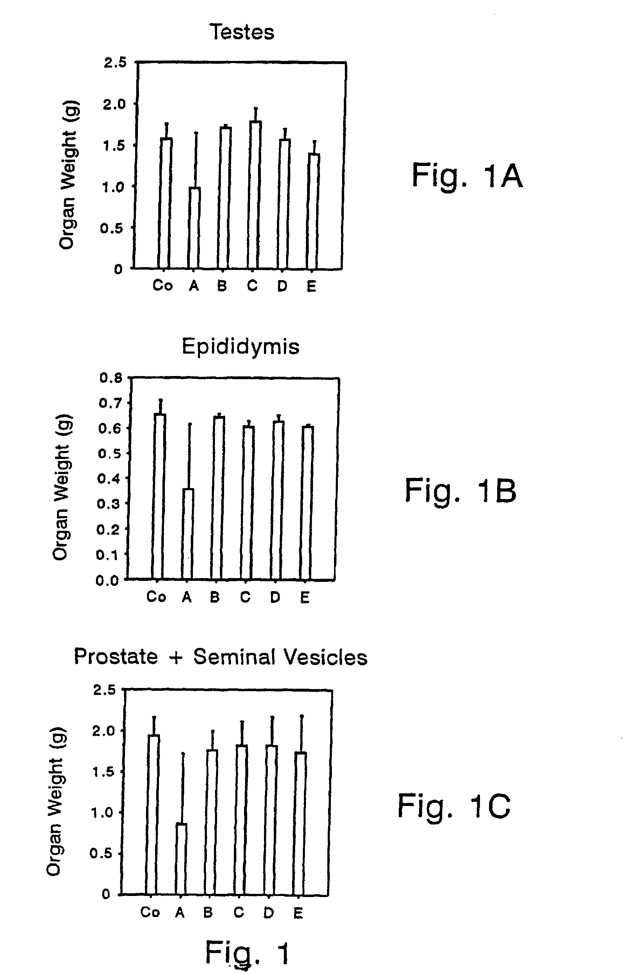

Fig. 1 graphically illustrates the average androgen-dependent organ weights (g) obtained 8 or 11 weeks after immunization of rats (n=5) with Peptides A-E. Panel A provides testes weight; Panel B provides epididymis weight; Panel C provides prostate plus associated seminal vesicles weight. Organ weights were obtained at 11 weeks for Peptides A-C and at 8 weeks for Peptides D and E. The average weight of the organs in control animals (n=8) is indicated by "Co".

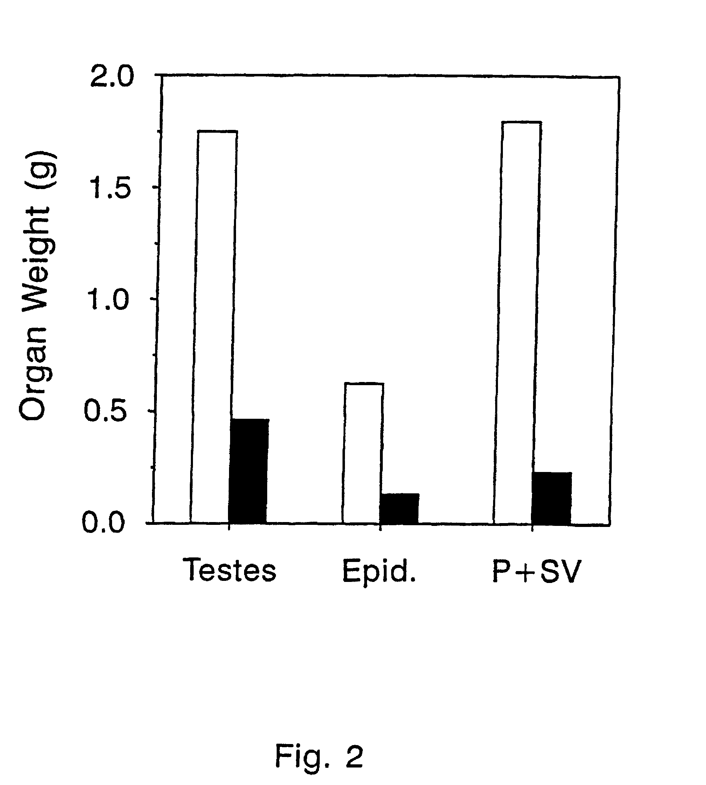

Fig. 2 shows the relative androgen-dependent organ weights (g) in the responder (solid bars) and non-responder (open bars) animals immunized with Peptide A. Abbreviations: Epid., epididymis; P+SV, prostate and seminal vesicles.

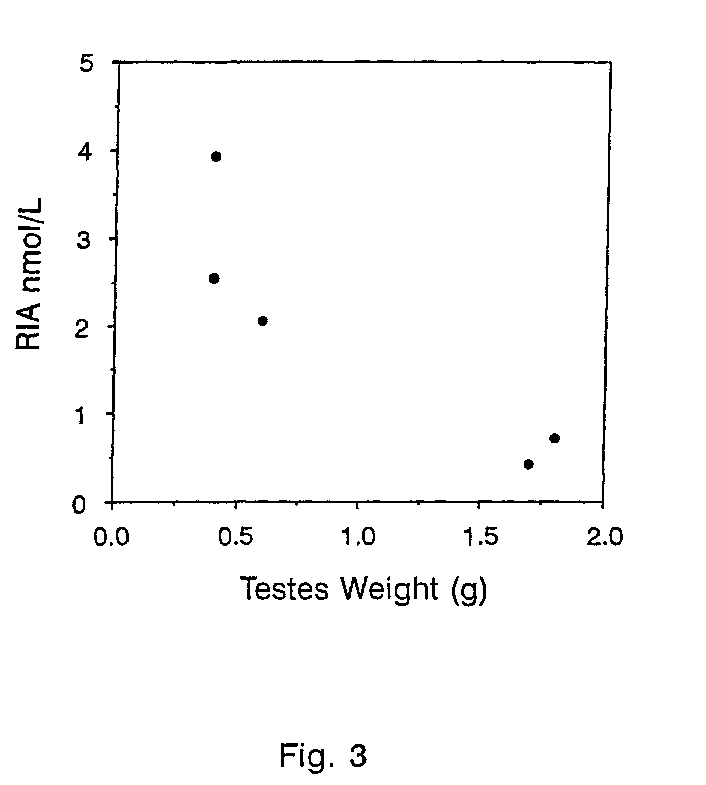

Fig. 3 graphically depicts the correlation between testes weight (g) and serum anti-LHRH antibody levels (nmole/L) as determined in a radioimmunoassay (RIA) after immunization with Peptide A.

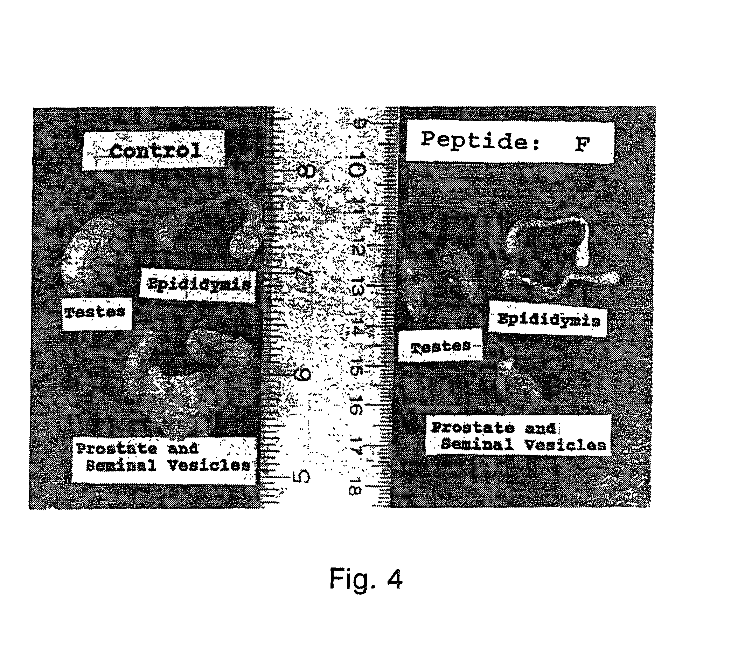

Fig. 4 is a photograph illustrating the size of androgen-dependent organs in controls or animals treated with a Peptide F.

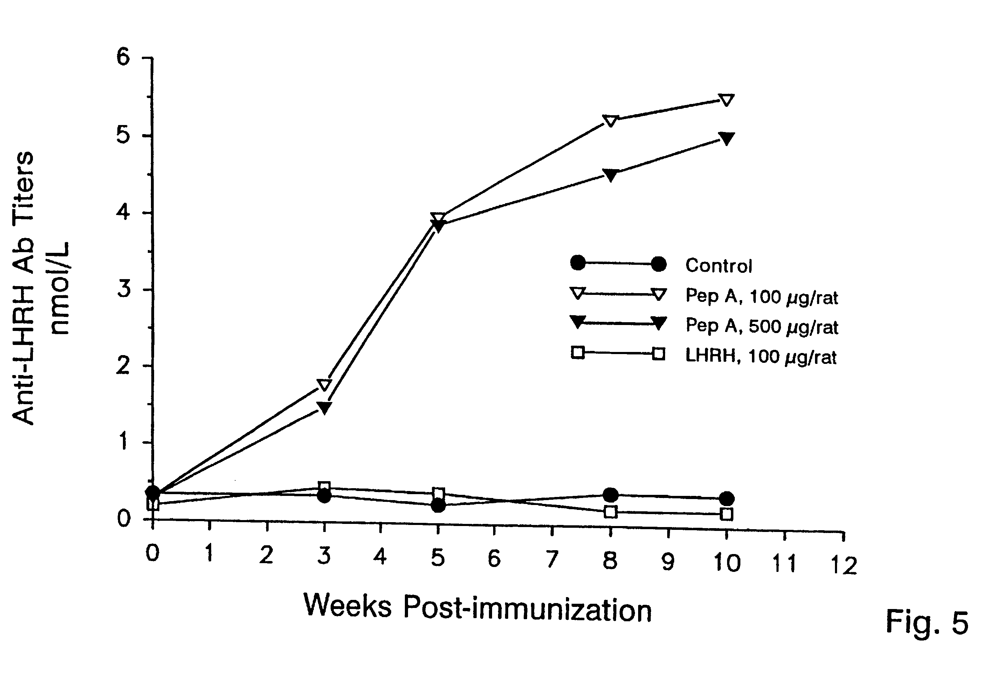

Fig. 5 graphically depicts levels of anti-LHRH specific antibody produced in rats following immunization with an immunogenic LHRH construct designated as HBSAg $T_h$: LHRH (peptide A). Eight sexually mature Sprague-Dawley male rats per group were given 100 μg or 500 μg of peptide A by intramuscular administration. The antigen was formulated in Freund's complete adjuvant and given at week 0, and in incomplete Freund's adjuvant and administered at weeks 3 and 6. LHRH-specific antibody as reported in this and subsequent figures was determined by standard radioimmunoassay and expressed as the mean value in nanomoles of total LHRH antibody per liter of serum. The control group was given unmodified LHRH in Freund's adjuvant using the same immunization schedule.

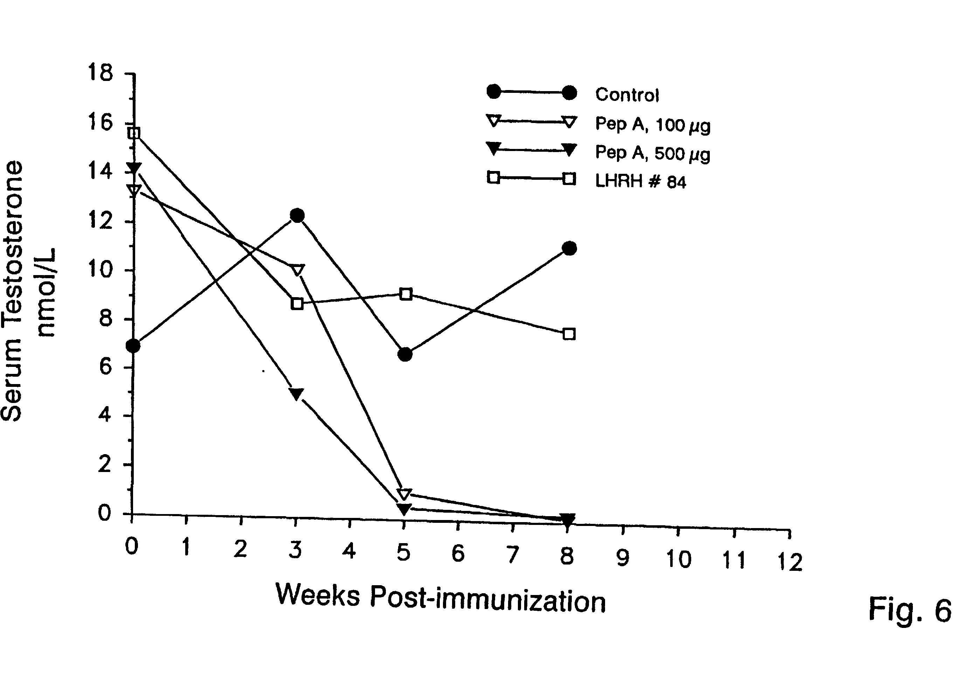

Fig. 6 graphically depicts serum testosterone levels in rats following administration of peptide A as described in Fig. 5. Testosterone as reported in this and subsequent figures was measured in the serum samples used for determining the LHRH-specific antibody titers. Serum testosterone was measured by radioimmunoassay, and expressed as the mean value in nanomoles of testosterone per liter of serum.

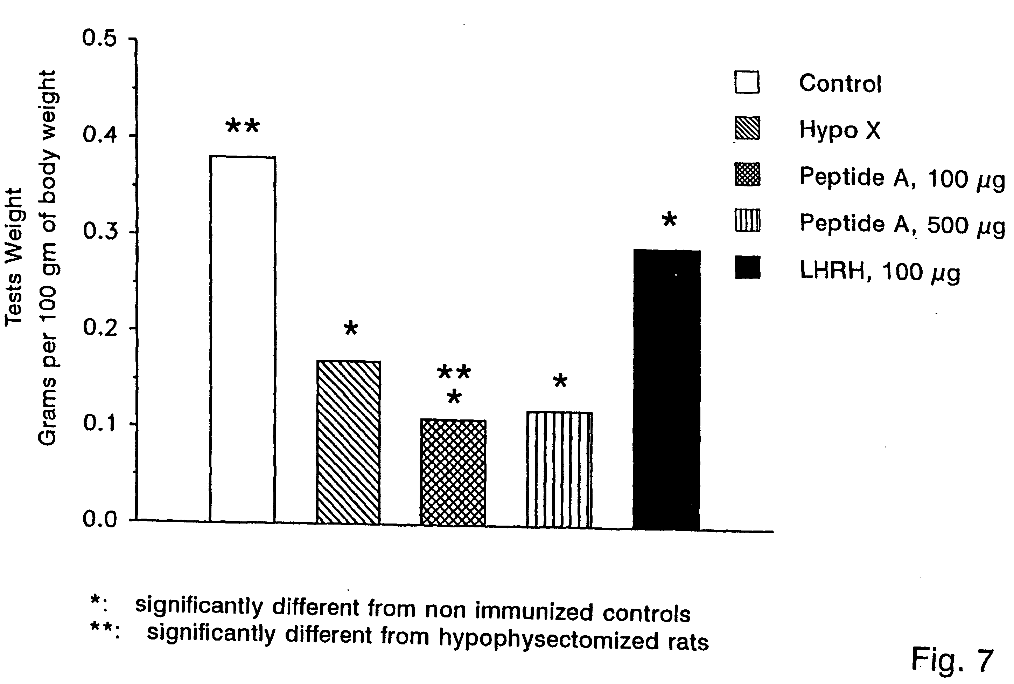

Fig. 7 graphically depicts testis weights of animals given peptide A as described in Fig. 5. At 11 weeks following the commencement of the experiment described in the legend to Fig. 5, animals were sacrificed and the relevant organs dissected and weighed. Testis weights are expressed as the mean value in grams of organ weight per 100 grams of body weight. HypoX designates hypophysectomized rats. Group 1 animals were immunized with Freund's adjuvant without antigen, using an identical schedule to the experimental groups.

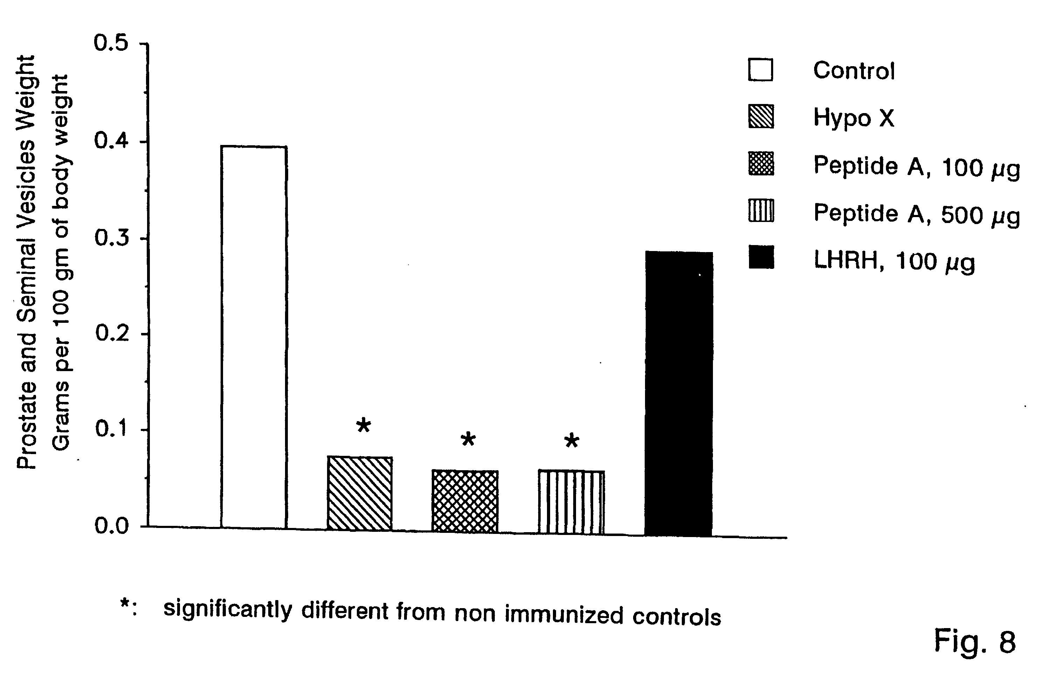

Fig. 8 graphically depicts prostate and seminal vesicle weights of animals given peptide A as described in Fig. 5. Prostate and seminal vesicles were weighed together and their collective weight expressed as the mean value in grams of tissue per 100 grams of body weight. HypoX designates hypophysectomized rats. Group 1 animals were immunized with Freund's adjuvant without antigen, using an identical schedule to the experimental groups.

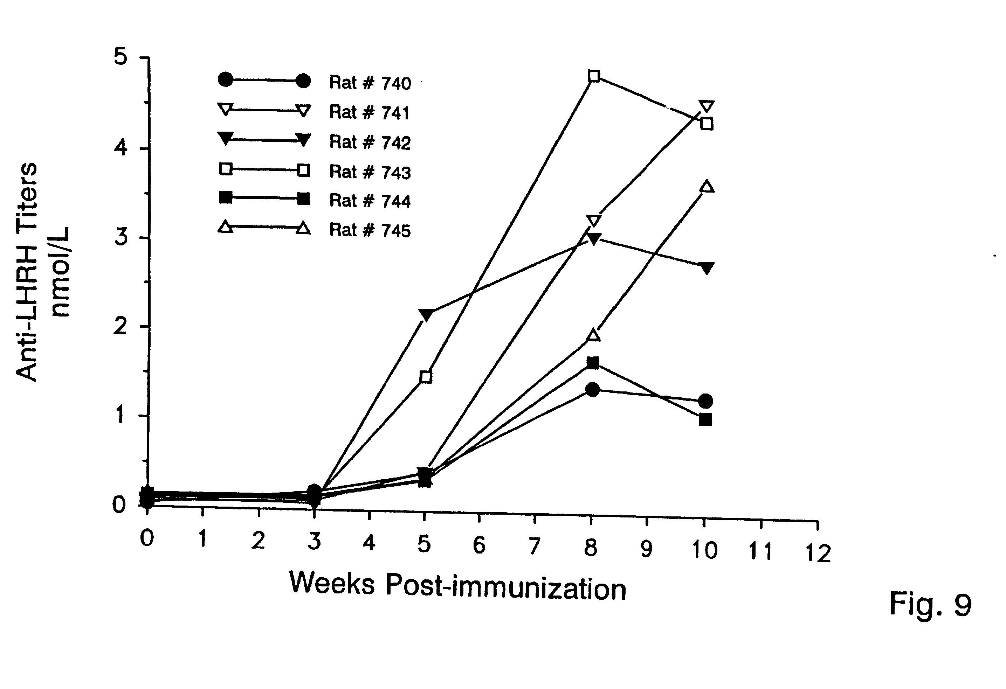

Fig. 9 graphically depicts levels of anti-LHRH specific antibody produced in rats following immunization with an immunogenic LHRH construct designated as HBSAg $T_h$: GG : LHRH (peptide 18). Six sexually mature Sprague-Dawley male rats per group were given 100 μg of peptide 18 by subcutaneous administration. The antigen was formulated in Freund's complete adjuvant and given at week 0, and in incomplete Freund's adjuvant and administered at weeks 3 and 6. The control group was given unmodified LHRH in Freund's adjuvant using the same immunization schedule.

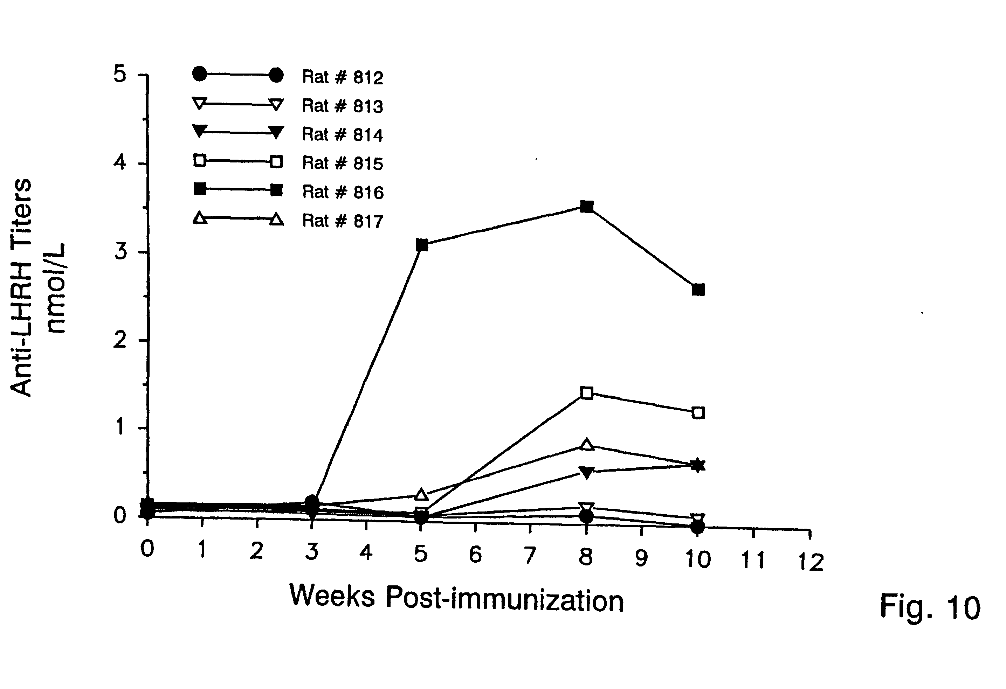

Fig. 10 graphically depicts levels of anti-LHRH specific antibody produced in rats following immunization with HBsAg $T_h$: LHRH (peptide A). Six sexually mature Sprague-Dawley male rats per group were given 100μg peptide A by subcutaneous administration. The antigen was formulated in Freund's complete adjuvant and given at week 0, and in incomplete Freund's adjuvant and administered at weeks 3 and 6. The control group was given unmodified LHRH in Freund's adjuvant using the same immunization schedule.

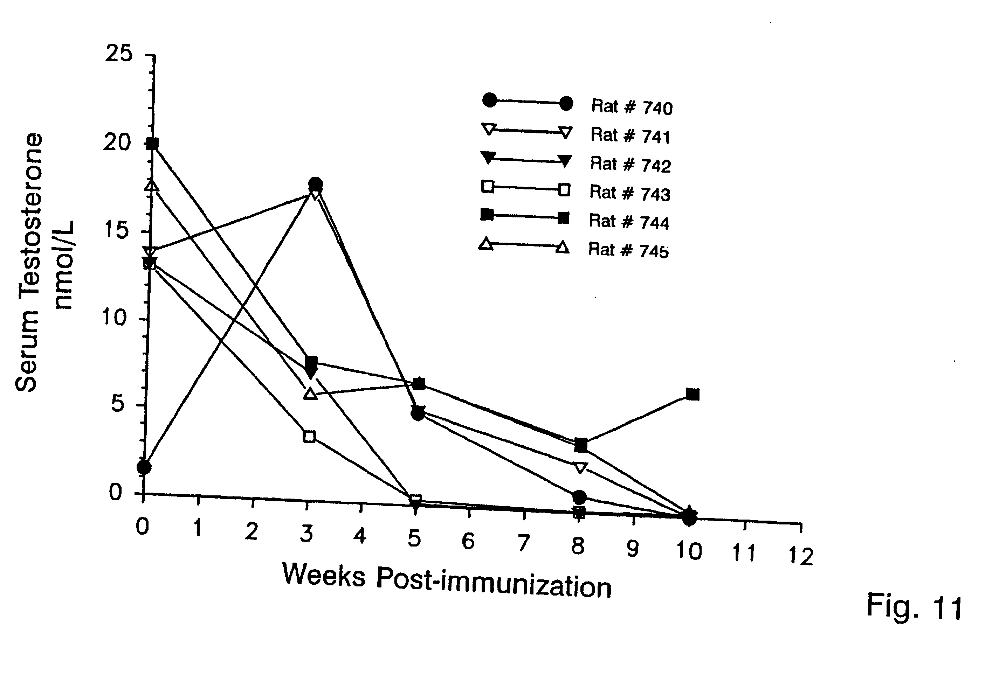

Fig. 11 graphically depicts serum testosterone levels in rats following administration of peptide 18 in Freund's adjuvant. The experimental design is that described in the legend to Fig. 9.

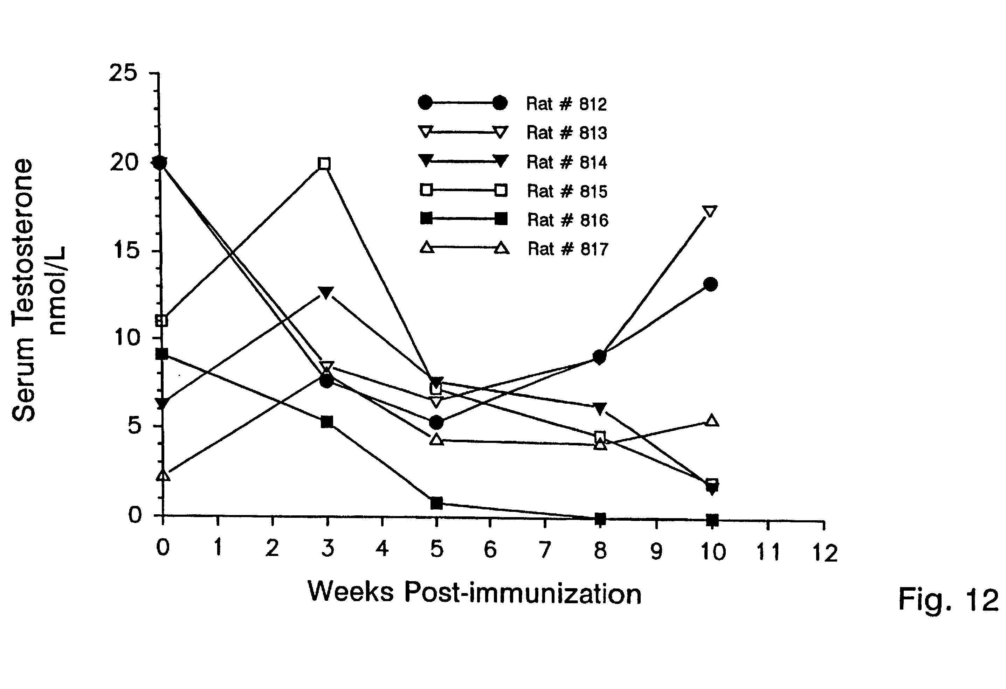

Fig. 12 graphically depicts serum testosterone levels in rats following administration of peptide A. The experimental design is that described in the legend to Fig. 10.

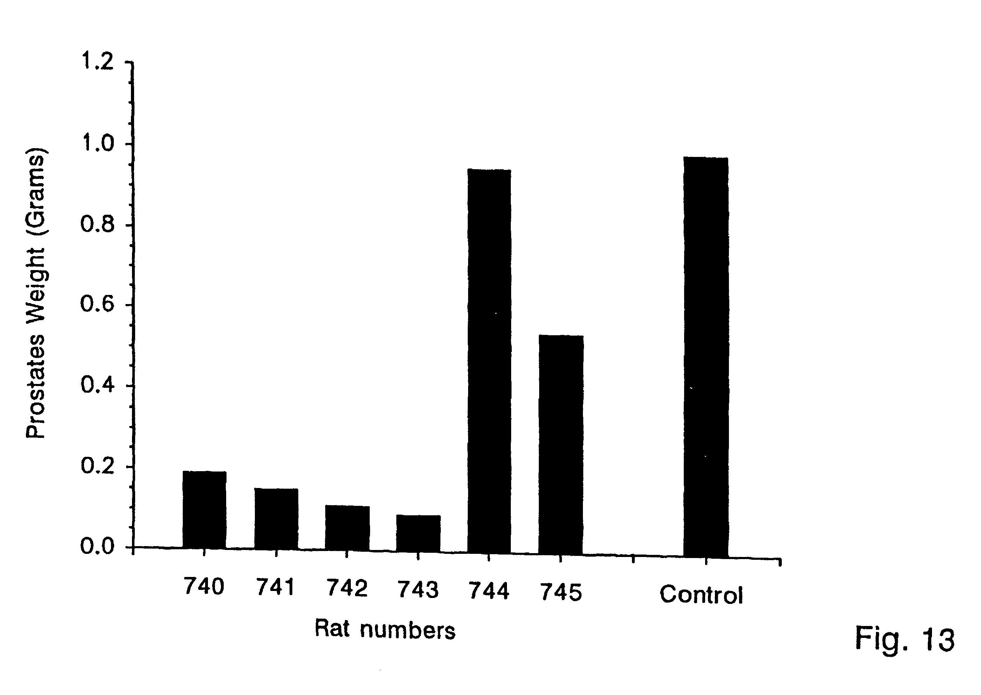

Fig. 13 graphically depicts prostate and seminal vesicle weights of animals given peptide 18. The experimental protocol is described in the legend to Fig. 9. Prostate and seminal vesicles were weighed together and their collective weight expressed as the mean value in grams of tissue per 100 grams of body weight. Control animals were immunized with Freund's adjuvant without antigen, using an identical schedule to the experimental groups.

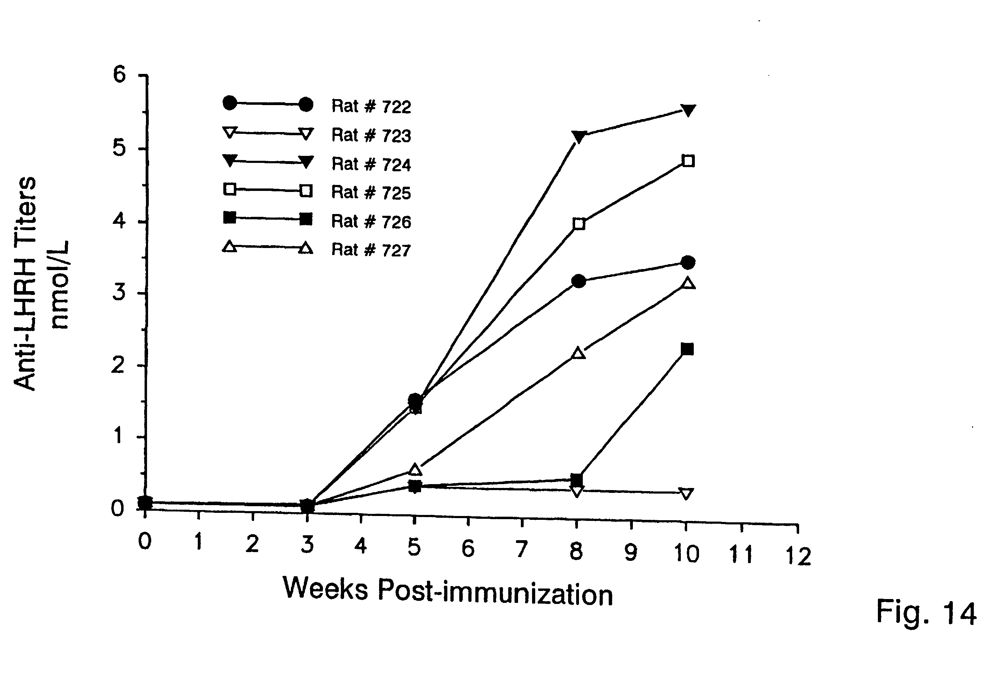

Fig. 14 graphically depicts levels of anti-LHRH specific antibody produced in rats following immunization with MV F $T_h$: LHRH (peptide 19). Peptide 19 consists of a segment of the F protein from measles virus linked to the amino terminus of LHRH. Six sexually mature Sprague-Dawley male rats per group were given peptide 19 equivalent to 100 μg of peptide A by subcutaneous administration. The antigen was formulated in Freund's complete adjuvant and given at week 0, and in incomplete Freund's adjuvant and administered at weeks 3 and 6. The control group was given unmodified LHRH in Freund's adjuvant using the same immunization schedule.

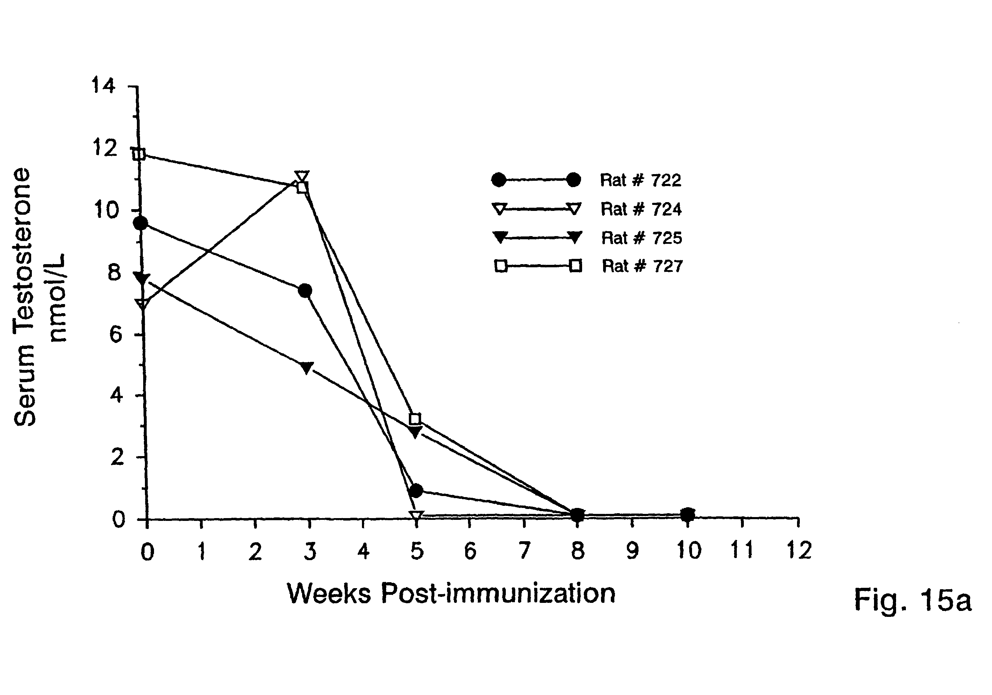

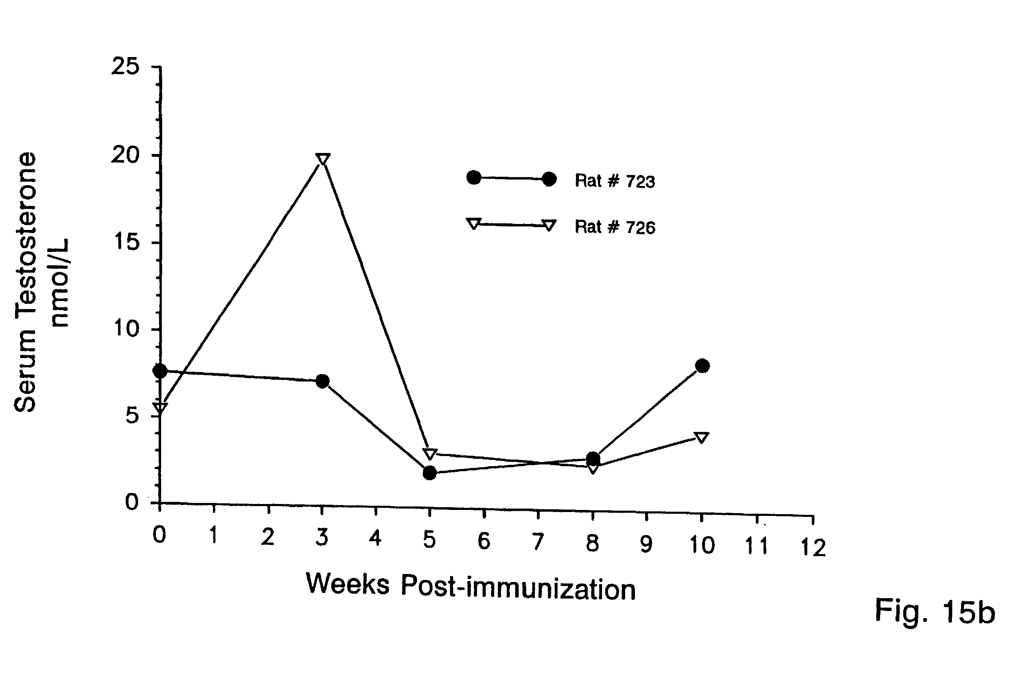

Fig. 15 graphically depicts serum testosterone levels in rats following administration of peptide 19. The experimental design is that described in the legend to Fig. 14. Panel A shows data for animals which achieved serum testosterone levels below the castration threshold, whereas Panel B shows data for animals which did not achieve castration levels of testosterone by week 8.

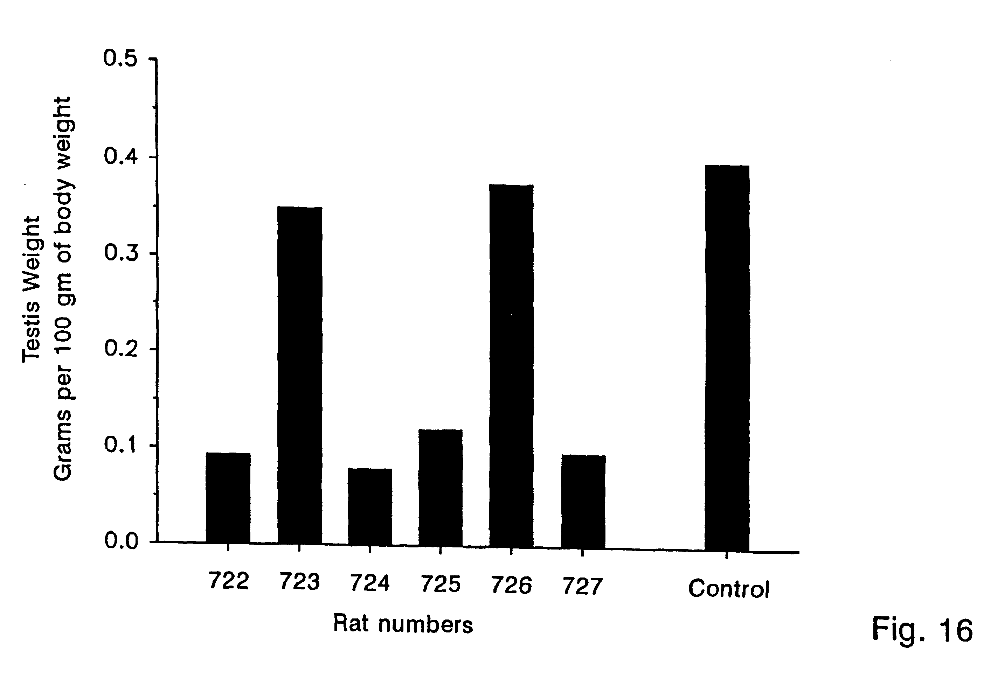

Fig. 16 graphically depicts testis weights of animals given peptide 19. At 10 weeks following the commencement of the experiment described in the legend to Fig. 14, animals were sacrificed and the relevant organs dissected and weighed. Testis weights are expressed as the mean value in grams of organ weight per 100 grams of body weight. Control animals were immunized with Freund's adjuvant without antigen, using an identical schedule to the experimental groups.

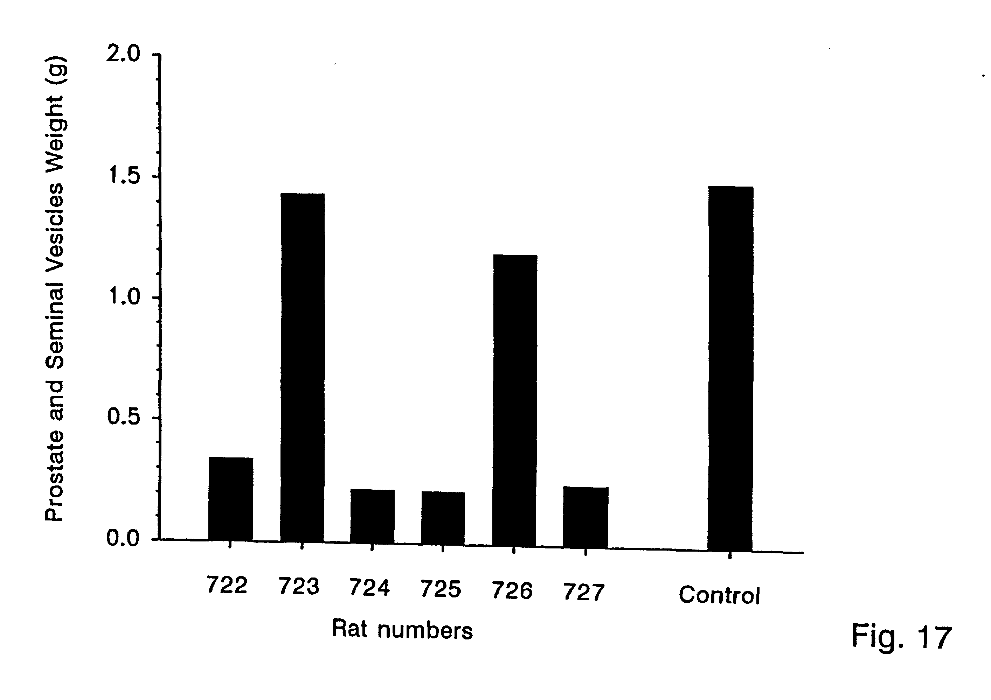

Fig. 17 graphically depicts prostate and seminal vesicle weights of animals given peptide 19. The experimental protocol is described in the legend to Fig. 14. Prostate and seminal vesicles were weighed together and their collective weight expressed as the mean value in grams of tissue per 100 grams of body weight. Control animals were immunized with Freund's adjuvant without antigen, using an identical schedule to the experimental groups.

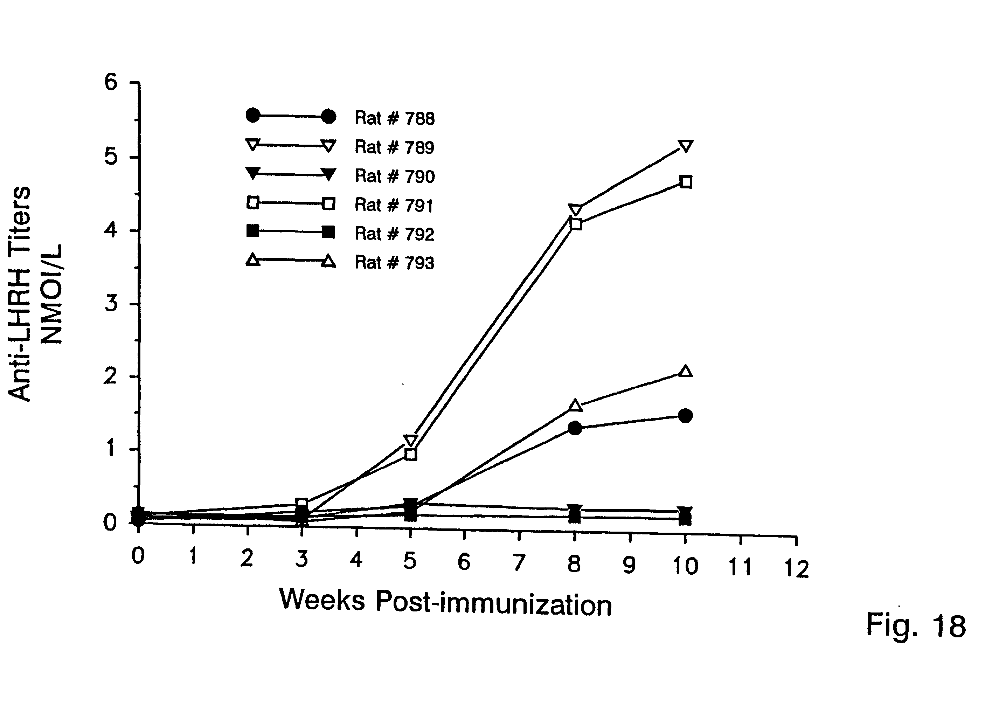

Fig. 18 graphically depicts anti-LHRH specific antibody produced in rats following immunization with PT $T_h2$: LHRH (peptide K, Seq ID No:16). Peptide K consists of a segment of pertussis toxin linked to the amino terminus of LHRH. Six sexually mature Sprague-Dawley male rats per group were given peptide K equivalent to 100 μg of peptide A by subcutaneous administration. The antigen was formulated in Freund's complete adjuvant and given at week 0, and in incomplete Freund's adjuvant and administered at weeks 3 and 6. The control group was given unmodified LHRH in Freund's adjuvant using the same immunization schedule.

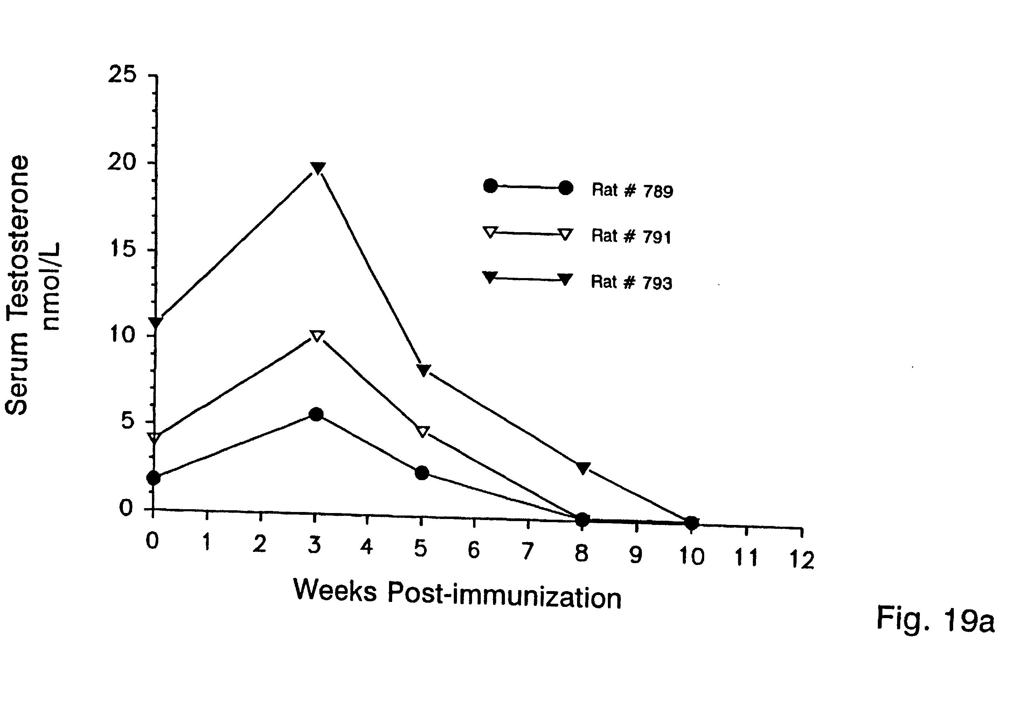

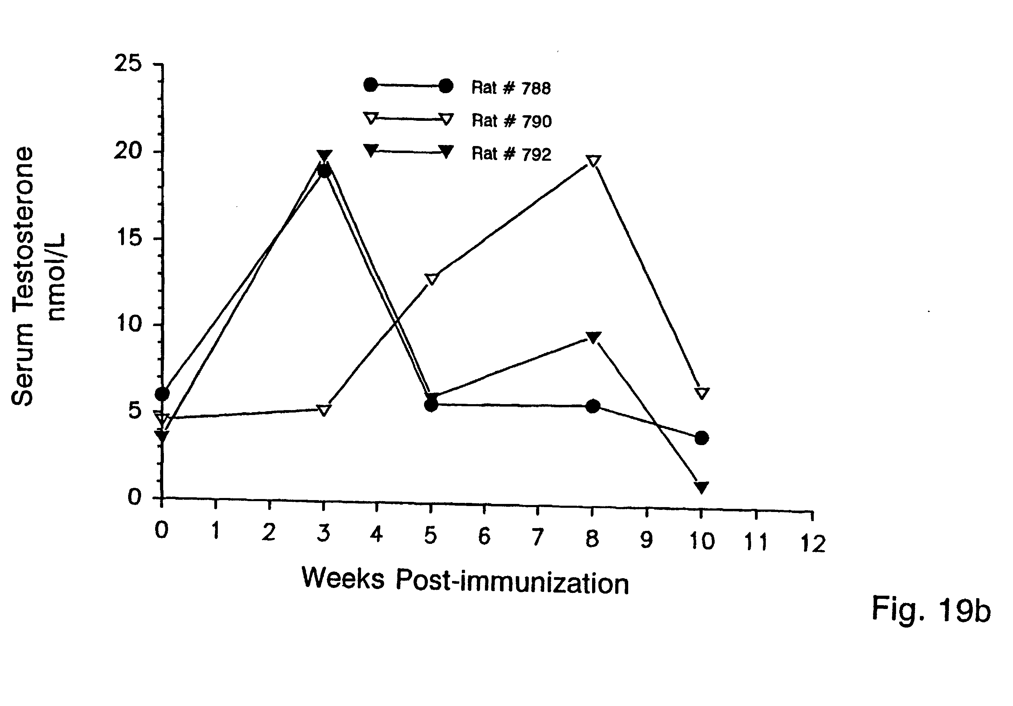

Fig. 19 graphically depicts serum testosterone levels in rats following administration of peptide K. The experimental design is that described in the legend to Fig. 18. Panel A shows data for animals which achieved serum testosterone levels below the castration threshold, whereas Panel B shows data for animals which did not achieve castration levels of testosterone by week 8.

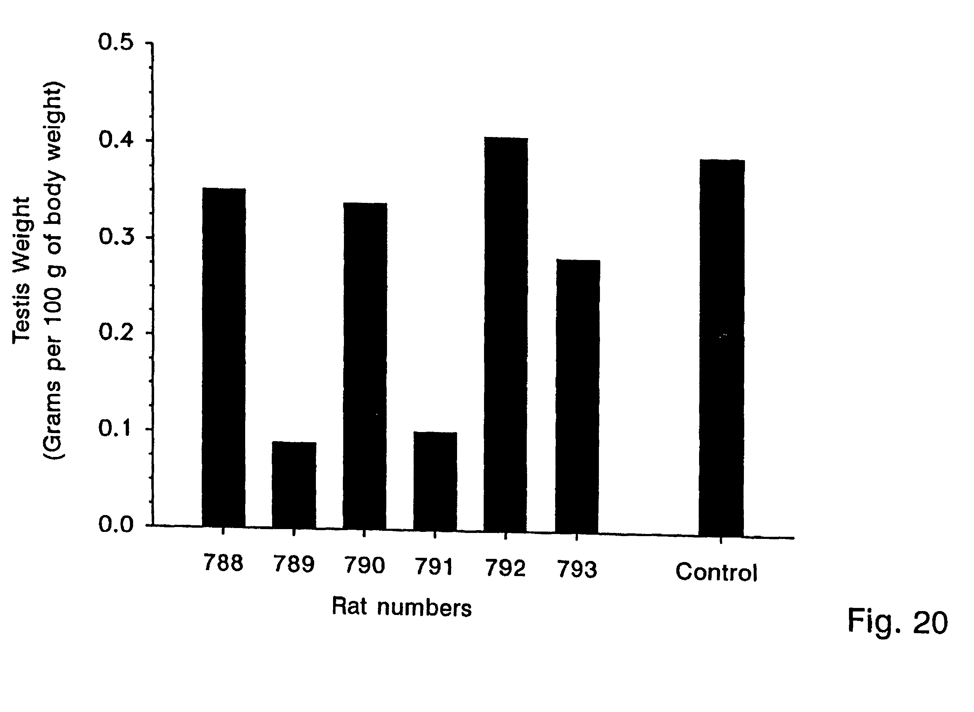

Fig. 20 graphically depicts testis weights of animals given peptide K. At 10 weeks following the commencement of the experiment described in the legend to Fig. 18, animals were sacrificed and the relevant organs dissected and weighed. Testis weights are expressed as the mean value in grams of organ weight per 100 grams of body weight. Control animals were immunized with Freund's adjuvant without antigen, using an identical schedule to the experimental groups.

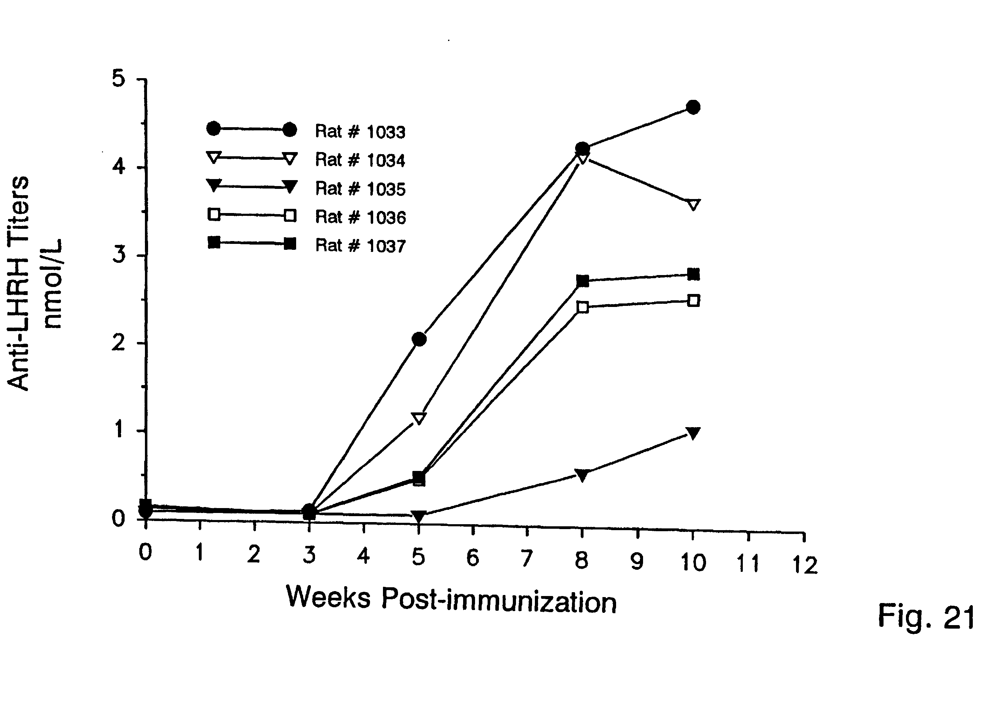

Fig. 21 graphically depicts levels of anti-LHRH specific antibody produced in rats following immunization with an immunogenic LHRH construct designated as TT $T_h1$ : LHRH (peptide H). Five sexually mature Sprague-Dawley male rats per group were given 100 μg of peptide H by subcutaneous administration. The antigen was formulated in Freund's complete adjuvant and given at week 0, and in incomplete Freund's adjuvant and administered at weeks 3 and 6. The control group was given unmodified LHRH on alum using the same immunization schedule.

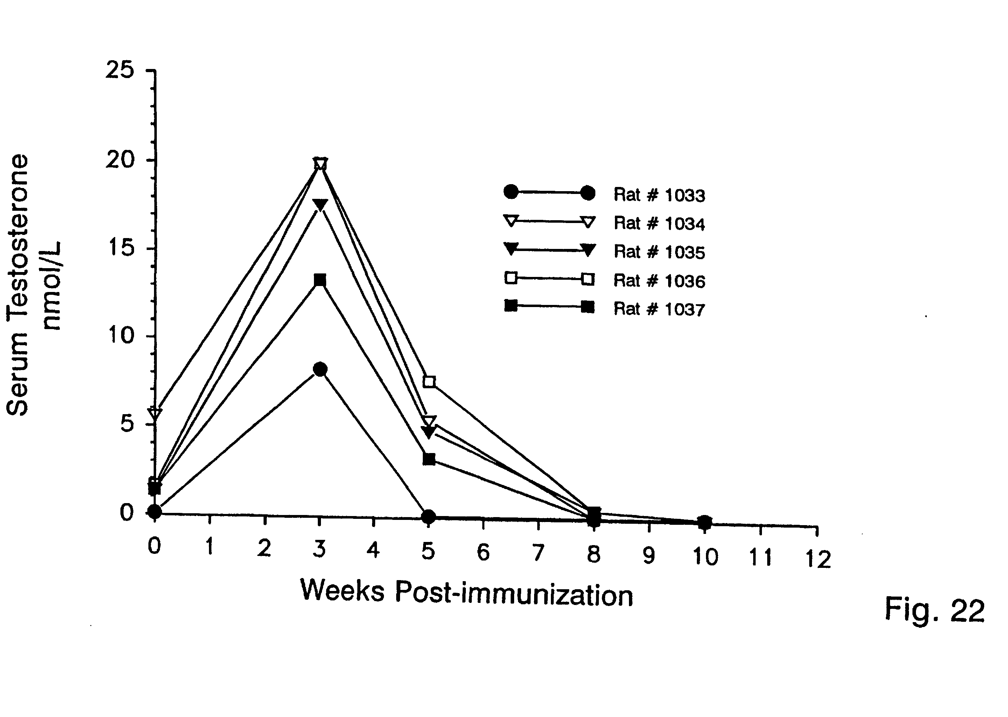

Fig. 22 graphically depicts serum testosterone levels in rats following administration of peptide H. The experimental design is that described in the legend to Fig. 21.

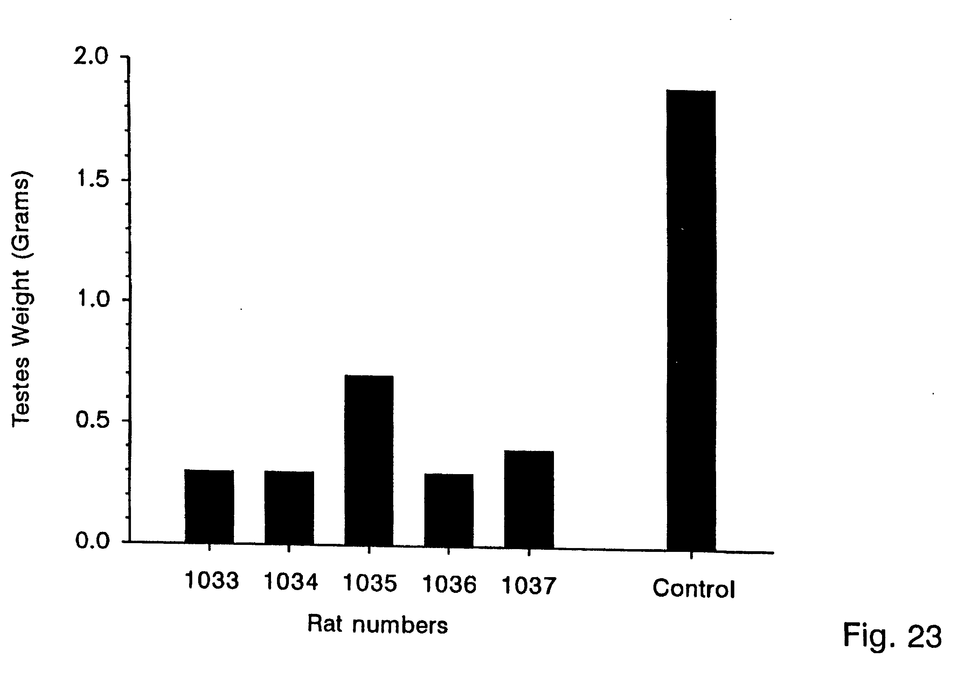

Fig. 23 graphically depicts testis weights of animals given peptide H. At 10 weeks following the commencement of the experiment described in the legend to Fig. 21, animals were sacrificed and the relevant organs dissected and weighed. Testis weights are expressed as the mean value in grams of organ weight per 100 grams of body weight. Control animals were immunized with alum adjuvant without antigen, using an identical schedule to the experimental groups.

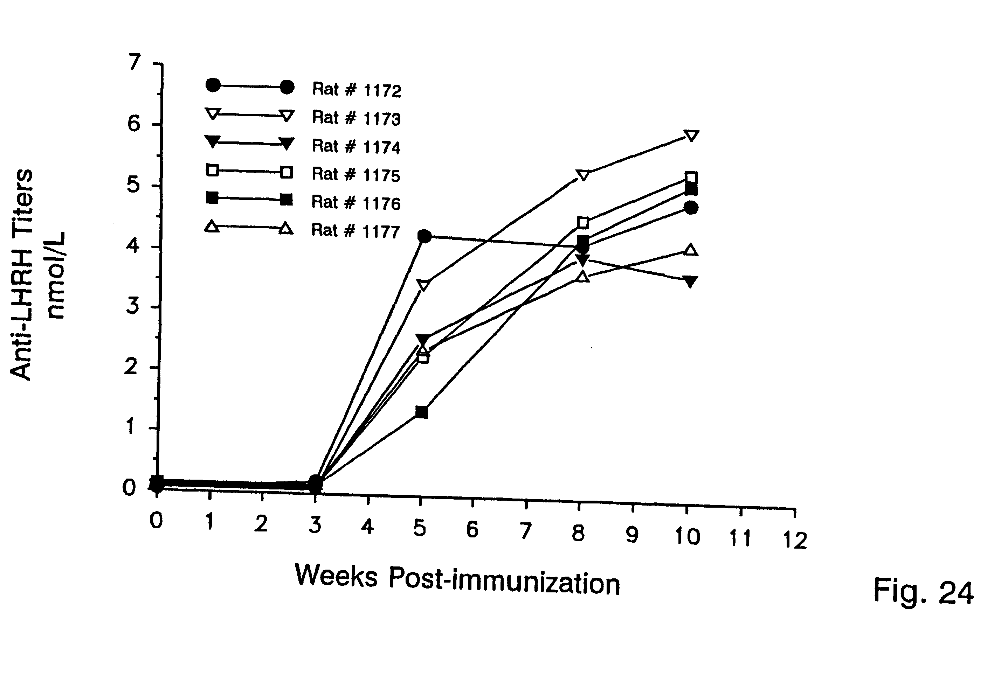

Fig. 24 graphically depicts levels of anti-LHRH specific antibody produced by immunization with a prototype immunogen cocktail formulated with Freund's adjuvant. Equimolar amounts of HBsAg$T_h$: LHRH + MV F $T_h$:LHRH + PT $T_h$:LHRH + TT $T_h$:LHRH were mixed and formulated in Freund's adjuvant. Six sexually mature Sprague-Dawley male rats were given a molar equivalent of the immunogen cocktail equal to 100 μg of peptide A in Freund's complete adjuvant at week 0 and in Freund's incomplete adjuvant at weeks 3 and 6. All immunizations were via the subcutaneous route.

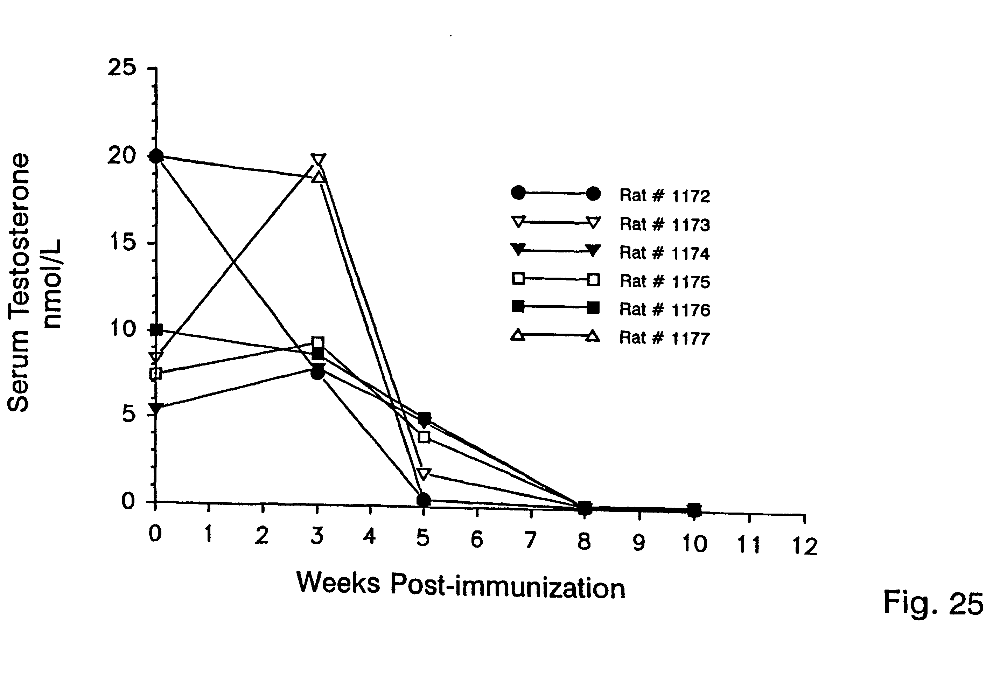

Fig. 25 graphically depicts serum testosterone levels in rats following administration of the prototype immunogen cocktail in Freund's adjuvant. The experimental design is that described in the legend to Fig. 24.

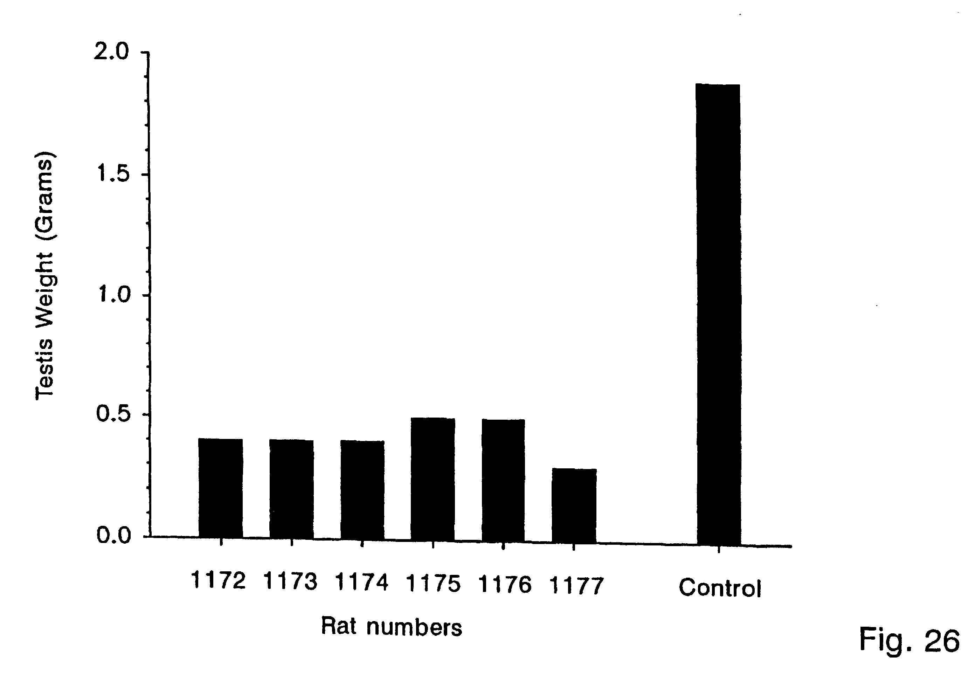

Fig. 26 graphically depicts testis weights of animals given the prototype immunogen cocktail in Freund's adjuvant. At 10 weeks following the commencement of the experiment described in the legend to Fig. 24, animals were sacrificed and the relevant organs dissected and weighed. Testis weights are expressed as the mean value in grams of organ weight per 100 grams of body weight. Control animals were immunized with Freund's adjuvant without antigen, using an identical schedule to the experimental groups.

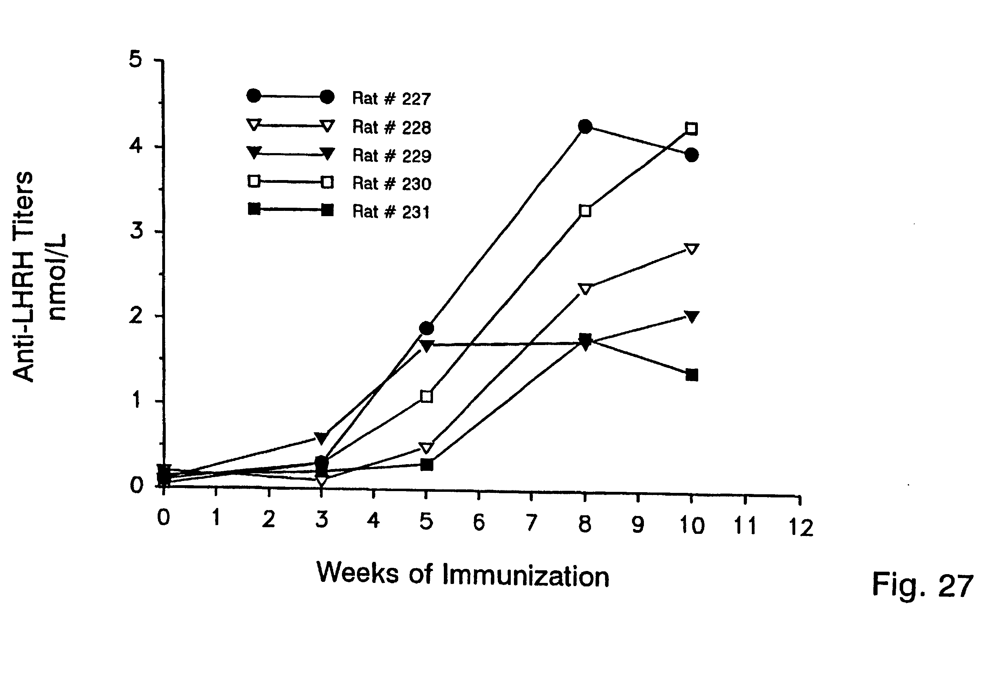

Fig. 27 graphically depicts levels of anti-LHRH specific antibody produced by immunization with a prototype immunogen cocktail. Equimolar amounts of HBsAg$T_h$: LHRH + MV F $T_h$:LHRH + PT $T_h$:LHRH + TT $T_h$:LHRH were mixed and formulated on alum. Six sexually mature Sprague-Dawley male rats per group were given a molar equivalent of the immunogen cocktail equal to 100 μg of peptide A by intramuscular administration at weeks 0, 3 and 6.

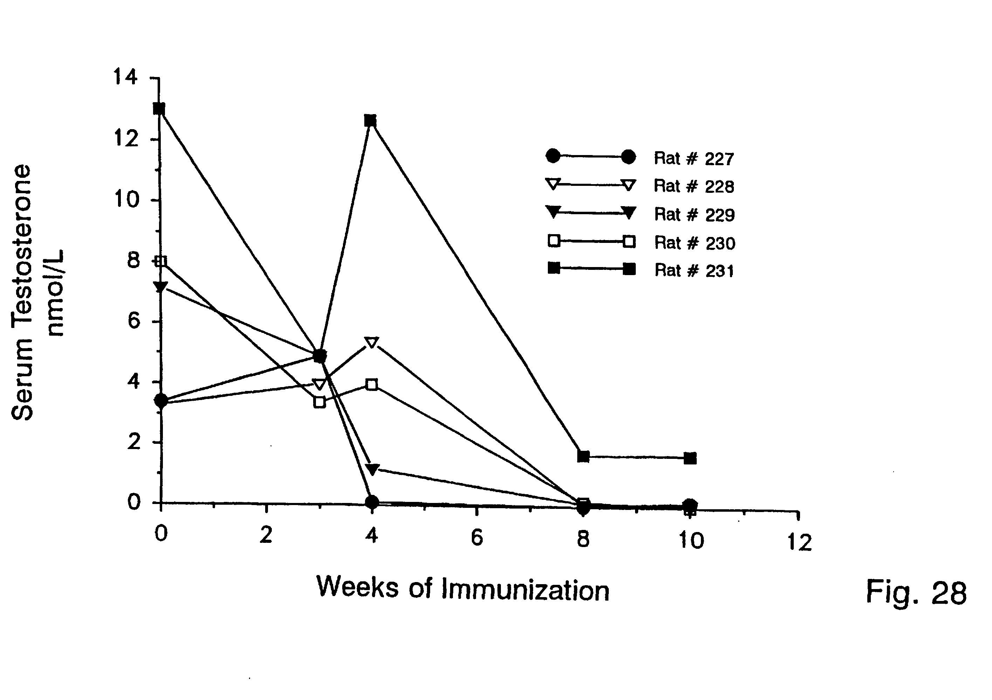

Fig. 28 graphically depicts serum testosterone levels in rats following administration of the prototype immunogen cocktail. The experimental design is that described in the legend to Fig. 27.

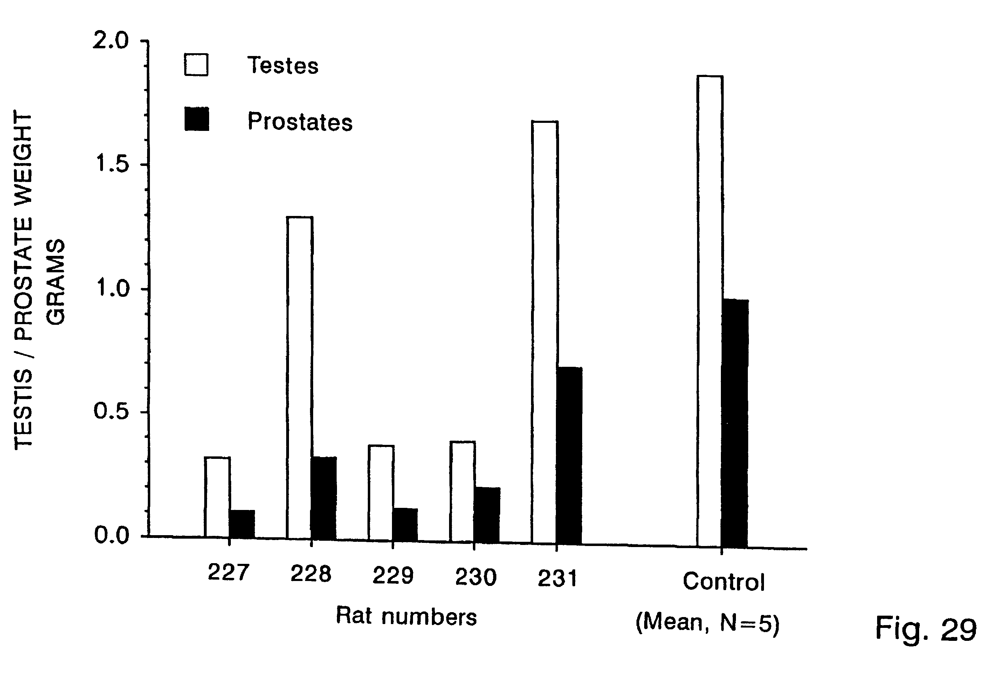

Fig. 29 graphically depicts testis weights of animals given the prototype immunogen cocktail. At 10 weeks following the commencement of the experiment described in the legend to Fig. 27, animals were sacrificed and the relevant organs dissected and weighed. Testis and prostate weights are expressed in grams. Control animals were immunized with alum adjuvant without antigen, using an identical schedule to the experimental groups.

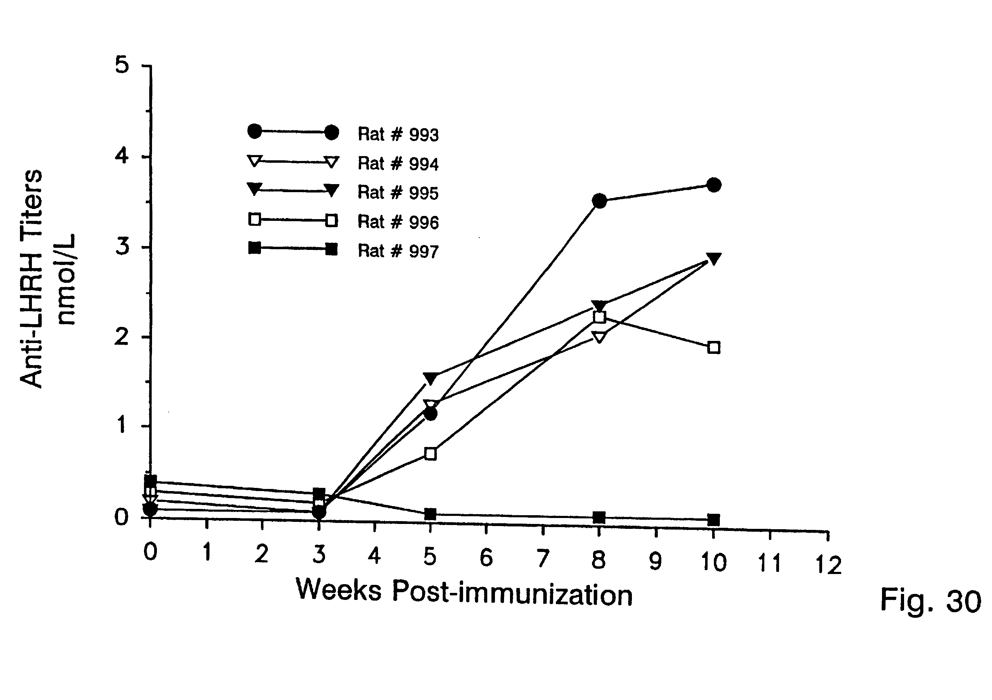

Fig. 30 graphically depicts levels of anti-LHRH specific antibody produced in rats following immunization with Inv: HBsAgT$_h$ : LHRH (peptide 32). Peptide 32 consists of a segment of Yersinnia adhesion molecule, Invasin, linked to a T cell helper epitope derived from the hepatitis B virus surface antigen linked to LHRH. Five sexually mature Sprague-Dawley male rats per group were given peptide 32 equivalent to 100 µg of peptide A by subcutaneous administration. The antigen was formulated on aluminum hydroxide and given at week 0, 3 and 6. The control group was given unmodified LHRH on alum using the same immunization schedule.

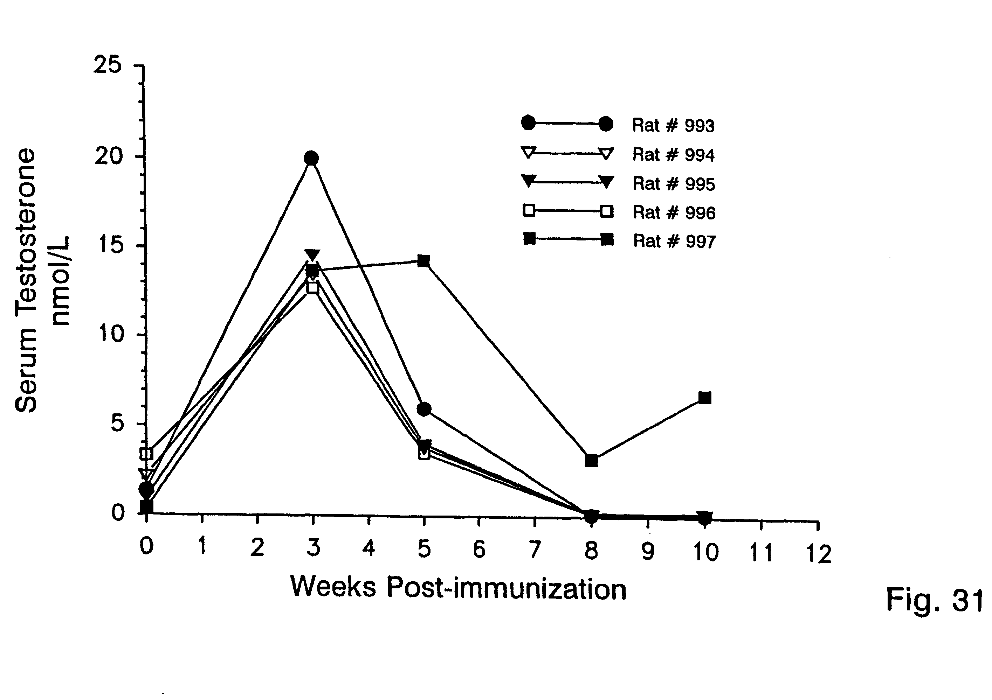

Fig. 31 graphically depicts serum testosterone levels in rats following administration of peptide 32. The experimental design is that described in the legend to Fig. 30.

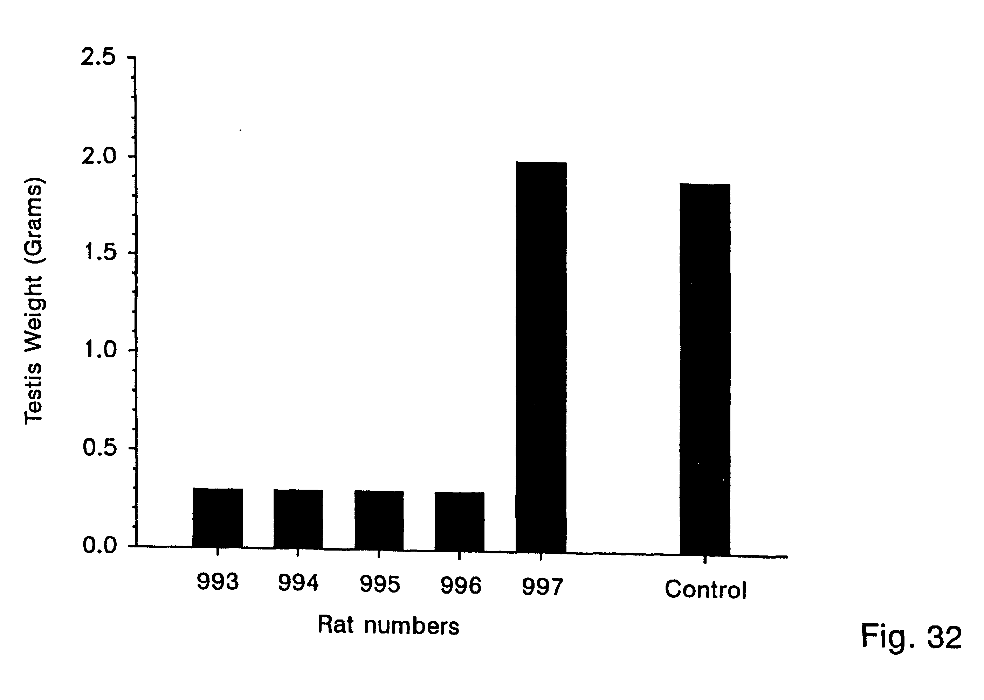

Fig. 32 graphically depicts testis weights of animals given peptide 32. At 10 weeks following the commencement of the experiment described in the legend to Fig. 30, animals were sacrificed and the relevant organs dissected and weighed. Testis weights are expressed as the mean value in grams of organ weight per 100 grams of body weight. Control animals were immunized with alum adjuvant without antigen, using an identical schedule to the experimental groups.

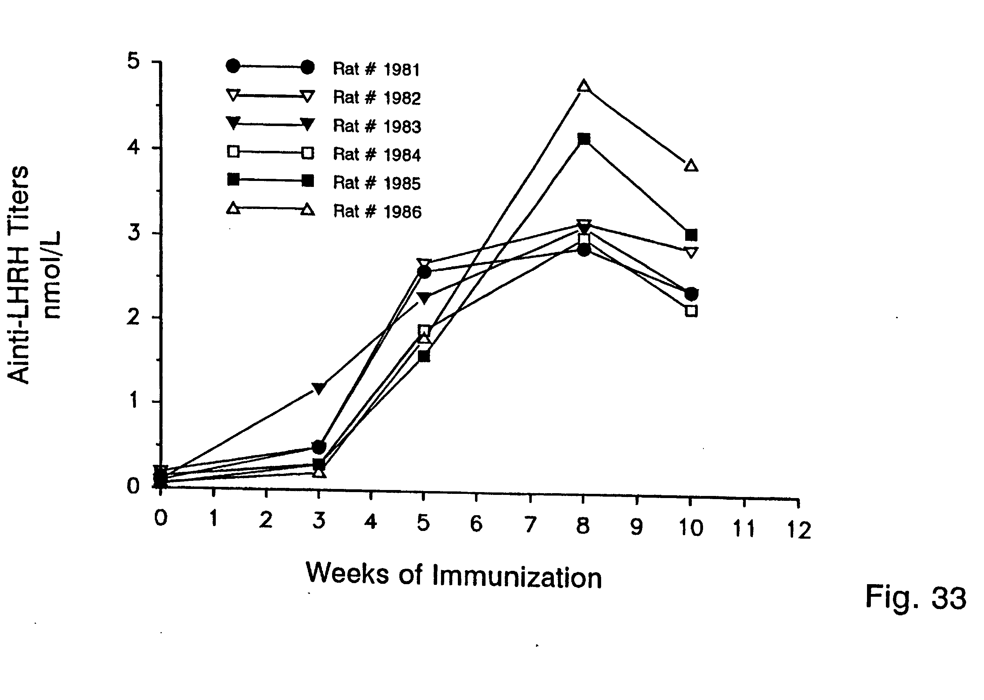

Fig. 33 graphically depicts levels of anti-LHRH specific antibody produced by immunization with a immunogen cocktail containing peptide H. Equimolar amounts of Inv:HBsAgT$_h$: LHRH + MV F T$_h$:LHRH + PT T$_h$:LHRH + TT T$_h$: LHRH were mixed and formulated on alum. Five sexually mature Sprague-Dawley male rats per group were given a molar equivalent of the immunogen cocktail equal to 100 µg of peptide A by intramuscular administration at weeks 0, 3 an 6.

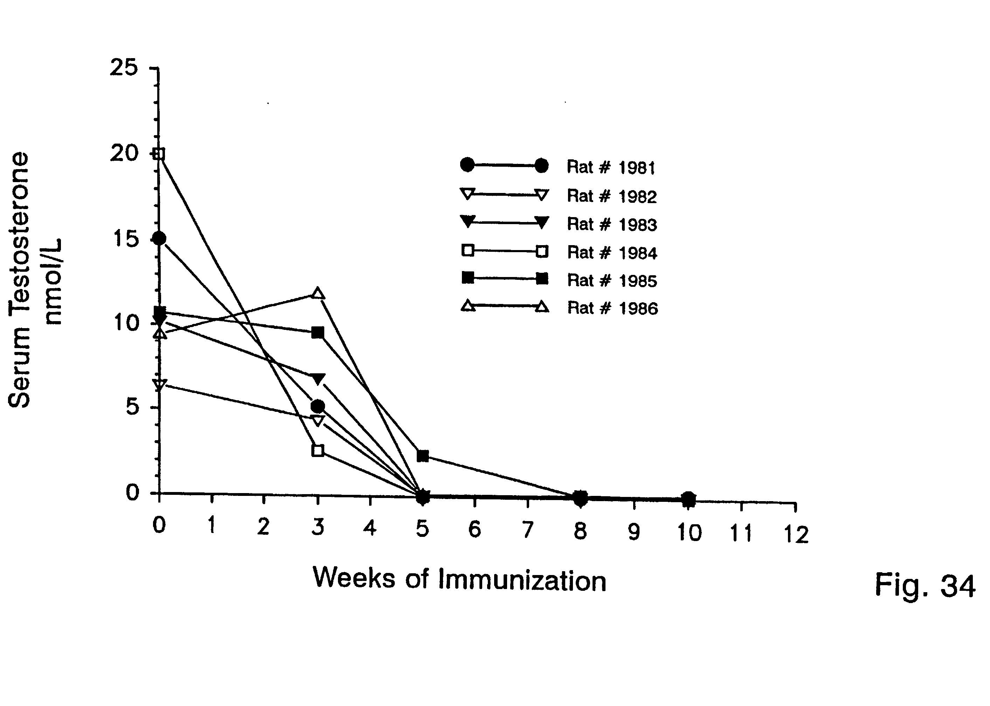

Fig. 34 graphically depicts serum testosterone levels in rats following administration of the prototype immunogen cocktail. The experimental design is that described in the legend to Fig. 33.

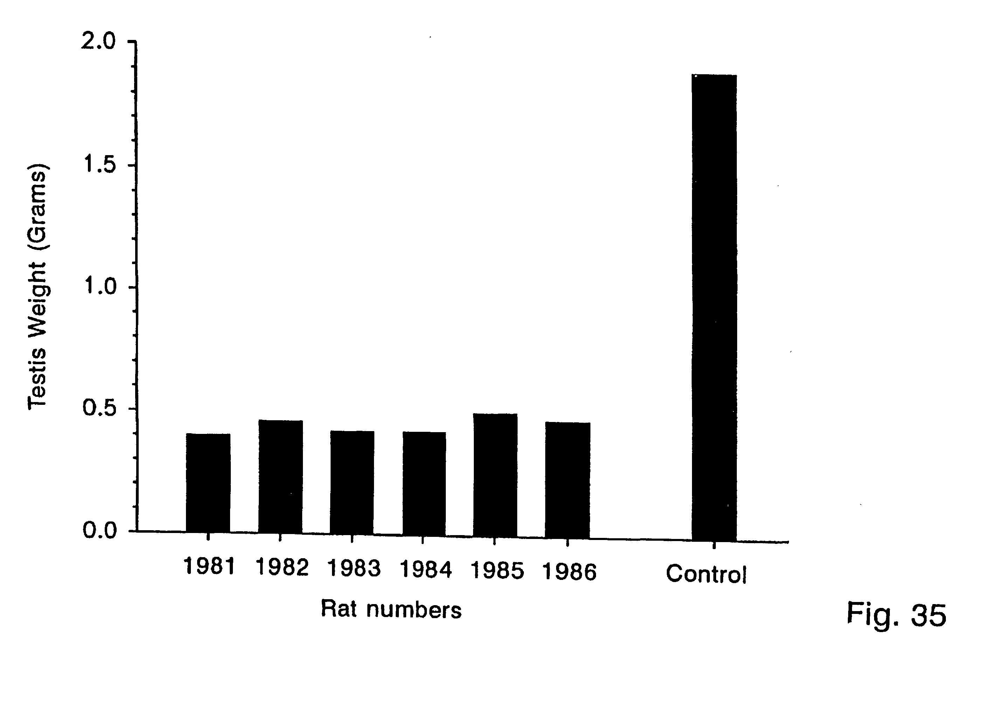

Fig. 35 graphically depicts testis weights of animals given the prototype immunogen cocktail. At 10 weeks following the commencement of the experiment described in the legend to Fig. 33, animals were sacrificed and the relevant organs dissected and weighed. Testis weights are expressed in grams. Control animals were immunized with alum adjuvant without antigen, using an identical schedule to the experimental groups.

The present invention relates to peptides, preferably synthetic peptides, which are capable of inducing antibodies against LHRH, which antibodies lead to the suppression of active LHRH levels in males or females. For the present invention, the following factors contribute to the immunoefficacy of the subject LHRH constructs. These factors, singly or in combination, are considered important aspects for preparing peptides in accordance with the present invention.

**1. Addition of Promiscuous Helper T (T$_h$) Cell Epitopes.** To evoke an efficient antibody response, immunogens must be presented in conjunction with major histocompatibility (MHC) class II antigens. The MHC class II antigens produced by antigen-presenting cells (APCs) bind to T cell epitopes present in the immunogen in a sequence specific manner. This MHC class II-immunogen complex is recognized by CD4$^+$ lymphocytes (T$_h$ cells), which cause the proliferation of specific B cells capable of recognizing a B cell epitope from the presented immunogen and the production of B cell epitope-specific antibody by them. Since LHRH is a self molecule, it does not possess any recognizable T$_h$ epitopes. Such epitopes can be provided by specific sequences derived from potent immunogens including tetanus toxin, pertussis toxin, the measles virus F protein and the hepatitis B virus surface antigen (HBsAg). The T$_h$ epitopes selected are, preferably, capable of eliciting helper T cell responses in large numbers of individuals expressing diverse MHC haplotypes. These epitopes function in many different individuals of a heterogeneous population and are considered to be promiscuous T$_h$ epitopes. Promiscuous T$_h$ epitopes provide an advantage of eliciting potent LHRH antibody responses in most members of genetically diverse population groups.

Thus, the helper epitopes of this invention are selected not only for a capacity to cause immune responses in most members of a given population, but also for a capacity to cause memory/recall responses. The vast majority of human patients receiving LHRH immunotherapy will already have been immunized with the pediatric vaccines (i.e., measles + mumps + rubella and diphtheria + pertussis + tetanus vaccines) and, possibly, the newer hepatitis B virus vaccine. These patients have therefore been previously exposed to more than one of the T$_h$ epitopes present in the immunogen mixture. Prior exposure to a T$_h$ epitope through immunization with the standard vaccines should establish T$_h$ cell clones which can immediately proliferate upon administration of the LHRH immunotherapy (i.e. a recall response), thereby stimulating rapid B cell responses to LHRH. In addition, the T$_h$ epitopes avoid any pathogen-specific B cell and/or suppressor T cell epitopes which could lead to carrier-induced immune suppression, a problem encountered when toxin molecules are used to elicit helper T cell responses.

**2. Addition of Spacer Residues Between ImmunogenicElements.** Inmunogenicity can be improved through the addition of spacer residues (e.g. Gly-Gly) between the promiscuous T$_h$ epitope and LHRH. In addition to physically separating the T$_h$ epitope from the B cell epitope (i.e., LHRH), the glycine residues can disrupt any artificial secondary structures created by the joining of the T$_h$ epitope with LHRH --and thereby eliminate interference between the T and/or B cell responses. The conformational separation between the helper epitope and the antibody eliciting domain thus permits more efficient interactions between the presented immunogen and the appropriate T$_h$ and B cells.

**3. Mixing of T$_h$ Epitope-modified Immunogens to Cause Broad-spectrum Efficacy.** The T$_h$ epitopes of

the invention are promiscuous but not universal. This characteristic means that the $T_h$ epitopes are reactive in a large segment of an outbred population expressing different MHC antigens (reactive in 50 to 90% of the population), but not in all members of that population. To provide a comprehensive, approaching universal, immune reactivity for the LHRH immunotherapeutic construct, a combination of LHRH constructs with different $T_h$ epitopes can be prepared. For example, a combination of four $T_h$ epitope: LHRH constructs, including promiscuous $T_h$ epitopes from tetanus and pertussis toxins, measles virus F protein and from the HBsAg is particularly effective. On an equimolar basis, this mixture is more broadly effective than any single immunogen in the mixture.

**4. Production of $T_h$ Epitope Libraries.** (not with the scope of the present invention) In another embodiment the $T_h$ epitope can be a structured synthetic antigen library (SSAL) as described in U.S. Serial No. 143,412, filed Oct. 26, 1993, which is incorporated herein by reference. This technology can be used as another, and perhaps, a more efficient means to obtain universal immune reactivity (as opposed to mixing promiscuous helper epitope constructs). An SSAL is composed of an ordered set of from 2 to several trillion different, but related, peptides made simultaneously in a single, automated peptide synthesis. The sequences of the peptides within a library are defined by a set of peptides or protein domains which share common structural and/ or functional properties. The order within any SSAL is provided by invariant amino acid residues which define the core sequence of the library. The core sequence is determined by aligning the primary amino acid sequences of a related family of epitopes, identifying the invariant loci within the alignment and the specific amino acid residues present at each invariant position. The SSAL is then synthesized with conserved amino acid residues at the invariant positions as defined by the alignment. The degeneracy within the library is determined by the loci within the alignment that harbor different amino acid residues when the ordered epitopes are compared. The degree of degeneracy within an array is determined by the number of variant loci within the alignment and the number of different amino acids found at each variant locus.

Promiscuous $T_h$ epitopes are included in structured libraries since they often share common structural features as based upon similar landmark sequences. For example, promiscuous $T_h$ epitopes range in size from about 15 to about 30 residues. Amphipathic helices are a common feature of the $T_h$ epitopes. An amphipathic helix is defined by an alpha-helical structure with hydrophobic amino acid residues dominating one face of the helix, and charged and polar residues dominating the surrounding faces. $T_h$ epitopes frequently contain additional primary amino acid patterns such as: a Gly or a charged reside followed by two to three hydrophobic residues followed in turn by a charged or polar residue. This pattern defines Rothbard sequences. $T_h$ epitopes often obey the 1, 4, 5, 8 rule, where a positively charged residue is followed by hydrophobic residues at the fourth, fifth and eighth positions after the charged residue. Since all of these structures are composed of common hydrophobic, charged and polar amino acids, each structure can exist simultaneously within a single $T_h$ epitope.

**5. Covalent Addition of an Invasin Domain as an Adjuvant.** The invasins of the pathogenic bacteria Yersinia spp. are outer membrane proteins which mediate entry of the bacteria into mammalian cells (Isberg and Leong, 1990, Cell 60:861). Invasion of cultured mammalian cells by the bacterium was demonstrated to require interaction between the Yersinia invasin molecule and several species of the β1 family of integrins present on the cultured cells (Tran Van Nhieu and Isberg, 1991, J. Biol. Chem. 266:24367). Since T lymphocytes are rich in β1 integrins (especially activated immune or memory T cells) the effects of invasin upon human T cell have been investigated (Brett et al., 1993, Eur. J. Immunol. 23:1608). It is thought that integrins facilitate the migration of immune T cells out of the blood vessels and through connective tissues to sites of antigenic challenge through their interaction with extracellular matrix proteins including fibronectin, laminin and collagen. The carboxy-terminus of the invasin molecule was found to be co-stimulatory for naive human CD4[+] T cells in the presence of the non-specific mitogen, anti-CD3 antibody, causing marked proliferation and expression of cytokines. The specific invasin domain which interacts with the β1 integrins to cause this stimulation also was identified (Brett et al., 1993). Because of the demonstrated T cell co-stimulatory properties associated with this domain, it can be linked it to promiscuous $T_h$ epitope: LHRH constructs.

**6. Selection of an Adjuvant/Emulsion Formulation to Maximize Antibody Responses.** In addition to the significant adjuvanting properties associated with covalent modifications of the $T_h$ epitope: LHRH constructs (e.g. the invasin domain , addition of exogenous adjuvant/emulsion formulations which maximize immune responses to the LHRH immunotherapeutic immunogens have been investigated. The adjuvants and carriers that have been evaluated are those: (1) which have been successfully used in Phase I human trials; (2) based upon their lack of reactogenicity in preclinical safety studies, have the potential for approval for use in humans; or (3) have been approved for use in food and companion animals.

**7. Microparticle Delivery of Modified Immunogens.** Immunotherapy regimens which produce maximal immune responses following the administration of the fewest number of doses, ideally only one dose, are highly desirable. This result can be approached through entrapment of immunogen in microparticles. For example, the absorbable suture material poly(lactide-co-glycolide) co-polymer can be fashioned into microparticles containing immunogen. Following oral or parenteral administration, microparticle hydrolysis *in vivo* produces the non-toxic byproducts, lactic and glycolic acids, and releases immunogen largely unaltered by the entrapment process. The rate of microparticle degradation and the release of entrapped immunogen can be controlled by several parameters, which include (1) the ratio of polymers used in particle formation (particles with higher coglycolide concentrations degrade more rapidly); (2) particle size, (smaller particles degrade more rapidly than larger ones); and, (3) entrapment efficiency, (particles with higher concentrations of entrapped antigen degrade more rapidly than particle with lower loads). Microparticle formulations can also provide primary and subsequent booster immunizations in a single administration by mixing immunogen entrapped microparticles with different release rates. Single dose formulations capable of releasing antigen ranging from less than one week to greater than six months can be readily achieved [see, for example, U.S. Serial No. 201,524, filed February 25, 1994]. Moreover, delivery of promiscuous $T_h$ epitope: LHRH immunogens entrapped in microparticles can also provide improved efficacy when the microparticulate immunogen is mixed with an exogenous adjuvant/emulsion formulations.

The peptides of this invention have a helper T cell epitope (Th epitope) and carboxyl-terminal LHRH. Moreover, the subject peptides can have LHRH replaced by an immunogenic analog of LHRH.

The peptides of this invention are represented by the formula

$$(A)_n\text{-}(Th)_m\text{-}(B)_o\text{-LHRH}$$

wherein A is independently an amino acid, $\alpha$-$NH_2$, an invasin domain or an immunostimulatory analog of the corresponding invasin domain;

B is an amino acid;
each Th is independently seq ID No. 2-9, 42, 45-49,
LHRH is luteinizing hormone releasing hormone or an immunogenic analog thereof;
n is from 1 to about 10;
m is from 1 to about 4; and
o is from 0 to about 10.

The peptides of the present invention have from about 20 to about 100 amino acid residues, preferably from about 20 to about 50 amino acid residues and more preferably from about 20 to about 35 amino acid residues. In another preferred embodiment, the peptide has from about 25 to about 40 amino acid residues.

When A is an amino acid, then it can be any non-naturally occurring amino acid or any naturally occurring amino acid. Non-naturally occuring amino acids include, but are not limited to, $\beta$-alanine, ornithine, norleucine, norvaline, hydroxyproline, thyroxine, gamma-amino butyric acid, homoserine, citrulline and the like. Naturally-occurring amino acids include alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Moreover, when m is greater than one, and two or more of the A groups are amino acids, then each amino acid is independently the same or different.

When A is an invasin domain it is an immunostimulatory epitope from the invasin protein of a Yersinia species. This invasin domain is also capable of interacting with the $\beta1$ integrin molecules present on T cells, particularly activated immune or memory T cells, as described above under point 5 in the Detailed Description of the Invention. In a preferred embodiment the invasin domain has the sequence:

```
Thr-Ala-Lys-Ser-Lys-Lys-Phe-Pro-Ser-Tyr-Thr-Ala~Thr-
Tyr-Gln-Phe                                    Seq ID No: 53
```

or is an immunostimulatory analog thereof from the corresponding region in another Yersinia species invasin protein. Such analogs thus have substitutions, deletions or insertions to accommodate strain to strain variation, provided that the analogs retain its immunostimulatory properties.

In one embodiment, n is four and A is $\alpha$-$NH_2$, lysine, lysine and lysine in that order. In another embodiment n is one and A is $\alpha$-$NH_2$. In yet another embodiment, m is four and A is $\alpha$-$NH_2$, an invasin domain, glycine and glycine in that order.

The amino acids for B can be the naturally occuring amino acids or the non-naturally occurring amino acids as described above. Each B is independently the same or different. When B is lysine then a polymer can be formed. For example, if o is 7 and all seven B groups are lysine then a branching heptalysyl core ($K_4K_2K$ or K core) is formed when peptide synthesis is performed without protection of the lysyl side chain $\in$-amino group. Peptides with a K core have eight branch arms, with each branch arm being identical and represented by the formula $(A)_n\text{-}(Th)_m\text{-}(B)_o\text{-}$. In addition, the amino acids of B can form a flexible hinge, or spacer, to enhance the immune reponse to the Th epitope and LHRH. Examples of sequences encoding flexible hinges are found in the immunoglobulin heavy chain hinge region. Flexible hinge sequences are often proline rich. One particularly useful flexible hinge is provided by the sequence Pro-Pro-Xaa-Pro-Xaa-Pro, where Xaa is any amino acid, and preferably aspartic acid. An example of a spacer is provided by the sequence Gly-Gly.

Th is a sequence of amino acids (natural or non-natural amino acids) that comprises a Th epitope as indicated before. A Th epitope can consist of a continuous or discontinuous epitope. Hence not every amino acid of Th is necessarily part of the epitope. Accordingly, Th epitopes, including analogs and segments of Th epitopes, are capable of enhancing or stimulating an immune response to LHRH. Immunodominant Th epitopes are broadly reactive in animal and human populations with widely divergent MHC types [Celis *et al.* (1988) J. Immunol. 140:1808-1815; Demotz *et al.* (1989) J. Immunol. 142:394-402; Chong *et al.* (1992) Infect. Immun. 60:4640-4647]. The Th domain of the subject peptides has from about 10 to about 50 amino acids and preferably from about 10 to about 30 amino acids. When multiple Th epitopes are present (i.e. $n \geq 2$), then each Th epitope is independently the same or different.

Th epitope analogs include substitutions, deletions and insertions of from one to about 10 amino acid residues in the Th epitope. Th segments are contiguous portions of a Th epitope that are sufficient to enhance or stimulate an immune response to LHRH. An example of Th segments is a series of overlapping peptides that are derived from a single longer peptide.

Th epitopes of the present invention include hepatitis B surface antigen helper T cell epitopes ($HB_s$Th), pertussis toxin helper T cell epitopes (PT Th), tetanus toxin helper T cell epitopes (TT Th), measles virus F protein helper T cell epitope ($MV_F$ Th), Chlamyidia trachomitis major outer membrane protein helper T cell epitopes (CT $T_h$), diphtheria toxin helper T cell epitopes (DT $T_h$), Plasmodium falciparum circumsporozoite helper T cell epitopes (PF $T_h$), Schistosoma mansoni triose phosphate isomerase helper T cell epitopes (SM $T_h$), Escherichia coli TraT helper T cell epitopes (TraT $T_h$) and immune-enhancing analogs and segments of any of these Th epitopes. Examples of Th epitope sequences are provided below:

HB, Th:    Phe-Phe-Leu-Leu-Thr-Arg-Ile-Leu-thr-Ile-Pro-Gln-
Ser-Leu-Asp,,                    SEQ ID NO:2

PT$_1$ Th:    Lys-Lys-Leu-Arg-Arg-Leu-Leu-Tyr-Met-Ile-Tyr-Met-
Ser-Gly-Leu-Ala-Val-Arg-Val-His-Val-Ser-Lys-Glu-
Glu-Gln-Tyr-Tyr-Asp-Tyr,           SEQ ID NO:3

TT$_1$ Th:    Lys-Lys-Gln-Tyr-Ile-Lys-Ala-Asn-Ser-Lys-Phe-Ile-
Gly-Ile-Thr-Glu-Leu,             SEQ ID NO:4

TT$_2$ Th:    Lys-Lys-Phe-Asn-Asn-Phe-Thr-Val-Ser-Phe-Trp-Leu-
Arg-Val-Pro-Lys-Val-Ser-Ala-Ser-His-Leu
                             SEQ ID NO:5

PT$_{1A}$ Th:    Tyr-Met-Ser-Gly-Leu-Ala-Val-Arg-Val-His-Val-Ser-
Lys-Glu-Glu,               SEQ ID NO:6

TT$_3$ Th:    Tyr-Asp-Pro-Asn-Tyr-Leu-Arg-Thr-Asp-Ser-Asp-Lys-
Asp-Arg-Phe-Leu-Gln-Thr-Met-Val-Lys-Leu-Phe-Asn-
Arg-Ile-Lys,               SEQ ID NO:7

PT$_2$ Th:    Gly-Ala-Tyr-Ala-Arg-Cys-Pro-Asn-Gly-Thr-Arg-Ala-
Leu-Thr-Val-Ala-Glu-Leu-Arg-Gly-Asn-Ala-Glu-Leu
                        SEQ ID NO:8.

MV$_F$ Th:    Leu-Ser-Glu-Ile-Lys-Gly-Val-Ile-Val-His-Arg-Leu-
Glu-Gly-Val               SEQ ID NO:9

MV$_{F2}$ T$_h$:    Gly-His-Leu-Glu-Ser-Arg-Gly-His-Lys-Ala-Arg-His-
Thr-His-Val-Asp-Thr-Glu-Ser-Tyr      SEQ ID NO:42

TT$_4$ T$_h$:
(comparative)    Trp-Val-Arg-Asp-His-His-Asp-Asp-Phe-Thr-Asn-Glu-
Ser-Ser-Gln-Lys-Thr           SEQ ID NO:43

TT₃ Tₕ: *(Comparative)* Asp-Val-Ser-Thr-His-Val-Pro-Tyr-His-Gly-Pro-Ala-Leu-Asn-His-Val      SEQ ID NO:44

CT Tₕ: Ala-Leu-Asn-His-Trp-Asp-Arg-Phe-Asp-Val-Phe-Cys-Thr-Leu-Gly-Ala-Thr-Thr-Gly-Tyr-Leu-Lys-Gly-Asn-Ser      SEQ ID NO:45

DT₁ Tₕ: Asp-Ser-Glu-Thr-Ala-Asp-Asn-Leu-Glu-Lys-Thr-Val-Ala-Ala-Leu-Ser-His-Leu-Pro-Gly-His-Gly-Cys      SEQ ID NO:46

DT₂ Tₕ: Glu-Glu-His-Val-Ala-Gln-Ser-His-Ala-Leu-Ser-Ser-Leu-Met-Val-Ala-Gln-Ala-His-Pro-Leu-Val-Gly-Glu-Leu-Val-Asp-His-Gly-Phe-Ala-Ala-Thr-Asn-Phe-Val-Glu-Ser-Cys      SEQ ID NO:47

PF Tₕ: Asp-His-Glu-Lys-Lys-His-Ala-Lys-Met-Glu-Lys-Ala-Ser-Ser-Val-Phe-Asn-Val-Val-Asn-Ser      SEQ ID NO:48

SM Tₕ: Lys-Trp-Phe-Lys-Thr-Asn-Ala-Pro-Asn-Gly-Val-Asp-Glu-Lys-His-Arg-His      SEQ ID NO:49

TraT₁ Tₕ: *(comparative)* Gly-Leu-Gln-Gly-Lys-Hfis-Ala-Asp-Ala-Val-Lys-Ala-Lys-Gly      SEQ ID NO:50

TraT₂ Tₕ: *(comparative)* Gly-Leu-Ala-Ala-Gly-Leu-Val-Gly-Met-Ala-Ala-Asp-Ala-Met-Val-Glu-Asp-Val-Asn      SEQ ID NO:51

TraT₃ Tₕ: *(comparative)* Ser-Thr-Glu-Thr-Gly-Asn-Gln-His-His-Tyr-Gln-Thr-Arg-Val-Val-Ser-Asn-Ala-Asn-Lys      SEQ ID NO:52

In a preferred embodiment the Th epitope is HB, Th, PT Th or $TT_1$ Th or $MV_{F1}T_h$.

LHRH has the amino acid sequence Glu-His-Trp-Ser-Tyr-Gly-Leu-Arg-Pro-Gly (SEQ ID NO:1). LHRH analogs according to the invention have a substitution, deletion, or insertion of from one to about four amino acid residues provided that the analog is capable of stimulating an immmune response crossreactive with LHRH. For example, replacing the glycine residue at position six with a D-amino acid, preferably D-lysine, produces an immunogenic analog of LHRH (Jayashankar *et al.*). The substitutions and insertions can be accomplished with natural or non-natural amino acids as defined herein.

Accordingly, peptides of this invention are Peptide A (SEQ ID NO:10; Table 1), Peptides F-L (SEQ ID NOS:

11-17; Table 4) and Peptides 18-41 (SEQ ID NOS:18-41; Table 5). Preferred peptides include Peptide A, Peptide F and Peptide H. More preferred peptides include peptides 18, 19, 32-35, H and K, and most preferably 19, 32, H and K.

The peptides of this invention can be made by synthetic chemical methods which are well known to the ordinarily skilled artisan. see, for example, Grant, ed. (1992) Synthetic Peptides: A User's Guide, W.H.Freeman & Co., New York, NY, pp. 382. Hence, peptides can be synthesized using the automated Merrifield techniques of solid phase synthesis with either t-Boc or F-moc chemistry on an Applied Biosystems Peptide Synthesizer Model 430A or 431. To synthesize a K core moiety, unprotected [Di(tBoc) or Di(Fmoc)-N$^\alpha$, N$^\epsilon$] lysine residues are used in place of lysine residues with a protected $\epsilon$-amino group. To improve the specificity of the final coupling reaction, the solid-phase peptide can be elongated with additional serine and lysine residues at the N-terminus.

After complete assembly of the desired peptide, the resin is treated according to standard procedures to cleave the peptide from the resin and deblock the protecting groups on the amino acid side chains. The free peptide is purified by HPLC and characterized biochemically, for example, by amino acid analysis or by sequencing. Purification and characterization methods for peptides are well known to one of ordinary skill in the art.

Alternatively, the longer linear peptides can be synthesized by well known recombinant DNA techniques. Any standard manual on DNA technology provides detailed protocols to produce the peptides of the invention. To construct a gene encoding a peptide of this invention, the amino acid sequence is reverse transcribed into a nucleic acid sequence, and preferably using optimized codon usage for the organism in which the gene will be expressed. Next, a synthetic gene is made, typically by synthesizing overlapping oligonucleotides which encode the peptide and any regulatory elements, if necessary. The synthetic gene is inserted in a suitable cloning vector and recombinants are obtained and characterized. The peptide is then expressed under suitable conditions appropriate for the selected expression system and host. The peptide is purified and characterized by standard methods.

The subject peptides can also be polymerized. Polymerization can be accomplished by reaction with dilute glutaraldehyde using routine methodology.

The efficacy of the peptides can be established and analyzed by injecting an animal, for example rats, and following the immune response to LHRH, the serum testosterone levels and palpating the testes. At the end of the experimental period the animal can be sacrificed and androgen-dependent organ weights obtained. Androgen-dependent organs include the testes, the epididymis, the prostate and the seminal vesicles. In a preferred method of measuring efficacy, the LHRH construct is formulated in alum and injected into rats. This method is detailed in the Examples.

Another aspect of this invention provides a vaccine composition comprising an immunologically-effective amount of one or more of the peptides of this invention and a pharmaceutically acceptable carrier. Such vaccine compositions are used in the methods of inducing infertility or treating prostatic hyperplasia, androgen-dependent carcinoma, prostatic carcinoma, testicular carcinoma, endometriosis, benign uterine tumors, recurrent functional ovarian cysts or (severe) premenstrual syndrome or prevention or treatment of estrogen-dependent breast tumors.

Accordingly, the subject peptides can be formulated as a vaccine composition using adjuvants, pharmaceutically-acceptable carriers or other ingredients routinely provided in vaccine compositions. Such formulations are readily determined by one of ordinary skill in the art and include formulations for immediate release and for sustained release, e.g., microencapsulation. The present vaccines can be administered by any convenient route including subcutaneous, oral, intramuscular, or other parenteral or enteral route. Similarly the vaccines can be administered as a single dose or divided into multiple doses for administration. Immunization schedules are readily determined by the ordinarily skilled artisan. For example, the adjuvants or emulsifiers that can be used in this invention include alum, incomplete Freund's adjuvant, liposyn, saponin, squalene, L121, emulsigen and ISA 720 as well as the other efficacious adjuvants and emulsifiers described in Tables 7-9. In a preferred embodiment, the adjuvants/emulsifiers are alum, incomplete Freund's adjuvant, a combination of liposyn and saponin, a combination of squalene and L121 or a combination of emulsigen and saponin.

The vaccine compositions of the instant invention contain an immunoeffective amount of one or more of the LHRH-containing peptides and a pharmaceutically acceptable carrier. such compositions in dosage unit form can contain about 0.5 $\mu$g to about 1 mg of each peptide per kg body weight. When delivered in multiple doses, the dosage unit form is conveniently divided into the appropriate amounts per dosage.

Vaccines which contain cocktails of two or more of the subject peptides enhance immunoefficacy in a broader population and thus provide a better immune response against LHRH. For example, a cocktail of Peptides A, F and H is useful. A preferred cocktail includes Peptides 18, 19, K and H; another includes 32, 19, K and H. Other immunostimulatory synthetic peptide LHRH immunogens are arrived at through modification into lipopeptides so as to provide built-in adjuvanticity for potent vaccines. The immune response to synthetic peptide LHRH immunogens can be improved by delivery through entrapment in or on biodegradable microparticles of the type described by O'Hagan et al. (1991) Vaccine 9:768-771. The immunogens can be encapsulated with or without adjuvant, including covalently attached Pam$_3$Cys (see Example 15), and such microparticles can be administered with an immunostimulatory adjuvant such as Freund's Incomplete Adjuvant or alum. The microparticles function to potentiate immune responses to an immunogen and to provide time-controlled release for sustained or periodic responses, for oral administration, and for topical administration [O'Hagan *et al*.; Eldridge *et al.* (1991) Molec. Immunol. 28:287-294].

A further aspect of the invention relates to a method for reducing or suppressing activity of LHRH levels in a mammal by administering one or more of the subject peptides to the mammal for a time and under conditions sufficient to induce functional antibodies directed against said LHRH. Suppression of LHRH levels can be used to induce infertility via suppression of spermatogenesis or ovulation. Likewise, suppression of functional, circulating LHRH levels is effective to treat prostatic hyperplasia, androgen-dependent carcinoma, prostatic carcinoma, testicular carcinoma, endometriosis, benign uterine tumors, recurrent functional ovarian cysts or (severe) premenstrual syndrome or estrogen-dependent breast tumors (treatment of such breast tumors includes prevention thereof). In animals, suppression of circulating levels of functional LHRH is useful to reduce boar taint in pigs, to immunocastrate dogs and cats, and to geld stallions.

Serum LHRH can be measured by radioimmunoassay (RIA), enzyme-linked immunoadsorbent assay (EIA) or other convenient method. Antibodies against LHRH are measured by RIA (see Example 2) or EIA. Serum testosterone is measured by RIA. The vaccine dosage needed to reduce or suppress activity of LHRH can be determined by the ordinarily skilled artisan. Such compositions in dosage unit form can contain about 0.5 µg to about 1 mg of each peptide per kg body weight. When delivered in multiple doses, the dosage unit form is conveniently divided into the appropriate amounts per dosage.

More particularly, the invention provides the use compounds according the present invention for inducing infertility in a mammal by administering the subject vaccine compositions to the mammal or a farm animal for a time and under conditions to produce an infertile state in the mammal or the farm animal. As used herein an infertile state is that state which prevents conception. Infertility can be measured by methods known in the art, e.g. evaluation of spermatogenesis or ovulation, as well as by statistical modeling of experimental animal data. An indicator of infertility in males includes reduction of serum testosterone to near castration levels. Compositions in dosage unit form can contain about 0.5 µg to about 1 mg of each peptide per kg body weight. When delivered in multiple doses, the dosage unit form is conveniently divided into the appropriate amounts per dosage.

Similarly, this invention relates to the use for treating androgen-dependent carcinoma by administering the subject vaccine compositions-to the mammal for a time and under conditions to effect regression of the carcinoma, or to prevent (further) growth of the carcinoma. Compositions in dosage unit form can contain about 0.5 µg to about 1 mg of each peptide per kg body weight. When delivered in multiple doses, the dosage unit form is conveniently divided into the appropriate amounts per dosage.

The identification and synthesis of peptides with a defined B-cell or a cytotoxic-T cell epitope and its immediate flanking sequences provides an essential component for the production of a synthetic peptide immunogen. However, additional required components, such as effective helper T cell epitopes, which must be present to provide the full range of immune responses necessary to elicit the desired biological effect, may not be included in such sequences. Addition of a universal synthetic immune stimulator to a poorly antigenic peptide immunogen provides an effective solution to this problem. A universal immune stimulator which when linked to any peptide or protein (i.e., the peptide hapten), containing either B cell and/or cytotoxic T lymphocyte (CTL) epitopes, causes potent immune responses to the coupled peptide or protein. The universal immune stimulator consists of a promiscuous helper T cell ($T_h$) epitope which elicits an immune response to the coupled peptide in members of a heterogeneous population expressing diverse HLA phenotypes (as hereinbefore defined) and an adjuvant peptide sequence from the invasin protein of Yersinia which is capable of specifically binding to $CD4^+$ and $CD8^+$ lymphocytes (as defined herein above). Further, the immune stimulator can have a lipid moiety or charged amino acid residues which act to increase the binding affinity of the immune stimulator for biological membranes. The target peptide hapten can be a self molecule and, therefore, not immunogenic without modification, such as LHRH, which following addition of the immune stimulator can be used in the treatment of cancer or other non-infectious diseases. Similarly, the peptide hapten can be a B cell epitope representing neutralizing determinants or CTL epitope peptides from a viral, bacterial or a parasitic pathogen for use as a vaccine or an immunotherapy.

In order to provide maximum coverage, that is maximum immune responses in members of a genetically diverse population (e.g. as broad-based response as possible), synthetic peptides contain the invasin domain, a promiscuous $T_h$ epitope, and a B cell epitope (or a CTL epitope) can be mixed together and formulated with adjuvant and vaccine carrier. Alternatively, rather than peptide mixtures, peptide libraries (i.e. SSALs) which represent the promiscuous $T_h$ epitope and/or the B cell or CTL epitope are synthesized into the peptides of the invention and formulated for vaccine delivery. This technology, i.e. SSAL, provides a significant advantage in both simplifying the manufacture as well as improving the immunologic coverage provided relative to simple mixtures of peptides for use as immunogens.

The synthetic peptides of the invention are made by automated chemical synthesis as described above.

Specific peptide haptens of the present invention are described below together with diseases that can be ameliorated by immune responses to such peptides or immunotherapies provided by such peptides.

EXAMPLE 1

Immunization of Rats with Linear and Octameric LHRH-containing peptides

A. Immunogen preparation: Peptides A-E (Table 1) and all other peptides were synthesized using the strategy of solid phase synthesis employing the standard F-moc chemistry performed on an Applied Biosystems Peptide Synthesizer Model 430A or 431 according to manufacturer's instructions. Di(Fmoc)-$\alpha$, $\in$ NH$_2$ protected lysine was used, in doubling concentrations after each additional cycle of coupling, for synthesis of the heptalysyl core (K$_4$K$_2$K or K$_{core}$). After complete assembly of the peptide, the resin was treated with TFA (trifluroacetic acid) according to standard procedures to cleave the peptide from the resin and deblock the protecting groups on amino acid side chains. The free peptide was then purified by HPLC and characterized biochemically by amino acid analysis.

The structure of the peptides from the amino terminus to the carboxyl terminus is as follows: Peptide A is a linear peptide with three domains: 3 lysine residues (3K), the hepatitis B surface antigen helper T cell epitope (HB$_s$Th epitope) and LHRH. Peptide A is thus represented by 3K-HB$_s$Th-LHRH. Peptide B is an octameric peptide with each branch having two copies of LHRH. The branches are attached to a heptalysyl core that has a HB$_s$Th epitope attached to its C terminal tail. Peptide B is thus represented by (LHRH-LHRH)$_8$-K$_{core}$-HB$_s$Th. Peptide C, represented by (LHRH-LHRH-LHRH)$_8$-K$_{core}$-HB$_s$Th, is similar to Peptide B except the branch has three copies of LHRH. Peptide D, (LHRH-HB$_s$Th)$_8$-K$_{core}$-AA, is an octameric peptide with each branch having one LHRH domain and one HB$_s$Th domain. The branches are attached to a heptalysyl core with two alanine residues (AA) attached to its C-terminal lysine. Peptide E, (LHRH-LHRH-HB$_s$Th)$_8$-K$_{core}$-AA, is an octameric peptide with each branch having two LHRH domains and one HB$_s$Th domain. The branches are attached to a heptalysyl core with two alanine residues attached to its C-terminal lysine. The actual sequences of these peptides are shown in Table 1.

For immunizations administered at weeks 0 and 2, 600 µg of each peptide was dissolved in 3 mL of an adjuvant solution of 0.2% Tween 80, 2.5% Pluronic L 121, 0.9% NaCl (TP). The solution was stored at 4°C until use and vortexed for 3 to 5 min prior to injection. Each rat received 100 µg per injection in 0.5 ml. For the immunization administered at week 5 in Freunds' complete adjuvant, 4 mg of each peptide was dissolved in 2 mL of 0.9% NaCl and emulsified with an equal volume of Freunds' complete adjuvant. Each rat received 500 µg per injection.

B. Immunization schedule and serum collection: Sexually mature, male Sprague-Dawley rats (n=5) were immunized subcutaneously (s.c.). Booster injections were given s.c. at weeks 2 and 5. Blood was collected at weeks 3, 6, 7 and 11 for rats injected with Peptides A, B and C, or at weeks 3, 6, 7 and 8 for rats injected with Peptides D and E.

Blood collection from the middle caudal artery was performed by injecting the rats with 1 mL of sodium pentobarbital (64.8 mg/mL; Anthony Products Co., Accadia, CA) diluted 1 to 10 in 0.9% NaCl administered intraperitoneally. The tails were kept in 48°C ± 0.5°C water for 2 min and rapidly massaged with paper towels (i.e., milked). Blood was collected immediately into a 5 mL syringe outfitted with a 23 gauge needle. Typically, 3 to 4 mL of blood was obtained. The serum was collected by centrifugation for 25 min at 3000 rpm. The serum was aliquoted in 300 µL volumes and stored frozen until used for assays.

EXAMPLE 2

Immunogenic and Therapeutic Efficacy of Peptides A-E

A. Assay methods and organ weight determinations: The anti-LHRH titer in each serum sample was measured by RIA [Ladd *et al.* (1988) Am. J. Reprod. Immunol. 17:121-127]. Antisera were diluted 1:100 (V:V) in 1% bovine serum albumin (BSA), pH 7.4. An equal volume of diluted sera was added to 100 µL of [$^{125}$I]-LHRH diluted in 1% BSA to contain approximately 15000 cpm for 5.25 pg LHRH (New England Nuclear Company, Boston, MA). The solution was incubated overnight at room temperature and antibody-bound LHRH was precipitated with 400 µL of 25% polyethylene glycol (MW 8,000) in 0.01 M phosphate-buffered saline (PBS), pH 7.6, and 200 µL of 5 mg/mL bovine gamma globulin in PBS. Antibody titers are expressed as nmol iodinated LHRH bound per liter of serum.

Serum testosterone levels were measured using an RIA kit from Diagnostic Products (Los Angeles, CA) according to manufacturer's instructions. The lower detection limit for testosterone ranged from 0.01 to 0.03 nmol/L. Each sample was analyzed in duplicate.

At 11 weeks (Peptides A-C) or 8 weeks (Peptides D and E) after the initial injection, the rats were sacrificed by overexposure to carbon dioxide. The maximum amount of trunk blood was collected. The androgen-dependent sex organs (testes, epididymis, prostate and seminal vesicles) were dissected from each rat, paper towel dried and weighed.

B. Results:

Groups of five rats were immunized with Peptides A-E. During the course of the study, anti-LHRH titers and testosterone levels were monitored in each rat. At the end of the study the rats were sacrificed and the androgen-dependent organ weights were obtained. The anti-LHRH titer, testosterone level and testes weight for each rat at the time of sacrifice are shown in Table 2. A summary of this data is provided in Table 3 together with average weights of other androgen-dependent organs.

Rats immunized with Peptide A produced antibodies against LHRH as measured by the RIA. None of the rats immunized with the other peptides (e.g. B, C, D and E) profduced any significant antibody titers against LHRH. The average anti-LHRH titer (nmol/L) at week 11 (Peptides A-C), week 8 (Peptides D-E) and control rats are reported in Table 3. The average anti-LHRH titer for the 5 rats immunized with Peptide A was 1.94 nmol/L, whereas the rats from the remaining groups had titers ranging from 0.48 to 0.73 nmol/L. The average weights of androgen-dependent organs from these groups of animals are reported in Table 3 and depicted graphically in Fig. 1. Rats immunized with Peptide A showed a significant decrease (about 40%) in organ weights relative to the control animals.

The results indicate that the presence of LHRH at the C-terminus of the peptide is more effective at stimulating antibody production and the concomitant reduction of androgen-dependent organ weights. In this regard, Peptide A has a C-terminal LHRH domain, whereas non-effective Peptides B-E have N-terminal or internal copies of LHRH.

While the average reduction of androgen-dependent organ weights of the Peptide A rats relative to Peptide B-E rats and control rats was significant, this drop was attributed to dramatic reductions that occurred in three of the five animals. Hence, the group A rats were classified into responder and non-responders and the data reanalyzed. The average androgen-dependent organ weights of responders and non-responders depicted in Fig. 2 graphically illustrates the large difference between these two groups. Responder animals had undetectable levels of serum testosterone (Table 2). Fig. 3 shows the inverse relationship between anti-LHRH titers and testes organ weight. The relationship is similar for the other androgen-dependent organ weights.

EXAMPLE 3

Immunization with a Linear Peptide Containing a Pertussis Toxin Th Epitope

Peptide F ($PT_1$Th-LHRH; Table 4) was synthesized and purified as described in Example 1. The peptide was prepared for immunization as described in Example 1 except the adjuvant was 0.5% alum. Immunizations were administered s.c. to Sprague Dawley rats at weeks 0, 2 and 4. Determination of anti-LHRH titers, testosterone levels and androgen-dependent organ weights were obtained and analyzed as described in Example 2. Eleven weeks after the initial immunization, the testes, epididymis, prostate and seminal vesicles were significantly smaller than those obtained in control animals (Fig. 4).

EXAMPLE 4

Peptide Cocktails for Induction of anti-LHRH Response in Broad Populations

Mixtures of potent synthetic LHRH peptde immunogens are formulated in combinations to provide broadly potent vaccines. Peptides A, F and H (Table 1 and Table 4) are prepared as described in Example 1 and combined in a cocktail for immunization into sexuually mature male rats at weeks 0, 3 and 6. The primary injection is in Freunds' complete adjuvant and the booster injections are in Freunds' incomplete adjuvant. Bleeds are done at weeks 0, 3, 6, 9 and 11. Animals are sacrificed at week 11 for organ weight determinations. The results are assayed and evaluated as described in Example 2.

EXAMPLE 5

Dose Dependence of Peptide A

Peptide A, $3K-HB_sT_h$-LHRH, was synthesized as described in Example 1. This peptide was tested for efficacy in accordance with the experimental design set forth below:

**Experimental Design:**

| Immunogen | peptide A |
|---|---|
| Controls | unmodified LHRH & adjuvant groups |
| Dose | 100 or 500 µg per immunization |
| Route | intramuscular |
| Adjuvant | Freund's complete/incomplete |
| Schedule | week 0 (FCA), 3 and 6 weeks (IFA) |
| Species | 8 sexually mature Sprague-Dawley male rats/group |
| Assay | LHRH-specific antibody serum testosterone LH and FSH levels relative testis size by palpation |
| Necropsy | at 10 weeks determine testis weights prostate + seminal vesicle weights epididymis weights |

**Results:**

Blood samples were periodically withdrawn from the immunized and control rats. Sera from these samples were analyzed for the presence of peptide A-specific antibody, LHRH specific-antibody, and serum testosterone. At 10 weeks, the animals were sacrificed and relevant organs, including testis and prostate glands plus seminal vesicles were dissected and weighed. Hypophysectomized rats were included in the experiment as positive controls. By week 3 (the day of the first booster) measurable LHRH-specific antibody titers were observed and a significant increase in those titers was achieved through booster immunizations (Fig. 5). The antibody titers were measured by radioimmunoassay. These results indicate that the amount of antigen used was at saturating levels, since there were no significant differences between the responses elicited by either the 100 µg or 500 µg doses. Fig. 6 establishes a strong correlation between increases in serum antibody to LHRH and the reduction in serum testosterone (a concomitant dramatic decrease in both LH and FSH was also observed). By week 5 (post primary immunization), there was a ten-fold decrease in serum testosterone and by week 8, serum testosterone was at castration levels (less than 0.5 nmole/L) in all animals. Fig. 7 demonstrates the biological effect of reducing serum testosterone through LHRH immunization. The testes size of animals immunized with the 100 µg dose of peptide A was significantly reduced by the end of the experiment (week 10). Testis size reduction in these animals was even greater than the effect obtained through pituitary ablation (i.e. the hypophysectomized group). Although not tested through mating, the state of the testes (including histopathologic examination) indicated that every animal immunized with peptide A was functionally sterile before the end of the experiment. Prostate weights (Fig. 8) parallel the results obtained with the testes, i.e. peptide A immunization produced a significant atrophy of the prostate. By any measurement, no significant effect was observed through immunization with LHRH alone, demonstrating that linking promiscuous helper T cell epitopes to poor immunogens provides a means of stimulating a strong immune response to those immunogens.

**Conclusions:**

1. The HBs $T_h$ epitope induced potent antibody responses to LHRH.
2. Antibody to peptide A efficiently neutralized LHRH activity in vaccinated animals.
3. LHRH inhibition was sufficient to reduce serum testosterone to castration levels.
4. Immunization with peptide A produced the desired biological effects, i.e. dramatic shrinkage of the prostate and testis.

EXAMPLE 5A

Identification and Testing of Additional Efficacious Th: LHRH Constructs

The peptide A results have been reproduced consistently in a number of different studies with an aggregate efficiency (organ weight reduction used as the endpoint) exceeding 95%. However, to establish a system that reliably measured the relative efficacy, or lack thereof, of different "$T_h$ epitope:LHRH" constructs, we modified the immunization protocol. The initial experiments with the LHRH constructs fell into two distinct groups when evaluated by the experimental protocol described in Example 5 (i.e. intramuscular administration of Freund's adjuvant formulations). The constructs either lacked efficacy and did not cause any significant organ weight reduction, or were totally effective and mimicked the results for peptide A, making it impossible to establish the rank order of the efficacious candidates. Thus, a simple modification of the protocol described above, i.e. subcutaneous as opposed to intramuscular administration of the candidate peptide formulations, allowed a determination of rank order. For example, subcutaneous administration of peptide A in FCA/IFA mitigated the responses to this peptide such that approximately 30%, as opposed to greater than 95%, of the animals responded sufficiently to cause shrinkage of their testes and prostates.

Accordingly, equimolar amounts of different $T_h$: LHRH constructs (equivalent to 100 µg of peptide A) were formulated as above, but administered subcutaneously at 0, 3 and 6 weeks. The sequences of the tested peptides are provided in Table 5 and the results from several different experiments are compiled in Table 6. In each study, peptide A was included as a positive control to normalize data between different experiments. As shown, peptides which elicited significant anti-LHRH antibody titers caused the serum testosterone levels of immunized animals to drop to below castration levels and caused significant reduction in testis weights. The results from the experiments conducted to produce Table 6 are provided in the following Examples.

EXAMPLE 6

Efficacy of Peptide 18, an HBsAg $T_h$ Epitope: LHRH Construct Containing a Glycine Spacer

Peptide 18 is a 30 amino acid residue synthetic peptide which is organized in four linear domains, from the amino-to the carboxyl- terminus, as follows: 3 lysine residues ($K_3$), the hepatitis B virus helper epitope$_{19-33}$ (HBsAg $T_h$), a glycine spacer (GG), and LHRH. Peptide 18 is represented as $K_3$: HBsAg $T_h$: GG: LHRH. Thus, the structure of peptide 18 differs from peptide A simply by the addition of the Gly-Gly spacer sequence between the helper epitope and LHRH. The following describes analysis of the efficacy of peptide 18 when formulated in Freund's adjuvant and administered subcutaneously. The experimental design is the same as in Example 5 except as indicated otherwise.

**Experimental Design:**

| Immunogen | peptide A or peptide 18 (i.e., in separate groups) |
|---|---|
| Dose | 100 µg of peptide A, peptide 18 at molar equivalent to 100 µg of peptide A |
| Route | subcutaneous |
| Adjuvant | Freund's complete/incomplete |
| Species | 6 sexually mature Sprague-Dawley male rats/group |

**Results:**

Two weeks following the second booster immunization (i.e. at 8 weeks), 6 of 6 animals receiving peptide 18 expressed anti-LHRH antibody titers greater than 1 nmole/L (Fig. 9). These high levels of antibodies were maintained in all animals until the termination of the experiment (week 10). In contrast, only 2 of 6 animals immunized with peptide A, expressed anti-LHRH antibody titers greater than 1 nmole/L by week 10 (Fig. 10). The differences in LHRH-specific antibody titers between the two groups were also reflected in the levels of circulating testosterone present in these animals. By week 10 (when animals were sacrificed), 5 of 6 animals receiving peptide 18 expressed serum testosterone at castration levels (Fig. 11), while 1 of 6 animals receiving peptide A had castration levels of this hormone (Fig. 12). Dissection of organs at week 10 demonstrated that 5 of 6 animals receiving peptide 18 had significantly atrophied prostate glands (Fig. 13), while only 1 of 6 animals receiving peptide A exhibited shrunken prostates.

**Conclusions:**

1. Peptide 18 was effective in eliciting the desired biological responses, i.e. expression of LHRH-specific antibody, reduction in serum testosterone and relevant organ atrophy.
2. Insertion of the Gly-Gly spacer sequence between the $T_h$ epitope and LHRH improved the immune response to the peptide, as seen by comparison of the results from peptide 18 with those from peptide A.

EXAMPLE 7

Efficacy of Peptide 19, a Measles Virus Promiscuous $T_h$ Epitope: LHRH Construct

A 15 residue domain from the measles virus (MV) F glycoprotein was linked to the LHRH sequence by automated synthesis to produce peptide 19. Peptide 19 is organized in three linear domains, from the amino- to the carboxyl-terminus, as follows: the measles virus helper epitope ($MVF_1 T_h$), a glycine spacer (GG) and LHRH. Peptide 19 is thus represented as $MVF_1 T_h$: GG: LHRH. This peptide was formulated in Freund's adjuvant and administered subcutaneously as described below. The experimental design is the same as in Example 5 except as indicated otherwise.

**Experimental Design:**

| Immunogen | peptide 19 |
|---|---|
| Dose | molar equivalents to 100 µg of peptide A |
| Route | subcutaneous |
| Adjuvant | Freund's complete/incomplete |
| Species | 6 sexually mature Sprague-Dawley male rats/group |

**Results:**

Two weeks following administration of the second booster immunization (at 8 wks), significant LHRH-specific antibody titers were observed in 4 of the 6 animals immunized (Fig. 14). There was a modest increase in the LHRH antibody titers between weeks 8 and 10, and in addition, one of the initially non-responding animals (rat #726) began to express significant anti-LHRH antibody during this period. Fig. 15 again demonstrates the strong positive correlation between the presence of significant LHRH antibodies and the reduction of serum testosterone. The four animals expressing anti-LHRH titers greater than 2 nmole/L at week 8 had serum testosterone levels below 0.5 nmole/L by week 8, and these levels were maintained through week 10 (Fig. 15a). The remaining animals which had lower LHRH antibody titers appeared to have reduced testosterone levels, but not to castration levels (Fig. 15b). The significant reduction in serum testosterone to below castration levels caused the expected severe atrophy of the testis as demonstrated by Fig. 16. An essentially identical result for prostate atrophy was observed as well (Fig. 17). For Peptide 19 greater than 65% of the animals tested exhibited castration levels of testosterone and severe atrophy of the testis and prostate gland (in this "modified" protocol. When given intramuscularly, according to the protocol in Example 5, greater than 95% of the animals exhibit relevant organ atrophy by 10 weeks. The accumulated data for peptide 19 show that LHRH antibody titers of greater than 2 nmole/L will cause serum testosterone to fall to castration levels (below 0.5 nmole/L) which results in atrophy of both the testis and prostate gland. LHRH-specific antibody titers must be elevated for 1-2 weeks for it to have the desired effect, namely organ atrophy. Based upon this, it is likely that rat #726 would have achieved castration levels of testosterone if the study was extended beyond 10 weeks duration.

Testis weight reduction is a logical endpoint for screening experiments because testis atrophy is an absolute predictor of prostate gland atrophy: Prostate shrinkage precedes reduction in testis weight (i.e., the prostate gland is heavily dependent upon testosterone for its maintenance, thus elimination of serum testosterone causes rapid prostate gland shrinkage, which is only then followed by testis atrophy); testis removal is trivial relative to the complicated dissection required for removal of the prostate and associated seminal vesicles; and, the simple form of the testis relative to the prostate and seminal vesicles make testis weight measurements more accurate.

**Conclusions:**

1. Peptide 19 is efficacious (i.e. produces significant reduction in serum testosterone, plus testis and prostate weights) when administered with Freund's adjuvant.
2. Subcutaneous administration of the peptide formulation allows the means of ranking immunogen efficacy.
3. Peptide 19 has better efficacy than peptide A.

EXAMPLE 8

Efficacy of Peptide K, a Pertussis Toxin Promiscuous $T_h$ Epitope: LHRH Construct

A 24 residue long $T_h$ epitope from pertussis toxin was linked to LHRH through automated synthesis, to form peptide K. This peptide is organized into two linear domains, from the amino- to the carboxyl- terminus, as follows: the pertussis toxin helper epitope $T_h2$ ($PT_2\,T_h$), and LHRH. Peptide K is thus represented as $PT_2\,T_h$: LHRH. This peptide was tested for efficacy using the same protocol as described for the analysis of peptides 18 and 19 (Examples 6 and 7, above). The experimental design is the same as in Example 5 except as indicated otherwise.

**Experimental Design:**

| Immunogen | peptide K |
|---|---|
| Dose | molar equivalent to 100 µg of peptide A |
| Route | subcutaneous |
| Adjuvant | Freund's complete/incomplete |
| Species | 6 sexually mature Sprague-Dawley male rats/group |
| Necropsy | at 10 weeks determine testis weights |

**Results:**

Fig. 18 describes the LHRH-specific antibody titers expressed in animals given peptide K <u>subcutaneously</u>. Two animals exhibited significant LHRH-specific antibody titers (greater than 4 nmole/L) by week 8, two intermediate levels (1.5-2.0 nmole/L) and two animals exhibited essentially no response. Again, there was the expected correlation of anti-LHRH titers with serum testosterone levels. The two animals with high antibody titers had serum testosterone at castration levels by week 8, which remained at that level until the termination of the experiment (Fig. 19a). Rat #793 expressed LHRH antibody titers of greater than 2 nmole/L at week 10 and had castration levels of testosterone at that point. Rat #791 which had LHRH antibody titers of 1.6 nmole/L measured at week 10 (Fig. 18) had testosterone levels approaching the limit for castration at that time (Fig. 19b). Animals expressing high levels of LHRH antibodies (Fig. 18) had significantly atrophied testes at 10 weeks (Fig. 20). Rat #791 showed some reduction in testis weights, and based on the kinetics of serum testosterone levels, it is very probable that organ atrophy would have been significant if necropsy was conducted after week 11.

The variability in the responses to peptide K most probably reflects genetic differences within the outbred rat population used for this study, and define differences between animals in their ability to effectively recognize the $T_h$ epitope contained within this LHRH construct. This result supports the use of mixtures of constructs, containing different promiscuous $T_h$ epitopes to produce uniform potent responses in populations expressing diverse HLA haplotypes.

**Conclusions:**

1. Peptide K is efficacious (i.e. produces significant reduction in serum testosterone and testis weight size) when administered with Freund's adjuvant.
2. Subcutaneous administration of the peptide formulation provides the means of ranking immunogen efficacy.
3. Promiscuous $T_h$ constructs are capable of differing degrees of efficacy when viewed in genetically heterogeneous populations.
4. Peptide K has an efficacy approximates that achieved by peptide A.

EXAMPLE 9

Efficacy of Peptide H, a Tetanus Toxin Promiscuous $T_h$ Epitope: LHRH Construct

A 27 amino acid long peptide, consisting of a 15 amino acid $T_h$ epitope from tetanus toxin located near the amino-terminus and followed by the LHRH sequence, was synthesized using the standard automated synthesis techniques. This peptide, peptide H, is organized in three linear domains, from the amino- to the carboxyl- terminus, as follows: 2 lysine residues ($K_2$), the tetanus toxin helper T cell epitope 1 ($TT_1$ $T_h$) and LHRH. Peptide H is thus represented as $K_2$: $TT_1$ $T_h$: LHRH. The following describes analysis of the efficacy of peptide H when formulated in Freund's adjuvant and administered <u>subcutaneously.</u> The experimental design is the same as in Example 5 except as indicated otherwise.

**Experimental Design:**

| Immunogen | peptide H |
|---|---|
| Dose | molar equivalent to 100 µg of peptide A |
| Route | subcutaneous |
| Adjuvant | Freund's complete/incomplete |
| Species | 5 sexually mature Sprague-Dawley male rats/group |
| Necropsy | at 10 weeks determine testis weights |

**Results:**

Fig. 17 demonstrates the capacity of peptide H to cause the production of LHRH-specific antibodies. By week 8 (two weeks after the second booster administration) 4 of 5 animals express anti-LHRH antibody titers greater than 2.0 nmole/L. By week 10, the fifth animal expressed LHRH antibodies to greater than 1.0 nmole/L. At week 8, there was a significant reduction in serum testosterone levels in all animals, and by week 10, serum testosterone was at castration levels in all animals (Fig. 22). At week 10, animals were sacrificed and the relevant organs weighed. Four of 5 rats exhibited dramatically atrophied testes (Fig. 23). The fifth animal, rat #103, exhibited significantly reduced testes, but were slightly larger than the testes from the other animals receiving peptide H. This correlates with the lower levels of anti-LHRH antibodies expressed by this animal (Fig. 21).

**Conclusions:**

1. Peptide H is efficacious (i.e. produces significant reduction in serum testosterone, plus testis and prostate weights) when administered with Freund's adjuvant.
2. Subcutaneous administration of the peptide formulation provides the means of ranking immunogen efficacy.
3. Peptide H is significantly more efficacious than peptide A.

EXAMPLE 10

Immunogen Cocktail Administered in Freund's Adjuvant

Establishing the relative efficacies of the many different $T_h$ epitope: LHRH constructs (Examples 5-10; Table 6) permits selection of representative peptides for a cocktail of immunogens. Individual constructs carrying $T_h$ from measles virus F, hepatitis B surface antigen, tetanus toxin and pertussis toxin in the immunogen cocktail have demonstrated efficacy (Table 6) and are promiscuous, providing maximum coverage in a genetically diverse population. Moreover, because these $T_h$ epitopes are present in previously administered vaccines, they provide the potential for priming responses. The experiments below show the the rapid atrophy of the relevant organs using a cocktail approach. The experimental design is the same as in Example 5 except as indicated otherwise.

**Experimental Design:**

| Immunogen | Cocktail (HBs $T_h$:LHRH + $MV_{F1}T_h$:LHRH + $PT_2$ $T_h$:LHRH + $TT_1T_h$:LHRH) |
|---|---|
| Dose | molar equival. of each, to equal 100 µg of peptide A |
| Route | subcutaneous |
| Adjuvant | Freund's complete/incomplete |
| Species | 6 sexually mature Sprague-Dawley male rats/group |

(continued)

| Necropsy | at 10 weeks determine testis weights |
|---|---|

**Results:**

The mixture of $T_h$: LHRH constructs was administered <u>subcutaneously</u> in Freund's adjuvant by the methods described in Examples 6-9, above. As demonstrated by Fig. 24, rapid and potent anti-LHRH antibody responses were observed in all animals. By week 5, i.e. two weeks after the first booster immunization and prior to the second booster, 5 of 6 animals expressed LHRH specific antibody titers sufficient to reduce serum testosterone levels below that required for castration. Significant increase in antibody titers was achieved for all animals following the second booster immunization. Serum testosterone levels fell below the threshold required for castration between 5 and 8 weeks following the primary immunization and remained at these levels for the remainder of the experiment (Fig. 25). This marked decrease in serum testosterone caused significant atrophy in the testes for every animal measured at week 10 (Fig. 26). This experiment demonstrates the advantages provided by the cocktail of immunogens (compare Fig. 26 with Figs. 16, 20 & 23). The desired endpoint is achieved in all animals as opposed to a few. In addition to the uniformity of responses, the rapidity of the responses and their intensity were enhanced when the cocktail was administered in lieu of the individual components (compare Fig. 24 with Figs. 14, 18 & 21).

**Conclusions:**

1. A cocktail of $T_h$: LHRH immunogens is more efficacious than any individual peptide within the mixture.
2. A cocktail of immunogens is fully effective (greater than 95% of the animals exhibiting the desired characteristics) in producing the desired effect, i.e. relevant organ atrophy.

EXAMPLE 11

Immunogen Cocktail Formulated on Alum

For a human prostate cancer therapy, it is necessary to achieve similar levels of organ weight reduction using a vaccine formulation acceptable for use in humans. Therefore, the efficacy of a cocktail of $T_h$: LHRH constructs adjuvanted with aluminum hydroxide was tested. The following is a summary of that experiment. The experimental design is the same as in Example 5 except as indicated otherwise.

**Experimental Design:**

| Immunogen | Cocktail (HBs $T_h$:LHRH + MV$_{F1}$T$_h$:LHRH + PT$_2$ T$_h$:LHRH + TT$_1$T$_h$:LHRH) |
|---|---|
| Dose | 250 µg, molar equivalent of each |
| Route | intramuscular |
| Adjuvant | aluminum hydroxide |
| Schedule | week 0, 2 and 4 weeks |
| Species | 5 sexually mature Sprague-Dawley male rats/group |
| Necropsy | at 10 weeks determine testis weights |

**Results:**

At 8 weeks following the initiation of the experiment, significant LHRH-specific antibody titers were observed in all animals, three animals expressed titers above 2 nmole/L and two had titers between 1.5 and 2.0 nmole/L (Fig. 27). By week 10, 4 of 5 animals exhibited LHRH antibody titers above the 2 nmole/L. At this time, point 4 of 5 animals exhibited castration levels of serum testosterone (Fig. 28) and the same four animals had significantly atrophied prostate glands (Fig. 29). The fifth animal, #231, exhibited a marked, though incomplete, prostate weight reduction when compared to the other animals in the group. Its prostate weight is consistent with reduced, though measurable, levels of serum testosterone in this animal at the end of the experiment. This is the first report ever described where the desired biologic effect (namely, elimination of serum testosterone and significant prostate gland atrophy) was produced through immunization with LHRH constructs on alum. In all other cases thus far described in the literature, attempts to use alum with LHRH-based immunogens have failed, requiring the use of reactogenic formulations (e.g. Freund's adjuvant), to produce the desired effects.

The reduced efficacy of the alum-based formulation (Fig. 28), when compared to the same immunogen cocktail in Freund's adjuvant (Fig. 25), manifested as a delay in the timing of the desired responses. This is demonstrated by rat #228 (Fig. 29) which had an atrophied prostate gland, but normal testes weights at week 10. It is probable that this animal would have expressed shrunken testes if the experiment were to have continued beyond 10 weeks. In contrast, every animal receiving the Freund's adjuvan t-based formulation exhibited atrophied testes by week 10 (Fig. 26).

**Conclusions:**

1. Mixing promiscuous $T_h$: LHRH synthetic peptide constructs provides an efficacious LHRH immunotherapeutic vaccine.
2. This immunogen cocktail can be formulated with alum (one of the very few and most safe adjuvants approved for human use) and obtain the required biological effects, i.e. atrophy of the relevant organs.

EXAMPLE 12

Efficacy of an Artificial $T_h$ Epitope SSAL: LHRH Construct

Peptide 38 (also represented as peptide SSAL1) is a peptide library in which a degenerate $T_h$ sequence, modeled after the measles virus $F_1$ $T_h$ epitope, is linked to LHRH. This peptide is organized in three linear domains, from the amino- to the carboxyl- terminus, as follows: the structured synthetic antigen library representing a synthetic helper T cell epitope (SSAL $T_h$), a glycine spacer (GG), and LHRH. Peptide 38 may therefore be represented as SSAL1 $T_h$: GG: LHRH, and is analogous to peptide 19 (i.e. MVF$_1$ $T_h$: GG: LHRH).

The sequence of peptide 38 is as provided in Table 5. Peptide SSAL1; SSAL $T_h$1:GG: LHRH (SSAL $T_h$1MV$_{F1}$ $T_h$ Derivative).

This peptide library is composed of a mixture of approximately 5.24 x $10^5$ different sequences, where the precise measles virus $T_h$1 epitope is represented in only one of these sequences. The Gly spacer and LHRH are invariant in the library sequences.

The degenerate helper T cell epitope present in peptide SSAL1 is modeled after a promiscuous helper T cell epitope identified from the F protein of measles virus represented by residues 288-302 of the F protein and has the following amino acid sequence, LSEIKGVIVHRLEGV. The library sequence was constructed using this sequence as a template. Charged residues Glu (E) and Asp (D) were added at position 1 to increase the charge surrounding the hydrophobic face of the amphipathic helical epitope. This face is made up of residues at positions 2, 5, 8, 9, 10, 13 and 16. The hydrophobic residues commonly associated with promiscuous epitopes were added at these positions. A Rothbard sequence is present at residues 6-10 in the prototype sequence and its character is maintained throughout all sequences within the library. Sequences obeying the 1, 4, 5, 8 rule begin at residue 5 of the prototype sequence and are maintained in all sequences as well.

Peptide 38 was prepared by chemical synthesis using standard techniques well known in the art such as the solid-phase synthetic route pioneered by Merrifield. The coupling of multiple amino acids at a given position is accomplished by providing a mixture of the desired amino acids at the appropriate ratios as indicated in the formula. For example, at positions 2, 5, 8, 9, 10, 13, and 15 from the N-terminus, an equimolar amount of protected $N^\alpha$-amino group, Leu (L), Ile(I), Val(V) and Phe(F), instead of a single protected amino acid, was used for each of the corresponding coupling steps. If necessary the ratio of amino acids in the mixture can be varied to account for different coupling efficiency of those amino acids. At the end of the synthesis, the peptide libraries were cleaved individually according to standard procedures to release the free peptide mixtures. The experimental design is the same as in Example 5 except as indicated otherwise.

**Experimental Design:**

| Immunogen | peptide 38 or peptide 19 (in separate groups) |
|---|---|

(continued)

| Dose | 400 µg of each peptide |
|---|---|
| Route | intramuscular |
| Adjuvant | Incomplete Freund's |
| Schedule | week 0 (FCA), 3 and 6 weeks (IFA) |
| Species | 5 sexually mature Sprague-Dawley male rats/group |
| Necropsy | at 10 weeks determine testis weights |

**Results:**

Six weeks following the commencement of the experiment (i.e. 2 weeks after the first booster immunization and immediately prior to the second booster), 4 of 5 animals receiving peptide 38 expressed serum testosterone at castration levels. At 8 weeks, serum testosterone was at castration levels in 5 of 5 animals. Palpation of the testes at that time demonstrated that the 4 animals having negligible serum testosterone at week 6 also have atrophied organs. In contrast, only 1 of 5 animals immunized with peptide 19 expressed castration levels of serum testosterone by week 6, the remainder were in the normal range, and this number did not change by week 8. By week 8, the animal receiving peptide 19 which had negligible levels of testosterone at week 6, had atrophied testes by palpation.

**Conclusions:**

1. The $T_h$ epitope library has shown significant efficacy by causing reduction of serum testosterone to castration levels in all animals receiving peptide 38.
2. The $T_h$ epitope library peptide has provided what a single peptide immunogen composed of a promiscuous $T_h$ epitope linked to LHRH cannot provide, i.e. comprehensive efficacy in all members of an outbred population.

EXAMPLE 13

Further Modification of the LHRH Immunogens to Amplify Antibody Induction: Addition of an Invasin Domain

T cell activation can also be brought about by LHRH that is covalently linked to a specific fragment from the invasin protein of the pathogenic bacteria Yersinia spp. Peptide 32, in which a domain of the invasin protein is linked to the HBs $T_h$ epitope: LHRH construct (i.e. $Inv_{718-732}$ + peptide 18) has been synthesized. Peptide 32 is organized in five linear domains, from the amino- to the carboxyl-terminus, as follows: the invasin T cell stimulator (Inv), a glycine spacer (GG), the hepatitis B surface antigen helper T cell epitope (HBsAg $T_h$1), a glycine spacer (GG), and LHRH. Peptide 32 is thus represented as: Inv: GG: HBsAg $T_h$1: GG: LHRH. The following provides a specific example of the significant efficacy imparted to the LHRH immunogen by the addition of the invasin domain. The experimental design is the same as in Example 5 except as indicated otherwise.

**Experimental Design:**

| Immunogen | peptide 32 |
|---|---|
| Dose | 100 µg, per dose |
| Route | subcutaneous |
| Adjuvant | aluminum hydroxide |
| Species | 5 sexually mature Sprague-Dawley male rats/group |
| Necropsy | at 10 weeks determine testis weights |

**Results:**

Fig. 30 describes the LHRH-specific antibody titers produced in rats immunized with peptide 32. Significant titers were achieved after the first booster immunization (at 3 weeks) which continued to increase following the second booster immunization at 6 weeks. By week 8, 4 of 5 animals exhibited LHRH antibody titers above 2 nmole/L. Control animals immunized with an $Inv_{718-732}$: LHRH construct, lacking a $T_h$ epitope, did not produce any measurable LHRH-specific antibody. Serum testosterone levels (Fig. 31) fell precipitously in the animals responding to peptide 32, and by week 8, testosterone levels were below the threshold for castration. Serum testosterone in these animals remained unmeasurable for the remainder of the experiment. As demonstrated by Fig. 32, dramatic organ atrophy was achieved in the four responding animals. The testes of control animals immunized with peptide 18 (HBs $T_h$: GG: LHRH; lacking the invasin epitope) were unaffected at the end of this experiment (i.e. at week 10). This result is especially important since the invasin-containing LHRH peptide was formulated on alum and administered subcutaneously. Previous studies with LHRH linked to high molecular weight carrier molecules, e.g. tetanus and diphtheria toxins, required formulation with Freund's complete adjuvant or other reactogenic adjuvants to achieve any significant degree of efficacy.

**Conclusions:**

1. The invasin fragment provides a significant improvement in the immune responses to $T_h$: LHRH constructs.
2. Alum was a sufficient adjuvant for peptide 32.
3. Peptide 32 is capable of causing organ atrophy.

EXAMPLE 14

Efficacy of an LHRH Immunogen Cocktail Containing Peptide 32

An experiment testing the efficacy of the cocktail of immunogens as described in Examples 10 and 11, was conducted except that the HBs $T_h$: GG: LHRH construct was replaced with peptide 32. The protocol for this example is identical to that used in Example 11. As above, animals received 100 µg of peptide on alum, administered at 0, 3 & 6 weeks. As demonstrated by Fig. 33, rapid and potent anti-LHRH antibody responses were produced in response to immunization with the Invasin fragment-containing cocktail when formulated on alum. By 8 weeks, 6 of 6 animals receiving the peptide 32-containing cocktail expressed serum testosterone levels (Fig. 34) below the castration threshold (i.e. less than 0.5 nmole/L). In contrast, 4 of 6 animals receiving an equivalent dose of peptide 32 alone on alum had castration levels of testosterone. These data suggest that any genetic variability associated with responses to the invasin fragment are overcome by its presentation in the cocktail containing the different $T_h$ constructs. Fig. 35 describes the testis weights at the end of the experiment (at 10 weeks). Five of 5 animals receiving the peptide 32-containing cocktail of immunogens exhibited significant organ atrophy and by histological examination were functionally sterile.

$Invasin_{718-732}$ linked to: HBs $T_h$:GG:LHRH generates peptide 32, to $MV_{F1}T_h$:GG:LHRH generates peptide 33, to $PT_2T_h$:GG:LHRH generates peptide 34, to $TT_1T_h$:GG:LHRH generates peptide 35, to $TT_4T_h$:GG:LHRH generates peptide 36, and to $TT_5T_h$:GG:LHRH generates peptide 37. Experiments designed to evaluate the efficacy of peptides 32-37, alone and in combination, are conducted in accordance with this and Example 13.

EXAMPLE 16

Delivery of Peptide A in Microparticles

Efficient immune responses occur when an LHRH immunogen is entrapped microparticles of 10 µm or less were delivered subcutaneously or intramuscularly. These small microparticles were efficiently taken up by macrophages allowing for effective antigen presentation.

Microparticles containing peptide A were prepared with a poly(lactide-co-glycolide) copolymer as described in U.S. Series No. 201524, filed Feb. 25, 1994.

A sterile (water-in-oil)-in-water emulsion was prepared as follows: an aqueous solution of 1.5% w/w synthetic peptide A was prepared by passing the peptide solution through a sterile 0.2 µm filter. Polymer was dissolved in dichloromethane at a concentration of 4.0% w/w; 200 g of this solution was added to 20 g of peptide solution and mixed with a homogenizer (Model STD 1, Silverson Machiens, East Longmeadow, MA, 1" tubular head, 13,000 rpm, 4 min). After the water-in-oil emulsion was formed, 600 g of a 10% w/w solution of polyvinyl alcohol was added thereto and mixed with a homogenizer (13,000 rpm, 6 min). A stable (water-in-oil)-in-water emulsion formed and was transfered to a 2 L filtration flask. The dichloromethane was evaporated with stirring by a magnetic stir bar for 16 h under ambient conditions. Sterile air was introduced into the evaporation flask through a 0.2 µm filter and a gas dispersion tube placed 4 cm above the emulsion. As the dichloromethane evaporated, it was removed from the flask by the stream of air vented through a side arm on the flask. The air/dichloromethane mixture was passed into a dry ice-acetone cold trap to condense the dichloromethane. The evaporation of the dichloromethane was complete after 16 h and discrete particles had formed. The particles were recovered from the polyvinyl alcohol solution by centrifugation, washing with sterile water and lyophilized for 24 hours to provide a dry powder. Theparticle size is less than 10 µm. Immune responses to microparticulate peptide A was evaluated in rats in an experiment described below and summarized in Table 7. The experimental design is the same as in Example 5 except as indicated otherwise.

**Experimental Design:**

| Immunogen | peptide A (HBsAg $T_h$: LHRH, without spacer) in rapid-release microparticles (1: 1, polylactide:co-glycolide) |
|---|---|
| Dose | 100 µg of peptide A per dose |
| Route | subcutaneous |
| Adjuvant | the experimental variable |
| Species | 6 sexually mature Sprague-Dawley male rats/group |
| Necropsy | at 10 weeks determine testis weights |

**Results:**

Microparticulate peptide A caused significant LHRH-specific antibody production and dramatic atrophy of the testes in 2 of 6 immunized animals. When an equivalent dose of peptide A formulated on alum was administered in an identical manner, none of the animals exhibited significant organ weight reduction. Thus, microparticles were more efficient than alum in causing the desired effects, i.e. elevated LHRH-specific antibody titers, elimination of serum testosterone and organ atrophy. Microparticle delivery compares favorably with the efficacy exhibited by the delivery of soluble peptide A in Freund's adjuvant, which caused organ atrophy in 3 of 6 animals. By comparison, as demonstrated in Example 6, the simple addition of glycine spacer sequences (found in peptide 18) to the HBsAg $T_h$: LHRH construct significantly improved immunogenicity; 6 of 6 animals given peptide 18 in FCA/IFA had atrophied testes.

The effects of mixing peptide A loaded microparticles in various adjuvant/emulsion formulations was examined. As can be seen in Table 7, certain formulations including Liposyn + Saponin and Squalene + L121 (4 of 6 animals in each group had atrophied testes) appear to improve the immune responses elicited by microparticulate peptide A. Liposyn is a soy bean oil and safflower oil emulsion prepared for intravenous feeding of humans, saponin is a water soluble extract of Quil A, squalene is a metabolizable animal oil previously tested in humans as a vaccine carrier and L121 is a triblock polymer which has proven efficacious in human cancer therapy trials. 100% efficacy was achieved with peptide A-loaded microparticles formulated in Emulsigen + Saponin. Emulsigen is an adjuvant approved for use in food animals, and this formulation (i.e. immunogen cocktail in Emulsigen + saponin) can be used in a pet contraception vaccine or for the treatment of boar taint.

Polylactide-co-glycolyde microparticles containing an immunogen cocktail are formulated and tested for immune potency in accordance with this example.

EXAMPLE 17

Efficacy of $T_h$: LHRH Delivered in Emulsions

$T_h$ epitope: LHRH immunogens have been identified and ranked in order of their effectiveness (Table 6) using a standard Freund's complete/incomplete immunization protocol. These results have permitted the selection of a number of different $T_h$ constructs for formulation into a cocktail of immunogens. This cocktail, coupled to alum, has demonstrated significant efficacy (Example 11). The addition of the invasin domain has enhanced the immunogenicity of the subject LHRH constructs, such that a single invasin epitope-bearing peptide demonstrates significant efficacy when administered on alum (Example 13). The invasin-containing construct also elicits exceptional and uniform responses when it is a component of an immunogen cocktail (Example 14).

In addition to microparticle delivery of immunotherapeutic immunogens in emulsion formulations, adjuvant/emulsion-based formulations of soluble immunogen have been evaluated. Again, peptide A was used to provide a means of comparing the relative efficacies of the different formulations. A representation of the different adjuvant/emulsion combinations that have been evaluated are listed in Table 8. Table 8 indicates which adjuvant/emulsion combinations are suitable for human or animal use. Some of the more reactogenic adjuvants (e.g. Freund's incomplete) approved for use in cancer patients were included. Animals were immunized at 0, 3 and 6 weeks with 100 µg of peptide A in the indicated formulations administered subcutaneously. Significant efficacy, as good or better than that achieved with Freund's complete adjuvant was obtained with some of these formulations, e.g. Emulsigen + L121 and ISA 720.

EXAMPLE 18

Efficacy of the Invasin Containing-peptide Cocktail in Unique Emulsion Formulations

The adjuvant formulations which improved the efficacy of peptide A when compared to an alum-based formulation, e.g. IFA, ISA 720, ISA 51, Detox, Liposyn + Avridine, squalene + L121, MPL + TDE, Emulsigen + DDA, and Emulsigen + L121 were then used to prepare the peptide 32-containing cocktail described in Example 14. The results testing the effectiveness of these different formulations are summarized in Table 9. Significant efficacy (measured by serum testosterone levels below the threshold for castration at 8 weeks for 100% of the animals, and atrophied testes in 100% of the animals at week 10) was observed for several of the adjuvants. These findings demonstrate the power of combining a potent immunogen, namely a $T_h$ epitope: LHRH cocktail containing an Invasin domain with efficacious and safe emulsion formulations.

## TABLE 1

### Amino Acid Sequence of Peptides A-E

| Peptide | SEQ ID NO. | Amino Acid Sequence |
|---|---|---|
| A | 10 | Lys-Lys-Lys-Phe-Phe-Leu-Leu-Thr-Arg-Ile-Leu-Thr-Ile-Pro-Gln-Ser-Leu-Asp-Glu-His-Trp-Ser-Tyr-Gly-Leu-Arg-Pro-Gly |
| B | - | [Glu-His-Trp-Ser-Tyr-Gly-Leu-Arg-Pro-Gly-Glu-His-Trp-Ser-Tyr-Gly-Leu-Arg-Pro-Gly-]$_8$-Lys$_4$-Lys$_2$-Lys-Phe-Phe-Leu-Leu-Thr-Arg-Ile-Leu-Thr-Ile-Pro-Gln-Ser-Leu-Asp |
| C | - | [Glu-His-Trp-Ser-Tyr-Gly-Leu-Arg-Pro-Gly- Glu-His-Trp-Ser-Tyr-Gly-Leu-Arg-Pro-Gly- Glu-His-Trp-Ser-Tyr-Gly-Leu-Arg-Pro-Gly-]$_8$-Lys$_4$-Lys$_2$-Lys-Phe-Phe-Leu-Leu-Thr-Arg-Ile-Leu-Thr-Ile-Pro-Gln-Ser-Leu-Asp |
| D | - | [Glu-His-Trp-Ser-Tyr-Gly-Leu-Arg-Pro-Gly- Phe-Phe-Leu-Leu-Thr-Arg-Ile-Leu-Thr-Ile-Pro-Gln-Ser-Leu-Asp-Met]$_8$-Lys$_4$-Lys$_2$-Lys-Ala-Ala |
| E | - | [Glu-His-Trp-Ser-Tyr-Gly-Leu-Arg-Pro-Gly- Glu-His-Trp-Ser-Tyr-Gly-Leu-Arg-Pro-Gly- Phe-Phe-Leu-Leu-Thr-Arg-Ile-Leu-Thr-Ile-Pro-Gln-Ser-Leu-Asp-Met]$_8$-Lys$_4$-Lys$_2$-Lys-Ala-Ala |

| Immunogenicity and Therapeutic Effect after Immunization with Peptides A-E in Rats | | | | |
|---|---|---|---|---|
| Peptide | α-LHRH[a] | Testosterone[a] | Testes[b] | P+SV[c] |
| A | 3.93 | <0.01 | 0.4 | 0.2 |
|   | 2.55 | <0.01 | 0.4 | 0.3 |
|   | 2.06 | <0.01 | 0.6 | 0.2 |
|   | 0.72 | 5.3 | 1.8 | 1.8 |
|   | 0.42 | 2.1 | 1.7 | 1.8 |
| B | 0.53 | 14.0 | 1.7 | 1.7 |
|   | 0.51 | 16.5 | 1.7 | 1.6 |
|   | 0.49 | 12.6 | 1.7 | 1.6 |
|   | 0.45 | 4.6 | 1.6 | 1.7 |
|   | 0.42 | 10.5 | 1.7 | 2.2 |
| C | 0.78 | 5.6 | 1.6 | 2.3 |
|   | 0.45 | 12.3 | 1.8 | 1.6 |
|   | 0.41 | 3.9 | 2.1 | 1.6 |
|   | 0.41 | 5.3 | 1.6 | 1.8 |
|   | 0.39 | 11.2 | 1.7 | 1.8 |
| D | 1.44 | 2.6 | 1.7 | 2.1 |
|   | 0.44 | 3.6 | 1.7 | 1.3 |
|   | 0.43 | 2.3 | 1.6 | 2.0 |
|   | 0.39 | 2.1 | 1.7 | 1.6 |
|   | 0.39 | 2.8 | 1.4 | 2.1 |
| E | 1.69 | <0.01 | 1.4 | 2.0 |
|   | 0.66 | 0.9 | 1.5 | 1.9 |
|   | 0.51 | 3.3 | 1.2 | 1.9 |
|   | 0.40 | 4.0 | 1.6 | 2.0 |
|   | 0.40 | 13.9 | 1.3 | 0.9 |

[a] nmol/L

[b] Weight of testes in g

[c] Weight of prostate and seminal vesicles (P+SV) in g

TABLE 3

| Average Anti-LHRH Titers and Androgen-Dependent Organ Weights in Rats Immunized with Peptides A-E | | | | |
|---|---|---|---|---|
| Peptide[a] | α-LHRH (nmol/L) | Testes (g) | Epid[b] (g) | P+SV (g) |
| A | 1.94 | 1.0 | 0.4 | 0.9 |
| B | 0.48 | 1.7 | 0.6 | 1.8 |
| C | 0.49 | 1.8 | 0.6 | 1.8 |
| D | 0.62 | 1.6 | 0.6 | 1.8 |
| E | 0.73 | 1.4 | 0.6 | 1.7 |
| Control | 0.45 | 1.6 | 0.7 | 2.0 |

[a] Each peptide was injected in 5 rats.

[b] Abbreviations: Epid., epididymis; P+SV, prostate and seminal vesicles-

## TABLE 4

| Peptide | SEQ ID No. | Sequence |
|---|---|---|
| F (PT$_1$Th-LHRH) | 11 | Lys-Lys-Leu-Arg-Arg-Leu-Leu-Tyr-Met-Ile-Tyr-Met-Ser-Gly-Leu-Ala-Val-Arg-Val-His-Val-Ser-Lys-Glu-Glu-Gln-Tyr-Tyr-Asp-Tyr-Glu-His-Trp-Ser-Tyr-Gly-Leu-Arg-Pro-Gly |
| G (PT$_{1A}$Th-LHRH) | 12 | Tyr-Met-Ser-Gly-Leu-Ala-Val-Arg-Val-His-Val-Ser-Lys-Glu-Glu-Glu-His-Trp-Ser-Tyr-Gly-Leu-Arg-Pro-Gly |
| H (TT$_1$Th-LHRH) | 13 | Lys-Lys-Gln-Tyr-Ile-Lys-Ala-Asn-Ser-Lys-Phe-Ile-Gly-Ile-Thr-Glu-Leu-Glu-His-Trp-Ser-Tyr-Gly-Leu-Arg-Pro-Gly |
| I (TT$_2$Th-LHRH) | 14 | Lys-Lys-Phe-Asn-Asn-Phe-Thr-Val-Ser-Phe-Trp-Leu-Arg-Val-Pro-Lys-Val-Ser-Ala-Ser-His-Leu-Glu-His-Trp-Ser-Tyr-Gly-Leu-Arg-Pro-Gly |
| J (TT$_3$Th-LHRH) | 15 | Tyr-Asp-Pro-Asn-Tyr-Leu-Arg-Thr-Asp-Ser-Asp-Lys-Asp-Arg-Phe-Leu-Gln-Thr-Met-Val-Lys-Leu-Phe-Asn-Arg-Ile-Lys-Glu-His-Trp-Ser-Tyr-Gly-Leu-Arg-Pro-Gly |
| K (PT$_2$Th-LHRH) | 16 | Gly-Ala-Tyr-Ala-Arg-Cys-Pro-Asn-Gly-Thr-Arg-Ala-Leu-Thr-Val-Ala-Glu-Leu-Arg-Gly-Asn-Ala-Glu-Leu-Glu-His-Trp-Ser-Tyr-Gly-Leu-Arg-Pro-Gly |
| L (MV$_F$Th-LHRH) | 17 | Leu-Ser-Glu-Ile-Lys-Gly-Val-Ile-Val-His-Arg-Leu-Glu-Gly-Val-Glu-His-Trp-Ser-Tyr-Gly-Leu-Arg-Pro-Gly |

## TABLE 5

## Peptides of the Invention

| Peptide SEQ ID NO: | Sequence[a] |
|---|---|
| 18 (HB₅Tₕ-GG-LHRH) | K K K F F L L T R I L T I P Q S L D G<br>G E H W S Y<br>G L R P G |
| 19 (MV_F1 Tₕ-GG-LHRH) | L S E I K G V I V H R L E G V G G<br>E H W S Y G L R P G |
| 20 (MV_F1 Tₕ-MV_F1 Tₕ-GG-LHRH) | L S E I K G V I V H R L E G V L S E I<br>K G V I V H<br>R L E G V G G E H W S Y G L R P G |
| 21 (MV_F2 Tₕ-GG-LHRH) | G I L E S R G I K A R I T H V D T E S<br>Y G G E H W<br>S Y G L R P G |
| 22 (TT₄Tₕ-GG-LHRH) | K K W V R D I I D D F T N E S S Q K T<br>G G E H W S<br>Y G L R P G |
| 23 (TT₅Tₕ-GG-LHRH) | K K D V S T I V P Y I G P A L N I V G<br>G E H W S Y<br>G L R P G |
| 24 (CTTₕ-GG-LHRH) | A L N I W D R F D V F C T L G A T T G<br>Y L K G N S<br>G G E H W S Y G L R P G |
| 25 (DT₁Tₕ-GG-LHRH) | D S E T A D N L E K T V A A L S I L P<br>G I G C G G<br>E H W S Y G L R P G |
| 26 (DT₂Tₕ-GG-LHRH) | E E I V A Q S I A L S S L M V A Q A I<br>P L V G E L<br>V D I G F A A T N F V E S C G G E H W<br>S Y G L R P G |
| 27 (PFTₕ-GG-LHRH) | D I E K K I A K M E K A S S V F N V V<br>N S G G E H<br>W S Y G L R P G |
| 28 (SMTₕ-GG-LHRH) | K W F K T N A P N G V D E K I R I G G<br>E H W S Y G<br>L R P G |
| 29 | G L Q G K I A D A V K A K G G G E H W |

31

(TraT₁Tₕ-GG-LHRH)        S Y G L R P G

        30                   G L A A G L V G M A A D A M V E D V N
(TraT₂Tₕ-GG-LHRH)        G G E H W S Y G L R P G

| Peptide SEQ ID NO: | Sequence |
|---|---|
| 31 (TraT$_3$T$_h$-GG-LHRH) | S T E T G N Q H H Y Q T R V V S N A N<br>K G G E H W S Y G L R P G |
| 32 (Inv-GG-HB$_s$T$_h$-GG-LHRH) | T A K S K K F P S Y T A T Y Q F G G F<br>F L L T R I L T G G E H W S Y G L R P<br>G I P Q S L D |
| 33 (Inv-GG-MV$_{Fl}$T$_h$-GG-LHRH) | T A K S K K F P S Y T A T Y Q F G G L<br>S E I K G V I V H R L E G V G G E H W<br>S Y G L R P G |
| 34 (Inv-GG-PT$_2$T$_h$-GG-LHRH) | T A K S K K F P S Y T A T Y Q F G G G<br>A Y A R C P N G T R A L T V E L R G N<br>A E L G G E H W S Y G L R P G |
| 35 (Inv-GG-TT$_1$T$_h$-GG-LHRH) | T A K S K K F P S Y T A T Y Q F G G K<br>K Q Y I K A N S K F I G I T E L G G E<br>H W S Y G L R P G |
| 36 (Inv-GG-TT$_4$T$_h$-GG-LHRH) | T A K S K K F P S Y T A T Y Q F G G K<br>K W V R D I I D D F T N E S S Q K T G<br>G E H W S Y G L R P G |
| 37 (Inv-GG-TT$_5$-GG-LHRH) | T A K S K K F P S Y T A T Y Q F G G K<br>K D V S T I V P Y I G P A L N I V G G<br>E H W S Y G L R P G |
| 38 (SSAL1-GG-LHRH)[b] | D L S E L K G L L L H K L E G L G G-<br>E I   D I R   I I I   R I D   I<br>  V   V   V V V   V   V<br>  F   F   F F F   F   F<br><br>E H W S Y G L R P G |
| 39 (SSAL2-GG-LHRH)[b] | K K K L F L L T K L L T L P Q S L D-<br>R R R I K I I   R I I   I   L   I R<br>  V R V V   V V   V   V   V<br>  F F F F   F F   F   F   F<br><br>G G E H W S Y G L R P G |

| Peptide SEQ ID NO: | Sequence |
|---|---|
| 40 (Inv-GG-SSAL3-GG-LHRH)[b] | T A K S K K F P S Y T A T Y Q F G G |

**40 (Inv-GG-SSAL3-GG-LHRH)[b]**

```
T A K S K K F P S Y T A T Y Q F G G

D L S E L K G L L L H K L E G L-
E I   D I R   I I I   R I D   I
    V       V       V V V       V       V
    F       F       F F F       F       F

G G E H W S Y G L R P G
```

**41 (Inv-GG-SSAL4-GG-LHRH)[b]**

```
T A K S K K F P S Y T A T Y Q F G G

K K K L F L L T K L L T L P Q S L D-
R R R I K I I   R I I   I   L   I R
      V   V V       V V   V   I   V
      F   F F       F F   F   F   F
                                    V

G G E H W S Y G L R P G
```

[a] Sequences are given in the standard one-letter amino acid codes.
[b] For simplicity, the amino acids present at each position of the library are indicated below the main chain. Invariant amino acids are designated a molar value of one, and Variant amino acids are added during synthesis at an equimolar ratio depending on the number of variants at that position, i.e., if a position has 2 amino acids, then each is added in 0.5 ratio relative to the invariant amount, for 3 amino acids the ratio is 0.33, for 4 amino acids the ratio is 0.25, for 5 amino acids, the ratio is 0.20, etc.

Table 6

| Efficacy of $T_h$: LHRH Synthetic Peptides | | | | | |
|---|---|---|---|---|---|
| Peptide[a] | Seq ID No. | $T_h$ Epitope | $\alpha$-LHRH Ab[b] | Reduced S.T.[c] | Testis Atrophy[d] |
| A | 10 | HBs | 45 | 40 | 35/90 |
| 18 | 18 | HBs | 100 | 85 | 65/95 |
| 19 | 19 | $MV_{F1}\,T_h$ | 85 | 85 | 80/95 |
| K | 16 | $PT_2\,T_h$ | 65 | 50 | 35/90 |
| H | 13 | $TT_1\,T_h$ | 100 | 100 | 95/- |

[a] In each case, animals received equimolar amounts of peptide equivalent to 100 μg of peptide A. Peptide was administered subcutaneously at weeks 0 (in CFA) and at 3 and 6 weeks (in IFA).

[b] Percentage of animals having LHRH-specific antibody titers of 1.0 nmole/ L or greater.

[c] Percentage of animals having serum testosterone levels below 0.5 nmole/ L.

[d] Percentage of animals having mean testis weights less than 10% of adjuvant control groups. The first number is for animals receiving subcutaneous administration of the peptide; the second number is for animals receiving intramuscular administration of the peptide.

Table 6   (continued)

| Efficacy of $T_h$: LHRH Synthetic Peptides | | | | | |
|---|---|---|---|---|---|
| Peptide[a] | Seq ID No. | $T_h$ Epitope | $\alpha$-LHRH Ab[b] | Reduced S.T.[c] | Testis Atrophy[d] |
| I | 14 | $TT_2\ T_h$ | 80 | 60 | 40/- |
| 22 | 22 | $TT_4\ T_h$ | - | - | -/95 |
| 23 | 23 | $TT_5\ T_h$ | - | - | -/95 |
| LHRH | | None | 0 | 0 | 0 |

[a] In each case, animals received equimolar amounts of peptide equivalent to 100 µg of peptide A. Peptide was administered subcutaneously at weeks 0 (in CFA) and at 3 and 6 weeks (in IFA).

[b] Percentage of animals having LHRH-specific antibody titers of 1.0 nmole/ L or greater.

[c] Percentage of animals having serum testosterone levels below 0.5 nmole/ L.

[d] Percentage of animals having mean testis weights less than 10% of adjuvant control groups. The first number is for animals receiving subcutaneous administration of the peptide; the second number is for animals receiving intramuscular administration of the peptide.

TABLE 7

| Efficacy of HBsAg $T_h$: LHRH Delivery in Microparticles | | | |
|---|---|---|---|
| Formulation[a] | $\alpha$-LHRH Ab[b] | Reduced S.T.[c] | Testis Atrophy[d] |
| Detox | 65 | 15 | 15 |
| Squalene+L121 | 85 | 65 | 65 |
| FCA/ IFA | 50 | 0 | 0 |
| IFA/ IFA | 15 | 0 | 0 |
| Alum | 15 | 15 | 15 |
| Liposyn | 0 | 0 | 0 |
| Liposyn + Avridine | 65 | 35 | 35 |
| Liposyn + L121 | 50 | 0 | 0 |
| Liposyn + Saponin | 85 | 65 | 65 |
| Emulsigen | 85 | 50 | 50 |
| Emulsigen+ DDA | 85 | 65 | 65 |
| Emulsigen+ Saponin | 100 | 100 | 100 |
| Saline | 35 | 35 | 35 |
| Free peptide on alum[e] | 20 | 0 | 0 |
| Free peptide in FCA/ IFA[f] | 60 | 40 | 40 |

[a] Sprague-Dawley rats were administered 100 µg of peptide A entrapped in rapid release microparticles at 0, 3 and 6 weeks. All immunizations were given subcutaneously. Results are reported as the percentage of animals giving the indicated responses 14 weeks after the commencement of the study.

[b] LHRH-specific antibody titers of 1.0 nmole/ L or greater.

[c] Serum testosterone levels below 0.5 nmole/ L.

[d] Mean testis weights less than 10% of adjuvant control groups.

[e] Peptide A was administered in its soluble form bound to alum.

[f] Peptide A was administered in its soluble form formulated in Freund's adjuvant.

TABLE 8

| Formulation[1] | α-LHRH Ab[2] | Reduced S.T.[3] | Testis Atrophy[4] |
|---|---|---|---|
| FCA/ IFA | 80 | 80 | 60 |
| IFA/ IFA[h] | 80 | 60 | 40 |
| DETOX[h] | 60 | 20 | 0 |
| MPL[h] | 60 | 40 | 0 |
| MPL+TDE[h] | 40 | 0 | 0 |
| DEAE DEXTRAN[h] | 20 | 0 | 0 |
| LIPOSYN[h] | 0 | 0 | 0 |
| LIPOSYN + AVRIDINE[h] | 80 | 40 | 40 |
| LIPOSYN + L121[h] | 0 | 0 | 0 |
| ISA 51[h] | 60 | 40 | 40 |
| ISA 720[h] | 80 | 60 | 60 |
| EMULSIGEN[a] | 60 | 60 | 40 |
| EMULSIGEN+ DDA[a] | 60 | 40 | 40 |
| EMULSIGEN+ SAPONIN[a] | 0 | 0 | 0 |
| EMULSIGEN+ L121[a] | 100 | 80 | 80 |
| EMULSIGEN+ F127[a] | 60 | 20 | 20 |
| EMULSIGEN+ MDP[a] | 60 | 40 | 40 |
| L121+TWEEN[a] | 60 | 40 | 40 |
| ALUM[h] | 20 | 0 | 0 |

Efficacy of HBs $T_h$: LHRH in Emulsion Formulations

[1] Sprague-Dawley rats were administered 100 µg of peptide I formulated in the above emulsions at 0, 3 & 6 weeks. All immunizations were given subcutaneously. Results are reported as the percentage of animals giving the indicated responses 10 weeks following the commencement of the experiment.

[2] LHRH-specific antibody titers of 1.0 nmole/ L or greater.

[3] Serum testosterone levels below 0.5 nmole/ L.

[4] Mean testis weights less than 10% of adjuvant control groups. a. Adjuvants for use in animals. h. Adjuvants for use in humans.

[a] Adjuvants for use in animals.

[h] Adjuvants for use in humans.

TABLE 9

| Formulation[a] | α-LHRH Ab[b] | Reduced S.T.[c] | Testis Atrophy[d] |
|---|---|---|---|
| FIA | 100 | 100 | 100 |
| DETOX | 100 | 100 | 100 |
| MPL+TDE | 100 | 100 | 65 |
| MPL | 100 | 35 | 15 |
| SQUALENE+ L121 | 100 | 85 | 85 |
| ISA 51 | 100 | 85 | 80 |
| ISA 720 | 100 | 85 | 85 |
| liposyn + AVRIDINE | 100 | 100 | 100 |
| EMULSIGEN | 100 | 85 | 65 |

Efficacy of a Peptide 32-Containing $T_h$: LHRH Immunogen Cocktail

[a] Sprague-Dawley rats were administered 100 µg of a cocktail composed of equimolar amounts of Inv: GG: HBsAg $T_h$: GG: LHRH (peptide 32) + MV F $T_h$1: GG: LHRH + PT $T_h$2: LHRH + TT $T_h$1 :LHRH at 0, 3 & 6 weeks. All immunizations were given intramuscularly. Results are reported as the percentage of animals giving the indicated responses 10 weeks following the commencement of the experiment.

[b] LHRH-specific antibody titers of 1.0 nmole/ L or greater.

[c] Serum testosterone levels below 0.5 nmole/ L.

[d] Mean testis weights less than 10% of adjuvant control groups.

TABLE 9   (continued)

| Efficacy of a Peptide 32-Containing $T_h$: LHRH Immunogen Cocktail | | | |
|---|---|---|---|
| Formulation[a] | $\alpha$-LHRH Ab[b] | Reduced S.T.[c] | Testis Atrophy[d] |
| EMULSIGEN+ DDA | 100 | 100 | 100 |
| EMULSIGEN+ L121 | 100 | 100 | 85 |
| ALUM | 100 | 100 | 100 |
| cocktail w/o pept.32 in IFA[e] | 65 | 35 | 35 |

[a] Sprague-Dawley rats were administered 100 µg of a cocktail composed of equimolar amounts of Inv: GG: HBsAg $T_h$: GG: LHRH (peptide 32) + MV F $T_h$1: GG: LHRH + PT $T_h$2: LHRH + TT $T_h$1 :LHRH at 0, 3 & 6 weeks. All immunizations were given intramuscularly. Results are reported as the percentage of animals giving the indicated responses 10 weeks following the commencement of the experiment.

[b] LHRH-specific antibody titers of 1.0 nmole/ L or greater.

[c] Serum testosterone levels below 0.5 nmole/ L.

[d] Mean testis weights less than 10% of adjuvant control groups.

[e] A cocktail of the same peptides without peptide 32 (at a molar equivalence to the peptide 32-containing cocktail) was formulated in IFA and administered in an identical fashion to the above.

## TABLE 10

### Examples of Universal Synthetic Immunostimulators
### with GG Spacers

| Peptide SEQ ID NO: | Sequence |
|---|---|
| 54 (Inv-GG-HB$_s$T$_h$-GG) | T A K S K K F P S Y T A T Y Q F G G F F L L T R I L T I P Q S L D G G |
| 55 (Inv-GG-MV$_{Fl}$T$_h$-GG) | T A K S K K F P S Y T A T Y Q F G G L S E I K G V I V H R L E G V G G |
| 56 (Inv-GG-PT$_2$T$_h$-GG) | T A K S K K F P S Y T A T Y Q F G G G A Y A R C P N G T R A L T V A E L R G N A E L G G |
| 57 (Inv-GG-TT$_1$T$_h$-GG) | T A K S K K F P S Y T A T Y Q F G G K K Q Y I K A N S K F I G I T E L G G |
| 58 (Inv-GG-TT$_4$T$_h$-GG) | T A K S K K F P S Y T A T Y Q F G G K K W V R D I I D D F T N E S S Q K T G G |
| 59 (Inv-GG-TT$_5$T$_h$-GG) | T A K S K K F P S Y T A T Y Q F G G K K D V S T I V P Y I G P A L N I V G G |
| 60 (GG-HB$_s$T$_h$-GG-Inv) | G G F F L L T R I L T I P Q S L D G G T A K S K K F P S Y T A T Y Q F |
| 61 (GG-MV$_{Fl}$T$_h$-GG-Inv) | G G L S E I K G V I V H R L E G V G G T A K S K K F P S Y T A T Y Q F |
| 62 (GG-PT$_2$T$_h$-GG-Inv) | G G G A Y A R C P N G T R A L T V A E L R G N A E L G G T A K S K K F P S Y T A T Y Q F |
| 63 (GG-TT$_1$T$_h$-GG-Inv) | G G K K Q Y I K A N S K F I G I T E L G G T A K S K K F P S Y T A T Y Q F |
| 64 (GG-TT$_4$T$_h$-GG-Inv) | G G K K W V R D I I D D F T N E S S Q K T G G T A K S K K F P S Y T A T Y Q F |
| 65 (GG-TT$_5$-GG-Inv) | G G K K D V S T I V P Y I G P A L N I V G G T A K S K K F P S Y T A T Y Q F |

**Claims**

1. A peptide that stimulates an immune response to LHRH in a mammal wherein said peptide is represented by the formula

$$(A)_n - (Th)_m - (B)_o - LHRH$$

wherein
A is independently an amino acid, $\alpha$-$NH_2$, an invasin domain or an immunostimulatory analog of the corresponding invasin domain;
B is an amino acid;
each Th is independently SEQ ID NO. 2,3,4,5,6,7,8,9,42,45,46,47,48 or 49.
LHRH is luteinizing hormone releasing hormone or an immunogenic analog thereof;
n is from 1 to 10;
m is from 1 to 4; and
o is from 0 to 10.

2. The peptide of claim 1 wherein said LHRH has an amino acid sequence of SEQ ID No. 1.

**3.** The peptide of claim 1 wherein said peptide has an amino acid sequence of any one of SEQ ID Nos. 10-41.

**4.** The peptide of claim 1 wherein at least one A is an invasin domain.

**5.** The peptide of claim 1 wherein n is 4, and A is $\alpha$-NH$_2$, an invasin domain, glycine and glycine in that order.

**6.** The peptide of claim 1 or claim 5 wherein said invasin domain has an amino acid sequence of SEQ ID No. 53.

**7.** A peptide according to claim 1 comprising an amino acid sequence of SEQ ID Nos. 10, 13, 16, 18, 19, 32 or 38.

**8.** A peptide composition comprising a mixture of two or more peptides of any of claims 1 to 7

**9.** The composition of claim 8 wherein said mixture comprises a combination of peptides having amino acid sequences of SEQ ID Nos. 13, 16, 18 and 19.

**10.** The composition of claim 8 wherein said mixture comprises a combination of peptides having amino acid sequences of SEQ ID Nos. 13, 16, 19 and 32.

**11.** The peptide of claim 1 wherein immunization with said peptide is capable of causing a reduction in serum testosterone to less than 10% of normal values.

**12.** The peptide of claim 1 wherein immunization with said peptide causes atrophy of or prevents growth of the prostate.

**13.** A vaccine composition comprising an immunologically effective amount of a peptide of any one of claims 1 to 7 or a peptide composition according to any of claims 8 to 10 and a pharmaceutically acceptable carrier.

**14.** The vaccine composition of claim 13 wherein said immunologically effective amount of said peptide is 0.5 µg to 1 mg per kilogram body weight per dose.

**15.** Use of a peptide according to any one of claims 1 to 7 or a peptide composition according to any one of claims 8 to 10 for the preparation of a pharmaceutical.

**16.** Use of a peptide according to any one of claims 1 to 7 or a peptide composition according to any one of claims 8 to 10 for the preparation of a vaccine.

**17.** Use of a vaccine composition according to claim 13 or 14 for the preparation of a pharmaceutical for the induction of infertility in a mammal.

**18.** Use of a vaccine composition according to claim 13 or 14 for the preparation of a pharmaceutical for the treatment of androgen-dependent carcinoma to effect repression of or prevent growth of said carcinoma.

**19.** Use of a peptide according to any of claims 1 to 7 or a peptide composition according to any of claims 8 to 10 for the preparation of a medicament for suppressing the activity of LHRH in a mammal.

**20.** Use according to claim 19
wherein said suppression of LHRH activity is for the treatment of prostatic hyperplasia, androgen-dependent carcinoma, prostatic carcinoma, testicular carcinoma, endometriosis, benign uterine tumors, recurrent functional variant cysts (severe) premenstrual syndrome or oestrogen-dependent breast tumors, prevention of oestrogen-dependent breast cancer; or induction of infertility.

**21.** Use according to claim 19 wherein the LHRH activity suppression is for reducing boar taint in pigs, immunocastrating dogs or cats or gelding stallions.

**Patentansprüche**

**1.** Peptid, das die Immunantwort gegenüber LHRH in einem Säugetier stimuliert, wobei das Peptid durch die Formel dargestellt ist:

$$(A)_n - (Th)_m - (B)_o - LHRH$$

worin A unabhängig eine Aminosäure, $\alpha$-NH$_2$, eine Invasindomäne oder ein immunstimulatorisches Analog der entsprechenden Invasindomäne ist;

B ist eine Aminosäure;

jedes Th ist unabhängig SEQ ID Nr. 2, 3, 4, 5, 6, 7, 8, 9, 42, 45, 46, 47, 48 oder 49;

LHRH ist das das lutenisierende Hormon freisetzende Hormon oder ein immunogenes Analog davon;

n ist 1 bis 10;

m ist 1 bis 4; und

o ist 0 bis 10.

2. Peptid gemäss Anspruch 1, wobei das LHRH eine Aminosäuresequenz gemäss SEQ ID Nr. 1 hat.

3. Peptid gemäss Anspruch 1, wobei das Peptid eine Aminosäuresequenz gemäss einer der SEQ ID Nrn. 10 bis 41 hat.

4. Peptid gemäss Anspruch 1, worin mindestens ein A eine Invasindomäne ist.

5. Peptid gemäss Anspruch 1, worin n 4 ist und A ist $\alpha$-NH$_2$, eine Invasindomäne, Glycin und Glycin in dieser Reihenfolge.

6. Peptid gemäss Anspruch 1 oder Anspruch 5, wobei die Invasindomäne eine Aminosäuresequenz gemäss SEQ ID Nr. 53 hat.

7. Peptid gemäss Anspruch 1, entsprechend einer Aminosäuresequenz der SEQ ID Nrn. 10, 13, 16, 18, 19, 32 oder 38.

8. Peptidzusammensetzung, umfassend eine Mischung von zwei oder mehr Peptiden gemäss einem der Ansprüche 1 bis 7.

9. Zusammensetzung gemäss Anspruch 8, wobei die Mischung eine Kombination von Peptiden mit den Aminosäuresequenzen der SEQ ID Nrn. 13, 16, 18 und 19 umfasst.

10. Zusammensetzung gemäss Anspruch 8, wobei die Mischung eine Kombination der Peptide mit den Aminosäuresequenzen SEQ ID Nrn. 13, 16, 19 und 32 umfasst.

11. Peptid gemäss Anspruch 1, wobei die Immunisierung mit diesem Peptid in der Lage ist, eine Reduktion des Serumtestosterons auf weniger als 10 % der normalen Werte hervorzurufen.

12. Peptid gemäss Anspruch 1, wobei die Immunisierung mit diesem Peptid eine Atrophy von oder das Wachstum der Prostata verursacht bzw. verhindert.

13. Vaccinzusammensetzung, umfassend eine immunologisch wirksame Menge eines Peptids gemäss einem der Ansprüche 1 bis 7 oder eine Peptidzusammensetzung gemäss einem der Ansprüche 8 bis 10 und einen pharmazeutisch annehmbaren Träger.

14. Vaccinzusammensetzung gemäss Anspruch 13, wobei die immunologisch wirksame Menge des Peptids 0,5 $\mu$g bis 1 mg pro Kilogramm Körpergewicht pro Dosis ist.

15. Verwendung eines Peptids gemäss einem der Ansprüche 1 bis 7 oder einer Peptidzusammensetzung gemäss einem der Ansprüche 8 bis 10 zur Herstellung eines Pharmazeutikums.

16. Verwendung eines Peptids gemäss einem der Ansprüche 1 bis 7 oder einer Peptidzusammensetzung gemäss einem der Ansprüche 8 bis 10 zur Herstellung eines Vaccins.

17. Verwendung einer Vaccinzusammensetzung gemäss einem der Ansprüche 13 oder 14 zur Herstellung eines Pharmazeutikums zur Induktion der Unfruchtbarkeit in einem Säugetier.

**18.** Verwendung einer Vaccinzusammensetzung gemäss Anspruch 13 oder 14 zur Herstellung eines Pharmazeutikums zur Behandlung eines androgenabhängigen Karzinoms, um eine Repression zu bewirken oder das Wachstum dieses Karzinoms zu verhindern.

**19.** Verwendung eines Peptids gemäss einem der Ansprüche 1 bis 7 oder einer Peptidzusammensetzung gemäss einem der Ansprüche 8 bis 10 zur Herstellung eines Medikaments zum Unterdrücken der Aktivität von LHRH in einem Säugetier.

**20.** Verwendung gemäss Anspruch 19, wobei diese Unterdrückung der LHRH-Aktivität zur Behandlung von Prostatahyperplasie, androgenabhängigem Karzinom, Prostatakarzinom, Hodenkarzinom, Endometriose, benignen Gebärmuttertumoren, wiederauftretenden verschiedenen Zysten bei (schwerem) prämenstrualem Syndrom oder östrogenabhängigen Brusttumoren, Vorbeugung von östrogenabhängigem Brustkrebs oder Induktion von Unfruchtbarkeit ist.

**21.** Verwendung gemäss Anspruch 19 für die LHRH-Aktivitätsunterdrückung zur Reduktion des Geschlechtsbeigeschmacks bei Schweinen, immunkastrierten Hunden oder Katzen oder Wallachen.

**Revendications**

**1.** Peptide qui stimule une réponse immune à la LHRH chez un mammifère, ledit peptide étant représenté par la formule

$$(A)_n\text{-}(Th)_m\text{-}(B)_o\text{-}LHRH$$

dans laquelle
A est indépendamment un acide aminé, $\alpha$-NH$_2$, un domaine invasine ou un analogue immunostimulateur du domaine invasine correspondant;
B est un acide aminé;
chaque Th est indépendamment SEQ ID NO. 2,3,4,5,6,7,8,9,42,45,46,47,48 ou 49.
LHRH est une hormone libératrice de lutéostimuline ou son analogue immunogène;
n est de 1 à 10;
m est de 1 à 4; et
o est de 0 à 10.

**2.** Peptide selon la revendication 1, dans laquelle ladite LHRH possède une séquence d'acides aminés de SEQ ID No. 1.

**3.** Peptide selon la revendication 1, ledit peptide possédant une séquence d'acides aminés de l'un quelconque de SEQ ID Nos. 10-41.

**4.** Peptide selon la revendication 1, dans lequel au moins un A est un domaine invasine.

**5.** Peptide selon la revendication 1, dans lequel n est 4, et A est $\alpha$-NH$_2$, un domaine invasine, glycine et glycine dans cet ordre.

**6.** Peptide selon la revendication 1 ou la revendication 5, dans lequel ledit domaine invasine possède une séquence d'acides aminés de SEQ ID No. 53.

**7.** Peptide selon la revendication 1, comprenant une séquence d'acides aminés de SEQ ID Nos 10,13,16,18,19,32 ou 38.

**8.** Composition peptide comprenant un mélange de deux ou de plus de deux peptides selon l'une quelconque des revendications 1 à 7.

**9.** Composition de la revendication 8, dans laquelle ledit mélange comprend une combinaison de peptides ayant des séquences d'acides aminés de SEQ ID Nos 13,16,18 et 19.

**10.** Composition de la revendication 8, dans laquelle ledit mélange comprend une combinaison de peptides ayant des séquences d'acides aminés de SEQ ID Nos 13,16,19 et 32.

**11.** Peptide de la revendication 1, dans lequel l'immunisation avec ledit peptide est apte à provoquer une réduction de la testostérone dans le sérum jusqu'à moins de 10% des valeurs normales.

**12.** Peptide de la revendication 1, dans laquelle l'immunisation avec ledit peptide provoque l'atrophie ou empêche la croissance de la prostate.

**13.** Composition de vaccin comprenant une quantité immunologiquement efficace d'un peptide selon l'une quelconque des revendications 1 à 7 ou une composition peptide selon l'une quelconque des revendications 8 à 10 et un véhicule pharmaceutiquement acceptable.

**14.** Composition de vaccin de la revendication 13, dans laquelle ladite quantité immunologiquement efficace dudit peptide est de 0,5 µg à 1 mg par kilogramme de poids corporel par dose.

**15.** Utilisation d'un peptide selon l'une quelconque des revendications 1 à 7 ou une composition peptide selon l'une quelconque des revendications 8 à 10 pour la préparation d'un produit pharmaceutique.

**16.** Utilisation d'un peptide selon l'une quelconque des revendications 1 à 7 ou une composition peptide selon l'une quelconque des revendications 8 à 10 pour la préparation d'un vaccin.

**17.** Utilisation d'une composition de vaccin selon la revendication 13 ou 14 pour la préparation d'un produit pharmaceutique pour induire la stérilité chez un mammifère.

**18.** Utilisation d'une composition de vaccin selon la revendication 13 ou 14 pour la préparation d'un produit pharmaceutique pour le traitement de carcinome à dépendance androgène pour réprimer ou empêcher la croissance dudit carcinome.

**19.** Utilisation d'un peptide selon l'une quelconque des revendications 1 à 7 ou une composition peptide selon l'une quelconque des revendications 8 à 10 pour la préparation d'un médicament pour supprimer l'activité de la LHRH chez un mammifère.

**20.** Utilisation selon la revendication 19, dans laquelle ladite suppression de l'activité de la LHRH est destinée au traitement de l'hyperplasie de la prostate, du carcinome à dépendance androgène, du carcinome de la prostate, du carcinome testiculaire, de l'endométriose, des tumeurs utérines bénignes, de kystes variants fonctionnels récurrents, de syndrome prémenstruel (grave) ou de tumeurs du sein à dépendance oestrogène, à la prévention du cancer du sein à dépendance oestrogène; ou destinée à induire la stérilité.

**21.** Utilisation selon la revendication 19, dans laquelle la suppression de l'activité de la LHRH est destinée à réduire la saveur forte due à la circulation de la testostérone dans la viande de porc mâle ("boar taint"), à immunocastrer les chiens ou les chats ou à hongrer les étalons.

## Testes

Fig. 1A

## Epididymis

Fig. 1B

## Prostate + Seminal Vesicles

Fig. 1C

Fig. 1

Fig. 2

Fig. 3

Fig. 4

Fig. 5

Fig. 6

*: significantly different from non immunized controls
**: significantly different from hypophysectomized rats

Fig. 7

*: significantly different from non immunized controls

Fig. 8

Legend:
- □ Control
- ▨ Hypo X
- ▩ Peptide A, 100 $\mu$g
- ▥ Peptide A, 500 $\mu$g
- ■ LHRH, 100 $\mu$g

Y-axis: Prostate and Seminal Vesicles Weight Grams per 100 gm of body weight

EP 0 708 656 B1

Fig. 9

EP 0 708 656 B1

Fig. 10

Legend:
- ● Rat # 812
- ▽ Rat # 813
- ▶ Rat # 814
- □ Rat # 815
- ■ Rat # 816
- △ Rat # 817

Y-axis: Anti-LHRH Titers nmol/L

X-axis: Weeks Post-immunization

Fig. 11

Fig. 12

Fig. 13

Fig. 14

Fig. 15a

Fig. 15b

Fig. 16

EP 0 708 656 B1

Fig. 17

Fig. 18

EP 0 708 656 B1

Fig. 19a

EP 0 708 656 B1

Fig. 19b

EP 0 708 656 B1

Fig. 20

Fig. 21

EP 0 708 656 B1

Fig. 22

Fig. 23

Fig. 24

Fig. 25

Fig. 26

Testis Weight (Grams)

2.0  1.5  1.0  0.5  0.0

1172 1173 1174 1175 1176 1177    Control

Rat numbers

EP 0 708 656 B1

Fig. 27

Weeks of Immunization

Anti-LHRH Titers nmol/L

Rat # 227
Rat # 228
Rat # 229
Rat # 230
Rat # 231

139

Fig. 28

Fig. 29

EP 0 708 656 B1

Fig. 30

EP 0 708 656 B1

Fig. 31

EP 0 708 656 B1

Fig. 32

EP 0 708 656 B1

Fig. 33

Fig. 34

EP 0 708 656 B1

Fig. 35

EP 0 708 656 B1

SEQUENCE LISTING

(1) GENERAL INFORMATION:

(i) APPLICANT: Ladd, Anna
Wang, Chang Yi
Zamb, Timothy

(ii) TITLE OF INVENTION: Immunogenic LHRH peptide constructs and synthetic universal immune stimulators for vaccines

(iii) NUMBER OF SEQUENCES: 114

(iv) CORRESPONDENCE ADDRESS:

(A) ADDRESSEE: SCULLY, SCOTT, MURPHY & PRESSER
(B) STREET: 400 Garden City Plaza
(C) CITY: Garden City
(D) STATE: NY
(E) COUNTRY: USA
(F) ZIP: 11530

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER:
(B) FILING DATE:
(C) CLASSIFICATION:

(viii) ATTORNEY/AGENT INFORMATION:

(A) NAME: GROLZ, EDWARD W.
(B) REGISTRATION NUMBER: 33,705
(C) REFERENCE/DOCKET NUMBER: 9284

(ix) TELECOMMUNICATION INFORMATION:

(A) TELEPHONE: (516) 742-4343
(B) TELEFAX: (516) 742-4366

(2) INFORMATION FOR SEQ ID NO:1:

(i) SEQUENCE CHARACTERISTICS:

(A) LENGTH: 10 amino acids
(B) TYPE: amino acid
(D) TOPOLOGY: linear

(ii) MOLECULE TYPE: peptide

(xi) SEQUENCE DESCRIPTION: SEQ ID NO:1:

```
Glu His Trp Ser Tyr Gly Leu Arg Pro Gly
1               5                   10
```

(2) INFORMATION FOR SEQ ID NO:2:

    (i) SEQUENCE CHARACTERISTICS:

        (A) LENGTH: 15 amino acids
        (B) TYPE: amino acid
        (D) TOPOLOGY: linear

    (ii) MOLECULE TYPE: peptide

    (xi) SEQUENCE DESCRIPTION: SEQ ID NO:2:

```
Phe Phe Leu Leu Thr Arg Ile Leu Thr Ile Pro Gln Ser Leu Asp
 1               5                   10                  15
```

(2) INFORMATION FOR SEQ ID NO:3:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide

    (xi) SEQUENCE DESCRIPTION: SEQ ID NO:3:

```
Lys Lys Leu Arg Arg Leu Leu Tyr Met Ile Tyr Met Ser Gly Leu Ala
 1               5                   10                  15
Val His Val Ser Lys Glu Glu Gln Tyr Tyr Asp Tyr
                 20                  25
```

(2) INFORMATION FOR SEQ ID NO:4:

    (i) SEQUENCE CHARACTERISTICS:

        (A) LENGTH: 17 amino acids
        (B) TYPE: amino acid
        (D) TOPOLOGY: linear

    (ii) MOLECULE TYPE: peptide

    (Xi) SEQUENCE DESCRIPTION: SEQ ID NO:4:

```
Lys Lys Gln Tyr Ile Lys Ala Asn Ser Lys Phe Ile Gly Ile Thr Glu

 1               5                   10                  15
Leu
```

(2) INFORMATION FOR SEQ ID NO:5:

   (i) SEQUENCE CHARACTERISTICS:

      (A) LENGTH: 22 amino acids
      (B) TYPE: amino acid
      (D) TOPOLOGY: linear

   (ii) MOLECULE TYPE: peptide

   (xi) SEQUENCE DESCRIPTION: SEQ ID NO:5:

```
Lys Lys Phe Asn Asn Phe Thr Val Ser Phe Trp Leu Arg Val Pro Lys
 1               5                   10                  15
Val Ser Ala Ser His Leu
               20
```

(2) INFORMATION FOR SEQ ID NO:6:

   (i) SEQUENCE CHARACTERISTICS:

      (A) LENGTH: 15 amino acids
      (B) TYPE: amino acid
      (D) TOPOLOGY: linear

   (ii) MOLECULE TYPE: peptide

   (xi) SEQUENCE DESCRIPTION: SEQ ID NO:6:

```
Tyr Met Ser Gly Leu Ala Val Arg Val His Val Ser Lys Glu Glu
 1               5                   10                  15
```

(2) INFORMATION FOR SEQ ID NO:7:

   (i) SEQUENCE CHARACTERISTICS:

      (A) LENGTH: 27 amino acids
      (B) TYPE: amino acid
      (D) TOPOLOGY: linear

   (ii) MOLECULE TYPE: peptide

   (xi) SEQUENCE DESCRIPTION: SEQ ID NO:7:

```
Tyr Asp Pro Asn Tyr Leu Arg Thr Asp Ser Asp Lys Asp Arg Phe Leu

 1               5                   10                  15

Gln Thr Met Val Lys Leu Phe Asn Arg Ile Lys
               20                  25
```

(2) INFORMATION FOR SEQ ID NO:8:

    (i) SEQUENCE CHARACTERISTICS:

        (A) LENGTH: 24 amino acids
        (B) TYPE: amino acid
        (D) TOPOLOGY: linear

    (ii) MOLECULE TYPE: peptide

    (xi) SEQUENCE DESCRIPTION: SEQ ID NO:8:

```
Gly Ala Tyr Ala Arg Cys Pro Asn Gly Thr Arg Ala Leu Thr Val Ala
 1               5                   10                  15
Glu Leu Arg Gly Asn Ala Glu Leu
            20
```

(2) INFORMATION FOR SEQ ID NO:9:

    (i) SEQUENCE CHARACTERISTICS:

        (A) LENGTH: 15 amino acids
        (B) TYPE: amino acid
        (D) TOPOLOGY: linear

    (ii) MOLECULE TYPE: peptide

    (xi) SEQUENCE DESCRIPTION: SEQ ID NO:9:

```
Ser Glu Ile Lys Gly Val Ile Val His Arg Leu Glu Gly Val Leu
 1               5                   10                  15
```

(2) INFORMATION FOR SEQ ID NO:10:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide

    (xi) SEQUENCE DESCRIPTION: SEQ ID NO:10:

```
Lys Lys Lys Phe Phe Leu Leu Thr Arg Ile Leu Thr Ile Pro Gln Ser
```

EP 0 708 656 B1

```
    1                    5                   10                   15
Leu Asp Glu His Trp Ser Tyr Gly Leu Arg Pro Gly
                    20                   25
```

(2) INFORMATION FOR SEQ ID NO:11:

    (i) SEQUENCE CHARACTERISTICS:

        (A) LENGTH: 38 amino acids
        (B) TYPE: amino acid
        (D) TOPOLOGY: linear

    (ii) MOLECULE TYPE: peptide

    (xi) SEQUENCE DESCRIPTION: SEQ ID NO:11:

```
Lys Lys Leu Arg Arg Leu Leu Tyr Met Ile Tyr Met Ser Gly Leu Ala
 1                    5                   10                   15
Val His Val Ser Lys Glu Glu Gln Tyr Tyr Asp Tyr Glu His Trp Ser
                    20                   25                   30
Tyr Gly Leu Arg Pro Gly
                    35
```

(2) INFORMATION FOR SEQ ID NO:12:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide

    (xi) SEQUENCE DESCRIPTION: SEQ ID NO:12:

```
Tyr Met Ser Gly Leu Ala Val Arg Val His Val Ser Lys Glu Glu Glu
 1                    5                   10                   15
His Trp Ser Tyr Gly Leu Arg Pro Gly
                    20                   25
```

(2) INFORMATION FOR SEQ ID NO:13:

    (i) SEQUENCE CHARACTERISTICS:

        (A) LENGTH: 27 amino acids
        (B) TYPE: amino acid
        (D) TOPOLOGY: linear

    (ii) MOLECULE TYPE: peptide

(xi) SEQUENCE DESCRIPTION: SEQ ID NO:13:

```
Lys Lys Gln Tyr Ile Lys Ala Asn Ser Lys Phe Ile Gly Ile Thr Glu
1               5               10                  15

Leu Glu His Trp Ser Tyr Gly Leu Arg Pro Gly
                20              25
```

(2) INFORMATION FOR SEQ ID NO:14:

    (i) SEQUENCE CHARACTERISTICS:

        (A) LENGTH: 32 amino acids
        (B) TYPE: amino acid
        (D) TOPOLOGY: linear

    (ii) MOLECULE TYPE: peptide

    (xi) SEQUENCE DESCRIPTION: SEQ ID NO:14:

```
Lys Lys Phe Asn Asn Phe Thr Val Ser Phe Trp Leu Arg Val Pro Lys
1               5               10                  15

Val Ser Ala Ser His Leu Glu His Trp Ser Tyr Gly Leu Arg Pro Gly
                20              25                  30
```

(2) INFORMATION FOR SEQ ID NO:15:

    (i) SEQUENCE CHARACTERISTICS:

        (A) LENGTH: 37 amino acids
        (B) TYPE: amino acid
        (D) TOPOLOGY: linear

    (ii) MOLECULE TYPE: peptide

    (xi) SEQUENCE DESCRIPTION: SEQ ID NO:15:

```
Tyr Asp Pro Asn Tyr Leu Arg Thr Asp Ser Asp Lys Asp Arg Phe Leu
1               5               10                  15

Gln Thr Met Val Lys Leu Phe Asn Arg Ile Lys Glu His Trp Ser Tyr
                20              25                  30

Gly Leu Arg Pro Gly
            35
```

(2) INFORMATION FOR SEQ ID NO:16:

    (i) SEQUENCE CHARACTERISTICS:

        (A) LENGTH: 34 amino acids
        (B) TYPE: amino acid

(D) TOPOLOGY: linear

(ii) MOLECULE TYPE: peptide

(xi) SEQUENCE DESCRIPTION: SEQ ID NO:16:

```
Gly Ala Tyr Ala Arg Cys Pro Asn Gly Thr Arg Ala Leu Thr Val Ala
 1               5               10                  15

Glu Leu Arg Gly Asn Ala Glu Leu Glu His Trp Ser Tyr Gly Leu Arg
            20                  25                  30

Pro Gly
```

(2) INFORMATION FOR SEQ ID NO:17:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide

    (xi) SEQUENCE DESCRIPTION: SEQ ID NO:17:

```
Ser Glu Ile Lys Gly Val Ile Val His Arg Leu Glu Gly Val Leu Glu
 1               5               10                  15

His Trp Ser Tyr Gly Leu Arg Pro Gly
            20                  25
```

(2) INFORMATION FOR SEQ ID NO:18:

    (i) SEQUENCE CHARACTERISTICS:

        (A) LENGTH: 30 amino acids
        (B) TYPE: amino acid
        (D) TOPOLOGY: linear

    (ii) MOLECULE TYPE: peptide

    (xi) SEQUENCE DESCRIPTION: SEQ ID NO:18:

```
Lys Lys Lys Phe Phe Leu Leu Thr Arg Ile Leu Thr Ile Pro Gln Ser
 1               5               10                  15

Leu Asp Gly Gly Glu His Trp Ser Tyr Gly Leu Arg Pro Gly
            20                  25                  30
```

(2) INFORMATION FOR SEQ ID NO:19:

(i) SEQUENCE CHARACTERISTICS:

(A) LENGTH: 27 amino acids
(B) TYPE: amino acid
(D) TOPOLOGY: linear

(ii) MOLECULE TYPE: peptide

(xi) SEQUENCE DESCRIPTION: SEQ ID NO:19:

```
Leu Ser Glu Ile Lys Gly Val Ile Val His Arg Leu Glu Gly Val Gly
1               5                   10                  15

Gly Glu His Trp Ser Tyr Gly Leu Arg Pro Gly
            20                  25
```

(2) INFORMATION FOR SEQ ID NO:20:

(i) SEQUENCE CHARACTERISTICS:

(A) LENGTH: 42 amino acids
(B) TYPE: amino acid
(D) TOPOLOGY: linear

(ii) MOLECULE TYPE: peptide

(xi) SEQUENCE DESCRIPTION: SEQ ID NO:20:

```
Leu Ser Glu Ile Lys Gly Val Ile Val His Arg Leu Glu Gly Val Leu
1               5                   10                  15

Ser Glu Ile Lys Gly Val Ile Val His Arg Leu Glu Gly Val Gly Gly
            20                  25                  30

Glu His Trp Ser Tyr Gly Leu Arg Pro Gly
        35                  40
```

(2) INFORMATION FOR SEQ ID NO:21:

(i) SEQUENCE CHARACTERISTICS:

(A) LENGTH: 32 amino acids
(B) TYPE: amino acid
(D) TOPOLOGY: linear

(ii) MOLECULE TYPE: peptide

(xi) SEQUENCE DESCRIPTION: SEQ ID NO:21:

```
Gly Ile Leu Glu Ser Arg Gly Ile Lys Ala Arg Ile Thr His Val Asp
 1               5                  10                  15

Thr Glu Ser Tyr Gly Gly Glu His Trp Ser Tyr Gly Leu Arg Pro Gly
                20              25   .              30
```

(2) INFORMATION FOR SEQ ID NO:22:

    (i) SEQUENCE CHARACTERISTICS:

      (A) LENGTH: 31 amino acids
      (B) TYPE: amino acid
      (D) TOPOLOGY: linear

    (ii) MOLECULE TYPE: peptide

    (xi) SEQUENCE DESCRIPTION: SEQ ID NO:22:

```
Lys Lys Trp Val Arg Asp Ile Ile Asp Asp Phe Thr Asn Glu Ser Ser
 1               5                  10                  15

Gln Lys Thr Gly Gly Glu His Trp Ser Tyr Gly Leu Arg Pro Gly
                20              25                  30
```

(2) INFORMATION FOR SEQ ID NO:23:

    (i) SEQUENCE CHARACTERISTICS:

      (A) LENGTH: 30 amino acids
      (B) TYPE: amino acid
      (D) TOPOLOGY: linear

    (ii) MOLECULE TYPE: peptide

    (xi) SEQUENCE DESCRIPTION: SEQ ID NO:23:

```
Lys Lys Asp Val Ser Thr Ile Val Pro Tyr Ile Gly Pro Ala Leu Asn
 1               5                  10                  15

Ile Val Gly Gly Glu His Trp Ser Tyr Gly Leu Arg Pro Gly
                20              25                  30
```

(2) INFORMATION FOR SEQ ID NO:24:

    (i) SEQUENCE CHARACTERISTICS:

      (A) LENGTH: 37 amino acids
      (B) TYPE: amino acid
      (D) TOPOLOGY: linear

    (ii) MOLECULE TYPE: peptide

    (xi) SEQUENCE DESCRIPTION: SEQ ID NO:24:

```
Ala Leu Asn Ile Trp Asp Arg Phe Asp Val Phe Cys Thr Leu Gly Ala
 1               5               10              15

Thr Thr Gly Tyr Leu Lys Gly Asn Ser Gly Gly Glu His Trp Ser Tyr
            20              25              30

Gly Leu Arg Pro Gly
            35
```

(2) INFORMATION FOR SEQ ID NO:25:

    (i) SEQUENCE CHARACTERISTICS:

        (A) LENGTH: 35 amino acids
        (B) TYPE: amino acid
        (D) TOPOLOGY: linear

    (ii) MOLECULE TYPE: peptide

    (xi) SEQUENCE DESCRIPTION: SEQ ID NO:25:

```
Asp Ser Glu Thr Ala Asp Asn Leu Glu Lys Thr Val Ala Ala Leu Ser
 1               5               10              15

Ile Leu Pro Gly Ile Gly Cys Gly Gly Glu His Trp Ser Tyr Gly Leu
            20              25              30

Arg Pro Gly
        35
```

(2) INFORMATION FOR SEQ ID NO:26:

    (i) SEQUENCE CHARACTERISTICS:

        (A) LENGTH: 51 amino acids
        (B) TYPE: amino acid
        (D) TOPOLOGY: linear

    (ii) MOLECULE TYPE: peptide

    (xi) SEQUENCE DESCRIPTION: SEQ ID NO:26:

```
Glu Glu Ile Val Ala Gln Ser Ile Ala Leu Ser Ser Leu Met Val Ala
 1               5               10              15

Gln Ala Ile Pro Leu Val Gly Glu Leu Val Asp Ile Gly Phe Ala Ala
```

Thr Asn Phe Val Glu Ser Cys Gly Gly Glu His Trp Ser Tyr Gly Leu
20       25       30
      35       40       45

Arg Pro Gly
50

(2) INFORMATION FOR SEQ ID NO:27:

    (i) SEQUENCE CHARACTERISTICS:

        (A) LENGTH: 33 amino acids
        (B) TYPE: amino acid
        (D) TOPOLOGY: linear

    (ii) MOLECULE TYPE: peptide

    (xi) SEQUENCE DESCRIPTION: SEQ ID NO:27:

Asp Ile Glu Lys Lys Ile Ala Lys Met Glu Lys Ala Ser Ser Val Phe
1       5       10       15

Asn Val Val Asn Ser Gly Gly Glu His Trp Ser Tyr Gly Leu Arg Pro
      20       25       30

Gly

(2) INFORMATION FOR SEQ ID NO:28:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide

    (xi) SEQUENCE DESCRIPTION: SEQ ID NO:28:

Lys Trp Phe Lys Thr Asn Ala Pro Asn Gly Val Asp Glu Lys Ile Arg
1       5       10       15

Ile Gly Gly Glu His Trp Ser Tyr Gly Leu Arg Pro Gly
      20       25

(2) INFORMATION FOR SEQ ID NO:29:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide

(xi) SEQUENCE DESCRIPTION: SEQ ID NO:29:

```
Gly Leu Gln Gly Lys Ile Ala Asp Ala Val Lys Ala Lys Gly Gly Gly
 1               5               10              15

Glu His Trp Ser Tyr Gly Leu Arg Pro Gly
                20              25
```

(2) INFORMATION FOR SEQ ID NO:30:

(i) SEQUENCE CHARACTERISTICS:

(A) LENGTH: 31 amino acids
(B) TYPE: amino acid
(D) TOPOLOGY: linear

(ii) MOLECULE TYPE: peptide

(xi) SEQUENCE DESCRIPTION: SEQ ID NO:30:

```
Gly Leu Ala Ala Gly Leu Val Gly Met Ala Ala Asp Ala Met Val Glu
 1               5               10              15

Asp Val Asn Gly Gly Glu His Trp Ser Tyr Gly Leu Arg Pro Gly
                20              25              30
```

(2) INFORMATION FOR SEQ ID NO:31:

(i) SEQUENCE CHARACTERISTICS:

(A) LENGTH: 32 amino acids
(B) TYPE: amino acid
(D) TOPOLOGY: linear

(ii) MOLECULE TYPE: peptide

(xi) SEQUENCE DESCRIPTION: SEQ ID NO:31:

```
Ser Thr Glu Thr Gly Asn Gln His His Tyr Gln Thr Arg Val Val Ser
 1               5               10              15

Asn Ala Asn Lys Gly Gly Glu His Trp Ser Tyr Gly Leu Arg Pro Gly
                20              25              30
```

(2) INFORMATION FOR SEQ ID NO:32:

(i) SEQUENCE CHARACTERISTICS:

(A) LENGTH: 45 amino acids
(B) TYPE: amino acid
(D) TOPOLOGY: linear

(ii) MOLECULE TYPE: peptide

(xi) SEQUENCE DESCRIPTION: SEQ ID NO:32:

```
Thr Ala Lys Ser Lys Lys Phe Pro Ser Tyr Thr Ala Thr Tyr Gln Phe
1               5                   10                  15

Gly Gly Phe Phe Leu Leu Thr Arg Ile Leu Thr Ile Pro Gln Ser Leu
            20                  25                  30

Asp Gly Gly Glu His Trp Ser Tyr Gly Leu Arg Pro Gly
            35                  40                  45
```

(2) INFORMATION FOR SEQ ID NO:33:

    (i) SEQUENCE CHARACTERISTICS:

        (A) LENGTH: 45 amino acids
        (B) TYPE: amino acid
        (D) TOPOLOGY: linear

    (ii) MOLECULE TYPE: peptide

    (xi) SEQUENCE DESCRIPTION: SEQ ID NO:33:

```
Thr Ala Lys Ser Lys Lys Phe Pro Ser Tyr Thr Ala Thr Tyr Gln Phe
1               5                   10                  15

Gly Gly Leu Ser Glu Ile Lys Gly Val Ile Val His Arg Leu Glu Gly
            20                  25                  30

Val Gly Gly Glu His Trp Ser Tyr Gly Leu Arg Pro Gly
            35                  40                  45
```

(2) INFORMATION FOR SEQ ID NO:34:

    (i) SEQUENCE CHARACTERISTICS:

        (A) LENGTH: 54 amino acids
        (B) TYPE: amino acid
        (D) TOPOLOGY: linear

    (ii) MOLECULE TYPE: peptide

    (xi) SEQUENCE DESCRIPTION: SEQ ID NO:34:

```
Thr Ala Lys Ser Lys Lys Phe Pro Ser Tyr Thr Ala Thr Tyr Gln Phe
1               5                   10                  15

Gly Gly Gly Ala Tyr Ala Arg Cys Pro Asn Gly Thr Arg Ala Leu Thr
            20                  25                  30

Val Ala Glu Leu Arg Gly Asn Ala Glu Leu Gly Gly Glu His Trp Ser
            35                  40                  45

Tyr Gly Leu Arg Pro Gly
        50
```

(2) INFORMATION FOR SEQ ID NO:35:

    (i) SEQUENCE CHARACTERISTICS:

        (A) LENGTH: 47 amino acids
        (B) TYPE: amino acid
        (D) TOPOLOGY: linear

    (ii) MOLECULE TYPE: peptide

    (xi) SEQUENCE DESCRIPTION: SEQ ID NO:35:

```
Thr Ala Lys Ser Lys Lys Phe Pro Ser Tyr Thr Ala Thr Tyr Gln Phe
1               5                   10                  15

Gly Gly Lys Lys Gln Tyr Ile Lys Ala Asn Ser Lys Phe Ile Gly Ile
            20                  25                  30

Thr Glu Leu Gly Gly Glu His Trp Ser Tyr Gly Leu Arg Pro Gly
            35                  40                  45
```

(2) INFORMATION FOR SEQ ID NO:36:

    (i) SEQUENCE CHARACTERISTICS:

        (A) LENGTH: 49 amino acids
        (B) TYPE: amino acid
        (D) TOPOLOGY: linear

    (ii) MOLECULE TYPE: peptide

    (xi) SEQUENCE DESCRIPTION: SEQ ID NO:36:

```
Thr Ala Lys Ser Lys Lys Phe Pro Ser Tyr Thr Ala Thr Tyr Gln Phe
1               5                   10                  15

Gly Gly Lys Lys Trp Val Arg Asp Ile Ile Asp Asp Phe Thr Asn Glu
            20                  25                  30

Ser Ser Gln Lys Thr Gly Gly Glu His Trp Ser Tyr Gly Leu Arg Pro
            35                  40                  45

Gly
```

(2) INFORMATION FOR SEQ ID NO:37:

    (i) SEQUENCE CHARACTERISTICS:

        (A) LENGTH: 48 amino acids
        (B) TYPE: amino acid
        (D) TOPOLOGY: linear

(ii) MOLECULE TYPE: peptide

(xi) SEQUENCE DESCRIPTION: SEQ ID NO:37:

```
Thr Ala Lys Ser Lys Lys Phe Pro Ser Tyr Thr Ala Thr Tyr Gln Phe
 1               5                 10                15

Gly Gly Lys Lys Asp Val Ser Thr Ile Val Pro Tyr Ile Gly Pro Ala
            20                25                30

Leu Asn Ile Val Gly Gly Glu His Trp Ser Tyr Gly Leu Arg Pro Gly
        35                40                45
```

(2) INFORMATION FOR SEQ ID NO:38:

(i) SEQUENCE CHARACTERISTICS:

(A) LENGTH: 28 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 1
(D) OTHER INFORMATION: /note= "D0.50;E0.50"

(ix) FEATURE:

(A) NAME/KEY: Modified-site
(B) LOCATION: 2
(D) OTHER INFORMATION: /note="L0.25;I0.25;V0.25;F0.25"

(ix) FEATURE:

(A) NAME/KEY: Modified-site
(B) LOCATION: 4
(D) OTHER INFORMATION: /note= "E0.50;D0.50"

(ix) FEATURE:

(A) NAME/KEY: Modified-site
(B) LOCATION: 5
(D) OTHER INFORMATION: /note="L0.25;I0.25;V0.25;F0.25"

(ix) FEATURE:

(A) NAME/KEY: Modified-site
(B) LOCATION: 6
(D) OTHER INFORMATION: /note= "K0.50;R0.50"

(ix) FEATURE:

(A) NAME/KEY: Modified-site
(B) LOCATION: 8

(D) OTHER INFORMATION: /note="L0.25;I0.25;V0.25;F0.25"

(ix) FEATURE:

(A) NAME/KEY: Modified-site
(B) LOCATION: 9
(D) OTHER INFORMATION: /note="L0.25;I0.25;V0.25;F0.25"

(ix) FEATURE:

(A) NAME/KEY: Modified-site
(B) LOCATION: 10
(D) OTHER INFORMATION: /note="L0.25;I0.25;V0.25;F0.25"

(ix) FEATURE:

(A) NAME/KEY: Modified-site
(B) LOCATION: 12
(D) OTHER INFORMATION: /note= "K0.50;R0.50"

(ix) FEATURE:

(A) NAME/KEY: Modified-site
(B) LOCATION: 13
(D) OTHER INFORMATION: /note="L0.25;I0.25;V0.25;F0.25"

(ix) FEATURE:

(A) NAME/KEY: Modified-site
(B) LOCATION: 14
(D) OTHER INFORMATION: /note= "E0.50;D0.50"

(ix) FEATURE:

(A) NAME/KEY: Modified-site
(B) LOCATION: 16
(D) OTHER INFORMATION: /note="L0.25;I0.25;V0.25;F0.25"

(xi) SEQUENCE DESCRIPTION: SEQ ID NO:38:


```
Asp Leu Ser Glu Leu Lys Gly Leu Leu Leu His Lys Leu Glu Gly Leu
1               5                   10                  15

Gly Gly Glu His Trp Ser Tyr Gly Leu Arg Pro Gly
            20                  25
```


(2) INFORMATION FOR SEQ ID NO:39:

(i) SEQUENCE CHARACTERISTICS:

(A) LENGTH: 30 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 1
(D) OTHER INFORMATION: /note= "K0.50;K0.50"

(ix) FEATURE:

(A) NAME/KEY: Modified-site
(B) LOCATION: 2
(D) OTHER INFORMATION: /note= "K0.50;R0.50"

(ix) FEATURE:

(A) NAME/KEY: Modified-site
(B) LOCATION: 3
(D) OTHER INFORMATION: /note= "K0.50;R0.50"

(ix) FEATURE:

(A) NAME/KEY: Modified-site
(B) LOCATION: 4
(D) OTHER INFORMATION: /note="L0.25;I0.25;V0.25;F0.25"

(ix) FEATURE:

(A) NAME/KEY: Modified-site
(B) LOCATION: 5
(D) OTHER INFORMATION: /note= "F0.34;K0.33;R0.33"

(ix) FEATURE:

(A) NAME/KEY: Modified-site
(B) LOCATION: 6
(D) OTHER INFORMATION: /note="L0.25;I0.25;V0.25;F0.25"

(ix) FEATURE:

(A) NAME/KEY: Modified-site
(B) LOCATION: 7
(D) OTHER INFORMATION: /note="L0.25;I0.25;V0.25;F0.25"

(ix) FEATURE:

(A) NAME/KEY: Modified-site
(B) LOCATION: 9
(D) OTHER INFORMATION: /note= "K0.50;R0.50"

(ix) FEATURE:

(A) NAME/KEY: Modified-site
(B) LOCATION: 10
(D) OTHER INFORMATION: /note="L0.25;I0.25;V0.25;F0.25"

(ix) FEATURE:

(A) NAME/KEY: Modified-site
(B) LOCATION: 11
(D) OTHER INFORMATION: /note="L0.25;I0.25;V0.25;F0.25"

(ix) FEATURE:

    (A) NAME/KEY: Modified-site
    (B) LOCATION: 13
    (D) OTHER INFORMATION: /note="L0.25;I0.25;V0.25;F0.25"

(ix) FEATURE:

    (A) NAME/KEY: Modified-site
    (B) LOCATION: 15
    (D) OTHER INFORMATION: /note= "Q0.20;L0.20;I0.20;F0.20V0.20"

(ix) FEATURE:

    (A) NAME/KEY: Modified-site
    (B) LOCATION: 17
    (D) OTHER INFORMATION: /note="L0.25;I0.25;V0.25;F0.25"

(ix) FEATURE:

    (A) NAME/KEY: Modified-site
    (B) LOCATION: 18
    (D) OTHER INFORMATION: /note="D0.50;R0.50"

(xi) SEQUENCE DESCRIPTION: SEQ ID NO:39:

```
Lys Lys Lys Leu Phe Leu Leu Thr Lys Leu Leu Thr Leu Pro Gln Ser
 1               5               10                      15

Leu Asp Gly Gly Glu His Trp Ser Tyr Gly Leu Arg Pro Gly
                20              25              30
```

(2) INFORMATION FOR SEQ ID NO:40:

(i) SEQUENCE CHARACTERISTICS:

    (A) LENGTH: 46 amino acids
    (B) TYPE: amino acid
    (D) TOPOLOGY: linear

(ii) MOLECULE TYPE: peptide

(ix) FEATURE:

    (A) NAME/KEY: Modified-site
    (B) LOCATION: 19
    (D) OTHER INFORMATION: /note="D0.50;E0.50"

(ix) FEATURE:

    (A) NAME/KEY: Modified-site
    (B) LOCATION: 20
    (D) OTHER INFORMATION: /note="L0.25;I0.25;V0.25;F0.25"

(ix) FEATURE:

    (A) NAME/KEY: Modified-site

(B) LOCATION: 22
(D) OTHER INFORMATION: /note="E0.50;D0.50"

(ix) FEATURE:

(A) NAME/KEY: Modified-site
(B) LOCATION: 23
(D) OTHER INFORMATION: /note="L0.25;I0.25;V0.25;F0.25"

(ix) FEATURE:

(A) NAME/KEY: Modified-site
(B) LOCATION: 24
(D) OTHER INFORMATION: /note="K0.50;R0.50"

(ix) FEATURE:

(A) NAME/KEY: Modified-site
(B) LOCATION: 26
(D) OTHER INFORMATION: /note="L0.25;I0.25;V0.25;F0.25"

(ix) FEATURE:

(A) NAME/KEY: Modified-site
(B) LOCATION: 27
(D) OTHER INFORMATION: /note="L0.25;I0.25;V0.25;F0.25"

(ix) FEATURE:

(A) NAME/KEY: Modified-site
(B) LOCATION: 28
(D) OTHER INFORMATION: /note="L0.25;I0.25;V0.25;F0.25"

(ix) FEATURE:

(A) NAME/KEY: Modified-site
(B) LOCATION: 30
(D) OTHER INFORMATION: /note="K0.50;R0.50"

(ix) FEATURE:

(A) NAME/KEY: Modified-site
(B) LOCATION: 31
(D) OTHER INFORMATION: /note="L0.25;I0.25;V0.25;F0.25"

(ix) FEATURE:

(A) NAME/KEY: Modified-site
(B) LOCATION: 32
(D) OTHER INFORMATION: /note="E0.50;D0.50"

(ix) FEATURE:

(A) NAME/KEY: Modified-site
(B) LOCATION: 34
(D) OTHER INFORMATION: /note="L0.25;I0.25;V0.25;F0.25"

(xi) SEQUENCE DESCRIPTION: SEQ ID NO:40:

```
    Thr Ala Lys Ser Lys Lys Phe Pro Ser Tyr Thr Ala Thr Tyr Gln Phe


    1                   5                   10                  15
    Gly Gly Asp Leu Ser Glu Leu Lys Gly Leu Leu Leu His Lys Leu Glu
                  20                  25                  30
    Gly Leu Gly Gly Glu His Trp Ser Tyr Gly Leu Arg Pro Gly
              35                  40                  45
```

(2) INFORMATION FOR SEQ ID NO:41:

(i) SEQUENCE CHARACTERISTICS:

(A) LENGTH: 48 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 19
(D) OTHER INFORMATION: /note="K0.50;R0.50"

(ix) FEATURE:

(A) NAME/KEY: Modified-site
(B) LOCATION: 20
(D) OTHER INFORMATION: /note="K0.50;R0.50"

(ix) FEATURE:

(A) NAME/KEY: Modified-site
(B) LOCATION: 21
(D) OTHER INFORMATION: /note="K0.50;R0.50"

(ix) FEATURE:

(A) NAME/KEY: Modified-site
(B) LOCATION: 22
(D) OTHER INFORMATION: /note="L0.25;I0.25;V0.25;F0.25"

(ix) FEATURE:

(A) NAME/KEY: Modified-site
(B) LOCATION: 23
(D) OTHER INFORMATION: /note="F0.34;K0.33;R0.33"

(ix) FEATURE:

(A) NAME/KEY: Modified-site
(B) LOCATION: 24

(D) OTHER INFORMATION: /note="L0.25;I0.25;V0.25;F0.25"

(ix) FEATURE:

    (A) NAME/KEY: Modified-site
    (B) LOCATION: 25
    (D) OTHER INFORMATION: /note="L0.25;I0.25;V0.25;F0.25"

(ix) FEATURE:

    (A) NAME/KEY: Modified-site
    (B) LOCATION: 27
    (D) OTHER INFORMATION: /note="K0.50;R0.50"

(ix) FEATURE:

    (A) NAME/KEY: Modified-site
    (B) LOCATION: 28
    (D) OTHER INFORMATION: /note="L0.25;I0.25;V0.25;F0.25"

(ix) FEATURE:

    (A) NAME/KEY: Modified-site
    (B) LOCATION: 29
    (D) OTHER INFORMATION: /note="L0.25;I0.25;V0.25;F0.25"

(ix) FEATURE:

    (A) NAME/KEY: Modified-site
    (B) LOCATION: 31
    (D) OTHER INFORMATION: /note="L0.25;I0.25;V0.25;F0.25"

(ix) FEATURE:

    (A) NAME/KEY: Modified-site
    (B) LOCATION: 33
    (D) OTHER INFORMATION: /note= "Q0.20;L0.20;I0.20;F0.20;V0.20"

(ix) FEATURE:

    (A) NAME/KEY: Modified-site
    (B) LOCATION: 35
    (D) OTHER INFORMATION: /note="L0.25;I0.25;V0.25;F0.25"

(ix) FEATURE:

    (A) NAME/KEY: Modified-site
    (B) LOCATION: 36
    (D) OTHER INFORMATION: /note="D0.50;R0.50"

(xi) SEQUENCE DESCRIPTION: SEQ ID NO:41:

```
Thr Ala Lys Ser Lys Lys Phe Pro Ser Tyr Thr Ala Thr Tyr Gln Phe
 1               5                   10                  15

Gly Gly Lys Lys Lys Leu Phe Leu Leu Thr Lys Leu Leu Thr Leu Pro
             20                  25                  30

Gln Ser Leu Asp Gly Gly Glu His Trp Ser Tyr Gly Leu Arg Pro Gly
         35                  40                  45
```

(2) INFORMATION FOR SEQ ID NO:42:

   (i) SEQUENCE CHARACTERISTICS:

      (A) LENGTH: 20 amino acids
      (B) TYPE: amino acid
      (D) TOPOLOGY: linear

   (ii) MOLECULE TYPE: peptide

   (xi) SEQUENCE DESCRIPTION: SEQ ID NO:42:

```
Gly Ile Leu Glu Ser Arg Gly Ile Lys Ala Arg Ile Thr His Val Asp
 1               5                   10                  15

Thr Glu Ser Tyr
             20
```

(2) INFORMATION FOR SEQ ID NO:43:

   (i) SEQUENCE CHARACTERISTICS:

      (A) LENGTH: 17 amino acids
      (B) TYPE: amino acid
      (D) TOPOLOGY: linear

   (ii) MOLECULE TYPE: peptide

   (xi) SEQUENCE DESCRIPTION: SEQ ID NO:43:

```
Trp Val Arg Asp Ile Ile Asp Asp Phe Thr Asn Glu Ser Ser Gln Lys
 1               5                   10                  15

Thr
```

(2) INFORMATION FOR SEQ ID NO:44:

   (i) SEQUENCE CHARACTERISTICS:

      (A) LENGTH: 16 amino acids
      (B) TYPE: amino acid
      (D) TOPOLOGY: linear

   (ii) MOLECULE TYPE: peptide

(xi) SEQUENCE DESCRIPTION: SEQ ID NO:44:

```
Asp Val Ser Thr Ile Val Pro Tyr Ile Gly Pro Ala Leu Asn Ile Val
  1               5                   10                  15
```

(2) INFORMATION FOR SEQ ID NO:45:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide

    (xi) SEQUENCE DESCRIPTION: SEQ ID NO:45:

```
Ala Leu Asn Ile Trp Asp Arg Phe Asp Val Phe Cys Thr Leu Gly Ala
  1               5                   10                  15
Thr Thr Gly Tyr Leu Lys Gly Asn Ser
              20              25
```

(2) INFORMATION FOR SEQ ID NO:46:

    (i) SEQUENCE CHARACTERISTICS:

        (A) LENGTH: 23 amino acids
        (B) TYPE: amino acid
        (D) TOPOLOGY: linear

    (ii) MOLECULE TYPE: peptide

    (xi) SEQUENCE DESCRIPTION: SEQ ID NO:46:

```
Asp Ser Glu Thr Ala Asp Asn Leu Glu Lys Thr Val Ala Ala Leu Ser
  1               5                   10                  15
Ile Leu Pro Gly Ile Gly Cys
              20
```

(2) INFORMATION FOR SEQ ID NO:47:

    (i) SEQUENCE CHARACTERISTICS:

        (A) LENGTH: 39 amino acids
        (B) TYPE: amino acid
        (D) TOPOLOGY: linear

    (ii) MOLECULE TYPE: peptide

    (xi) SEQUENCE DESCRIPTION: SEQ ID NO:47:

```
Glu Glu Ile Val Ala Gln Ser Ile Ala Leu Ser Ser Leu Met Val Ala
 1               5                  10                  15

Gln Ala Ile Pro Leu Val Gly Glu Leu Val Asp Ile Gly Phe Ala Ala
                20                  25                  30

Thr Asn Phe Val Glu Ser Cys
                35
```

(2) INFORMATION FOR SEQ ID NO:48:

    (i) SEQUENCE CHARACTERISTICS:

        (A) LENGTH: 21 amino acids
        (B) TYPE: amino acid
        (D) TOPOLOGY: linear

    (ii) MOLECULE TYPE: peptide

    (xi) SEQUENCE DESCRIPTION: SEQ ID NO:48:

```
Asp Ile Glu Lys Lys Ile Ala Lys Met Glu Lys Ala Ser Ser Val Phe
 1               5                  10                  15

Asn Val Val Asn Ser
                20
```

(2) INFORMATION FOR SEQ ID NO:49:

    (i) SEQUENCE CHARACTERISTICS:

        (A) LENGTH: 17 amino acids
        (B) TYPE: amino acid
        (D) TOPOLOGY: linear

    (ii) MOLECULE TYPE: peptide

    (xi) SEQUENCE DESCRIPTION: SEQ ID NO:49:

```
Lys Trp Phe Lys Thr Asn Ala Pro Asn Gly Val Asp Glu Lys Ile Arg
 1               5                  10                  15

Ile
```

(2) INFORMATION FOR SEQ ID NO:50:

    (i) SEQUENCE CHARACTERISTICS:

        (A) LENGTH: 14 amino acids
        (B) TYPE: amino acid
        (D) TOPOLOGY: linear

    (ii) MOLECULE TYPE: peptide

    (xi) SEQUENCE DESCRIPTION: SEQ ID NO:50:

```
Gly Leu Gln Gly Lys Ile Ala Asp Ala Val Lys Ala Lys Gly
 1               5                   10
```

(2) INFORMATION FOR SEQ ID NO:51:

    (i) SEQUENCE CHARACTERISTICS:

        (A) LENGTH: 19 amino acids
        (B) TYPE: amino acid
        (D) TOPOLOGY: linear

    (ii) MOLECULE TYPE: peptide

    (xi) SEQUENCE DESCRIPTION: SEQ ID NO:51:

```
Gly Leu Ala Ala Gly Leu Val Gly Met Ala Ala Asp Ala Met Val Glu
 1               5                   10                  15

Asp Val Asn
```

(2) INFORMATION FOR SEQ ID NO:52:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide

    (xi) SEQUENCE DESCRIPTION: SEQ ID NO:52:

```
Ser Thr Glu Thr Gly Asn Gln His His Tyr Gln Thr Arg Val Val Ser
 1               5                   10                  15

Asn Ala Asn Lys
                20
```

(2) INFORMATION FOR SEQ ID NO:53:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide

    (xi) SEQUENCE DESCRIPTION: SEQ ID NO:53:

```
Thr Ala Lys Ser Lys Lys Phe Pro Ser Tyr Thr Ala Thr Tyr Gln Phe
 1               5                   10                  15
```

(2) INFORMATION FOR SEQ ID NO:54:

(i) SEQUENCE CHARACTERISTICS:

(A) LENGTH: 35 amino acids
(B) TYPE: amino acid
(D) TOPOLOGY: linear

(ii) MOLECULE TYPE: peptide

(xi) SEQUENCE DESCRIPTION: SEQ ID NO:54:

```
Thr Ala Lys Ser Lys Lys Phe Pro Ser Tyr Thr Ala Thr Tyr Gln Phe
 1               5                  10                  15

Gly Gly Phe Phe Leu Leu Thr Arg Ile Leu Thr Ile Pro Gln Ser Leu
            20                  25                  30

Asp Gly Gly
        35
```

(2) INFORMATION FOR SEQ ID NO:55:

(i) SEQUENCE CHARACTERISTICS:

(A) LENGTH: 35 amino acids
(B) TYPE: amino acid
(D) TOPOLOGY: linear

(ii) MOLECULE TYPE: peptide

(xi) SEQUENCE DESCRIPTION: SEQ ID NO:55:

```
Thr Ala Lys Ser Lys Lys Phe Pro Ser Tyr Thr Ala Thr Tyr Gln Phe
 1               5                  10                  15

Gly Gly Leu Ser Glu Ile Lys Gly Val Ile Val His Arg Leu Glu Gly
            20                  25                  30

Val Gly Gly
        35
```

(2) INFORMATION FOR SEQ ID NO:56:

(i) SEQUENCE CHARACTERISTICS:

(A) LENGTH: 43 amino acids
(B) TYPE: amino acid
(D) TOPOLOGY: linear

(ii) MOLECULE TYPE: peptide

(xi) SEQUENCE DESCRIPTION: SEQ ID NO:56:

```
Thr Ala Lys Ser Lys Lys Phe Pro Ser Tyr Thr Ala Thr Tyr Gln Phe
1                   5                   10                  15
Gly Gly Gly Ala Tyr Ala Arg Cys Pro Asn Gly Thr Arg Ala Leu Thr
                20                  25                  30
Val Ala Glu Leu Arg Gly Asn Ala Glu Leu Gly Gly
            35                  40
```

(2) INFORMATION FOR SEQ ID NO:57:

    (i) SEQUENCE CHARACTERISTICS:

        (A) LENGTH: 37 amino acids
        (B) TYPE: amino acid
        (D) TOPOLOGY: linear

    (ii) MOLECULE TYPE: peptide

    (xi) SEQUENCE DESCRIPTION: SEQ ID NO:57:

```
Thr Ala Lys Ser Lys Lys Phe Pro Ser Tyr Thr Ala Thr Tyr Gln Phe
1                   5                   10                  15
Gly Gly Lys Lys Gln Tyr Ile Lys Ala Asn Ser Lys Phe Ile Gly Ile
                20                  25                  30
Thr Glu Leu Gly Gly
            35
```

    (2) INFORMATION FOR SEQ ID NO:58:

        (i) SEQUENCE CHARACTERISTICS:

        (A) LENGTH: 39 amino acids
        (B) TYPE: amino acid
        (D) TOPOLOGY: linear

    (ii) MOLECULE TYPE: peptide

    (xi) SEQUENCE DESCRIPTION: SEQ ID NO:58:

```
Thr Ala Lys Ser Lys Lys Phe Pro Ser Tyr Thr Ala Thr Tyr Gln Phe
1                   5                   10                  15
Gly Gly Lys Lys Trp Val Arg Asp Ile Ile Asp Asp Phe Thr Asn Glu
                20                  25                  30
Ser Ser Gln Lys Thr Gly Gly
            35
```

    (2) INFORMATION FOR SEQ ID NO:59:

        (i) SEQUENCE CHARACTERISTICS:

(A) LENGTH: 38 amino acids
(B) TYPE: amino acid
(D) TOPOLOGY: linear

(ii) MOLECULE TYPE: peptide

(xi) SEQUENCE DESCRIPTION: SEQ ID NO:59:

```
Thr Ala Lys Ser Lys Lys Phe Pro Ser Tyr Thr Ala Thr Tyr Gln Phe
 1               5               10                      15

Gly Gly Lys Lys Asp Val Ser Thr Ile Val Pro Tyr Ile Gly Pro Ala
            20               25                      30

Leu Asn Ile Val Gly Gly
            35
```

(2) INFORMATION FOR SEQ ID NO:60:

(i) SEQUENCE CHARACTERISTICS:

(A) LENGTH: 35 amino acids
(B) TYPE: amino acid
(D) TOPOLOGY: linear

(ii) MOLECULE TYPE: peptide

(xi) SEQUENCE DESCRIPTION: SEQ ID NO:60:

```
Gly Gly Phe Phe Leu Leu Thr Arg Ile Leu Thr Ile Pro Gln Ser Leu
 1               5               10                      15

Asp Gly Gly Thr Ala Lys Ser Lys Lys Phe Pro Ser Tyr Thr Ala Thr
            20               25                      30

Tyr Gln Phe
        35
```

(2) INFORMATION FOR SEQ ID NO:61:

(i) SEQUENCE CHARACTERISTICS:

(A) LENGTH: 35 amino acids
(B) TYPE: amino acid
(D) TOPOLOGY: linear

(ii) MOLECULE TYPE: peptide

(xi) SEQUENCE DESCRIPTION: SEQ ID NO:61:

```
Gly Gly Leu Ser Glu Ile Lys Gly Val Ile Val His Arg Leu Glu Gly
  1               5                   10                  15

Val Gly Gly Thr Ala Lys Ser Lys Lys Phe Pro Ser Tyr Thr Ala Thr
               20                  25                  30

Tyr Gln Phe
           35
```

(2) INFORMATION FOR SEQ ID NO:62:

    (i) SEQUENCE CHARACTERISTICS:

        (A) LENGTH: 43 amino acids
        (B) TYPE: amino acid
        (D) TOPOLOGY: linear

    (ii) MOLECULE TYPE: peptide

    (xi) SEQUENCE DESCRIPTION: SEQ ID NO:62:

```
Gly Gly Gly Ala Tyr Ala Arg Cys Pro Asn Gly Thr Arg Ala Leu Thr
  1               5                   10                  15

Val Ala Glu Leu Arg Gly Asn Ala Glu Leu Gly Gly Thr Ala Lys Ser Lys
               20                  25                  30

Lys Phe Pro Ser Tyr Thr Ala Thr Tyr Gln Phe
           35                  40
```

(2) INFORMATION FOR SEQ ID NO:63:

    (i) SEQUENCE CHARACTERISTICS:

        (A) LENGTH: 37 amino acids
        (B) TYPE: amino acid
        (D) TOPOLOGY: linear

    (ii) MOLECULE TYPE: peptide

    (xi) SEQUENCE DESCRIPTION: SEQ ID NO:63:

```
Gly Gly Lys Lys Gln Tyr Ile Lys Ala Asn Ser Lys Phe Ile Gly Ile
  1               5                   10                  15

Thr Glu Leu Gly Gly Thr Ala Lys Ser Lys Lys Phe Pro Ser Tyr Thr
               20                  25                  30

Ala Thr Tyr Gln Phe
           35
```

(2) INFORMATION FOR SEQ ID NO:64:

    (i) SEQUENCE CHARACTERISTICS:

(A) LENGTH: 39 amino acids
(B) TYPE: amino acid
(D) TOPOLOGY: linear

(ii) MOLECULE TYPE: peptide

(xi) SEQUENCE DESCRIPTION: SEQ ID NO:64:

```
Gly Gly Lys Lys Trp Val Arg Asp Ile Ile Asp Asp Phe Thr Asn Glu
 1               5                   10                  15

Ser Ser Gln Lys Thr Gly Gly Thr Ala Lys Ser Lys Lys Phe Pro Ser
                20                  25                  30

Tyr Thr Ala Thr Tyr Gln Phe
            35
```

(2) INFORMATION FOR SEQ ID NO:65:

(i) SEQUENCE CHARACTERISTICS:

(A) LENGTH: 38 amino acids
(B) TYPE: amino acid
(D) TOPOLOGY: linear

(ii) MOLECULE TYPE: peptide

(xi) SEQUENCE DESCRIPTION: SEQ ID NO:65:

```
Gly Gly Lys Lys Asp Val Ser Thr Ile Val Pro Tyr Ile Gly Pro Ala
 1               5                   10                  15

Leu Asn Ile Val Gly Gly Thr Ala Lys Ser Lys Lys Phe Pro Ser Tyr
                20                  25                  30

Thr Ala Thr Tyr Gln Phe
            35
```

(2) INFORMATION FOR SEQ ID NO:66:

(i) SEQUENCE CHARACTERISTICS:

(A) LENGTH: 37 amino acids
(B) TYPE: amino acid
(D) TOPOLOGY: linear

(ii) MOLECULE TYPE: peptide

(xi) SEQUENCE DESCRIPTION: SEQ ID NO:66:

**68**

```
Lys Cys Asn Thr Ala Thr Cys Ala Thr Gln Arg Leu Ala Asn Phe Leu
1               5                   10                  15
Val His Ser Ser Asn Asn Phe Gly Ala Ile Leu Ser Ser Thr Asn Val
                20                  25                  30
Gly Ser Asn Thr Tyr
            35
```

(2) INFORMATION FOR SEQ ID NO:67:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide

    (xi) SEQUENCE DESCRIPTION: SEQ ID NO:67:

```
Lys Cys Asn Thr Ala Thr Cys Ala Thr Gln Arg Leu Ala Asn Phe Leu
1               5                   10                  15
Val His Ser Ser
            20
```

(2) INFORMATION FOR SEQ ID NO:68:

    (i) SEQUENCE CHARACTERISTICS:

        (A) LENGTH: 19 amino acids
        (B) TYPE: amino acid
        (D) TOPOLOGY: linear

    (ii) MOLECULE TYPE: peptide

    (xi) SEQUENCE DESCRIPTION: SEQ ID NO:68:

```
Ser Ser Asn Asn Phe Gly Ala Ile Leu Ser Ser Thr Asn Val Gly Ser
1               5                   10                  15
Asn Thr Tyr
```

(2) INFORMATION FOR SEQ ID NO:69:

    (i) SEQUENCE CHARACTERISTICS:

        (A) LENGTH: 34 amino acids
        (B) TYPE: amino acid
        (D) TOPOLOGY: linear

    (ii) MOLECULE TYPE: peptide

(xi) SEQUENCE DESCRIPTION: SEQ ID NO:69:

Gln Leu Gly Pro Gln Gly Pro Pro His Leu Val Ala Asp Pro Ser Lys
1               5               10                  15

Lys Gln Gly Pro Trp Leu Glu Glu Glu Glu Glu Ala Tyr Gly Trp Met
        20              25              30

Asp Phe


(2) INFORMATION FOR SEQ ID NO:70:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide

    (xi) SEQUENCE DESCRIPTION: SEQ ID NO:70:


Gln Leu Gly Pro Gln Gly Pro Pro His Leu Val Ala Asp Pro Ser Lys
1               5               10                  15

Lys Gln Gly Pro Trp Leu
        20


(2) INFORMATION FOR SEQ ID NO:71:

    (i) SEQUENCE CHARACTERISTICS:

        (A) LENGTH: 18 amino acids
        (B) TYPE: amino acid
        (D) TOPOLOGY: linear

    (ii) MOLECULE TYPE: peptide

    (xi) SEQUENCE DESCRIPTION: SEQ ID NO:71:


Gln Leu Gly Pro Gln Gly Pro Pro His Leu Val Ala Asp Pro Ser Lys
1               5               10                  15

Lys Gln


(2) INFORMATION FOR SEQ ID NO:72:

    (i) SEQUENCE CHARACTERISTICS:

        (A) LENGTH: 9 amino acids
        (B) TYPE: amino acid
        (D) TOPOLOGY: linear

(ii) MOLECULE TYPE: peptide

(xi) SEQUENCE DESCRIPTION: SEQ ID NO:72:


```
Gln Leu Gly Pro Gln Gly Pro Pro His
 1               5
```

(2) INFORMATION FOR SEQ ID NO:73:

(i) SEQUENCE CHARACTERISTICS:

(A) LENGTH: 11 amino acids
(B) TYPE: amino acid
(D) TOPOLOGY: linear

(ii) MOLECULE TYPE: peptide

(xi) SEQUENCE DESCRIPTION: SEQ ID NO:73:


```
Gln Leu Gly Pro Gln Gly Pro Pro Pro Pro Pro
 1               5                   10
```

(2) INFORMATION FOR SEQ ID NO:74:

(i) SEQUENCE CHARACTERISTICS:

(A) LENGTH: 17 amino acids
(B) TYPE: amino acid
(D) TOPOLOGY: linear

(ii) MOLECULE TYPE: peptide

(xi) SEQUENCE DESCRIPTION: SEQ ID NO:74:


```
Gln Gly Pro Trp Leu Glu Glu Glu Glu Glu Ala Tyr Gly Trp Met Asp
 1               5                   10                  15
Phe
```

(2) INFORMATION FOR SEQ ID NO:75:

(i) SEQUENCE CHARACTERISTICS:

(A) LENGTH: 12 amino acids
(B) TYPE: amino acid
(D) TOPOLOGY: linear

(ii) MOLECULE TYPE: peptide

(xi) SEQUENCE DESCRIPTION: SEQ ID NO:75:

```
Gln Gly Pro Trp Leu Glu Glu Glu Glu Glu Ala Tyr
 1               5                   10
```

(2) INFORMATION FOR SEQ ID NO:76:

    (i) SEQUENCE CHARACTERISTICS:

      (A) LENGTH: 8 amino acids
      (B) TYPE: amino acid
      (D) TOPOLOGY: linear

    (ii) MOLECULE TYPE: peptide

    (xi) SEQUENCE DESCRIPTION: SEQ ID NO:76:

```
Gln Gly Pro Trp Leu Glu Glu Glu
 1               5
```

(2) INFORMATION FOR SEQ ID NO:77:

    (i) SEQUENCE CHARACTERISTICS:

      (A) LENGTH: 27 amino acids
      (B) TYPE: amino acid
      (D) TOPOLOGY: linear

    (ii) MOLECULE TYPE: peptide

    (xi) SEQUENCE DESCRIPTION: SEQ ID NO:77:

```
Val Pro Leu Pro Ala Gly Gly Gly Thr Val Leu Thr Lys Met Tyr Pro
 1               5                   10                  15

Arg Gly Asn His Trp Ala Val Gly His Leu Met
         20                  25
```

(2) INFORMATION FOR SEQ ID NO:78:

    (i) SEQUENCE CHARACTERISTICS:

      (A) LENGTH: 10 amino acids
      (B) TYPE: amino acid
      (D) TOPOLOGY: linear

    (ii) MOLECULE TYPE: peptide

    (xi) SEQUENCE DESCRIPTION: SEQ ID NO:78:

```
Gly Asn His Trp Ala Val Gly His Leu Met
 1               5                   10
```

(2) INFORMATION FOR SEQ ID NO:79:

(i) SEQUENCE CHARACTERISTICS:

    (A) LENGTH: 10 amino acids
    (B) TYPE: amino acid
    (D) TOPOLOGY: linear

(ii) MOLECULE TYPE: peptide

(xi) SEQUENCE DESCRIPTION: SEQ ID NO:79:

```
Lys Thr Lys Gly Ser Gly Phe Phe Val Phe
 1               5                   10
```

(2) INFORMATION FOR SEQ ID NO:80:

(i) SEQUENCE CHARACTERISTICS:

    (A) LENGTH: 26 amino acids
    (B) TYPE: amino acid
    (D) TOPOLOGY: linear

(ii) MOLECULE TYPE: peptide

(ix) FEATURE:

    (A) NAME/KEY: Modified-site
    (B) LOCATION: 7
    (D) OTHER INFORMATION: /note= "A0.50;E0.50"

(ix) FEATURE:

    (A) NAME/KEY: Modified-site
    (B) LOCATION: 11
    (D) OTHER INFORMATION: /note= "S0.25;N0.25;K0.25;R0.25"

(ix) FEATURE:

    (A) NAME/KEY: Modified-site
    (B) LOCATION: 13
    (D) OTHER INFORMATION: /note= "T0.34;V0.33;A0.33"

(ix) FEATURE:

    (A) NAME/KEY: Modified-site
    (B) LOCATION: 14
    (D) OTHER INFORMATION: /note= "A0.50;E0.50"

(ix) FEATURE:

    (A) NAME/KEY: Modified-site
    (B) LOCATION: 15
    (D) OTHER INFORMATION: /note= "G0.50;D0.50"

(ix) FEATURE:

    (A) NAME/KEY: Modified-site

(B) LOCATION: 17
(D) OTHER INFORMATION: /note= "Q0.34;E0.33;S0.33"

(ix) FEATURE:

(A) NAME/KEY: Modified-site
(B) LOCATION: 18
(D) OTHER INFORMATION: /note= "N0.50;K0.50"

(ix) FEATURE:

(A) NAME/KEY: Modified-site
(B) LOCATION: 21
(D) OTHER INFORMATION: /note= "T0.34;V0.33;K0.33"

(ix) FEATURE:

(A) NAME/KEY: Modified-site
(B) LOCATION: 22
(D) OTHER INFORMATION: /note= "T0.34;A0.33;V0.33"

(xi) SEQUENCE DESCRIPTION: SEQ ID NO:80:


```
Glu Phe Gln Met Gly Ala Ala Pro Thr Thr Ser Asp Thr Ala Gly Leu
 1               5                  10                  15

Gln Asn Asp Pro Thr Thr Asn Val Ala Arg
              20              25
```

(2) INFORMATION FOR SEQ ID NO:81:

(i) SEQUENCE CHARACTERISTICS:

(A) LENGTH: 24 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 6
(D) OTHER INFORMATION: /note= "A0.50;D0.50"

(ix) FEATURE:

(A) NAME/KEY: Modified-site
(B) LOCATION: 11
(D) OTHER INFORMATION: /note= "T0.34;A0.33;S0.33"

(ix) FEATURE:

(A) NAME/KEY: Modified-site
(B) LOCATION: 12
(D) OTHER INFORMATION: /note= "T0.34;D0.33;S0.33"

(ix) FEATURE:

    (A) NAME/KEY: Modified-site
    (B) LOCATION: 15
    (D) OTHER INFORMATION: /note= "A0.50;S0.50"

(ix) FEATURE:

    (A) NAME/KEY: Modified-site
    (B) LOCATION: 16
    (D) OTHER INFORMATION: /note= "A0.34;T0.33;V0.33"

(ix) FEATURE:

    (A) NAME/KEY: Modified-site
    (B) LOCATION: 19
    (D) OTHER INFORMATION: /note= "S0.50;T0.50"

(ix) FEATURE:

    (A) NAME/KEY: Modified-site
    (B) LOCATION: 21
    (D) OTHER INFORMATION: /note= "L0.50;C0.50"

(xi) SEQUENCE DESCRIPTION: SEQ ID NO:81:


Glu Phe Gln Met Gly Ala Lys Pro Thr Thr Thr Thr Gly Asn Ala Ala
1               5                   10                  15

Ala Pro Ser Thr Leu Thr Ala Arg
                20


(2) INFORMATION FOR SEQ ID NO:82;

(i) SEQUENCE CHARACTERISTICS:

    (A) LENGTH: 25 amino acids
    (B) TYPE: amino acid
    (D) TOPOLOGY: linear

(ii) MOLECULE TYPE: peptide

(xi) SEQUENCE DESCRIPTION: SEQ ID NO:82:


Glu Phe Glu Met Gly Glu Ala Leu Ala Gly Ala Ser Gly Asn Thr Thr
1               5                   10                  15

Ser Thr Leu Ser Lys Leu Val Glu Arg
                20                  25


(2) INFORMATION FOR SEQ ID NO:83:

(i) SEQUENCE CHARACTERISTICS:

    (A) LENGTH: 26 amino acids

(B) TYPE: amino acid
(D) TOPOLOGY: linear

(ii) MOLECULE TYPE: peptide

(ix) FEATURE:

(A) NAME/KEY: Modified-site
(B) LOCATION: 6
(D) OTHER INFORMATION: /note= "Q0.50;K0.50"

(ix) FEATURE:

(A) NAME/KEY: Modified-site
(B) LOCATION: 7
(D) OTHER INFORMATION: /note= "S0.34;A0.33;Y0.33"

(ix) FEATURE:

(A) NAME/KEY: Modified-site
(B) LOCATION: 8
(D) OTHER INFORMATION: /note= "S0.50;T0.50"

(ix) FEATURE:

(A) NAME/KEY: Modified-site
(B) LOCATION: 9
(D) OTHER INFORMATION: /note= "N0.20;S0.20;G0.20;D0.20;K0.20"

(ix) FEATURE:

(A) NAME/KEY: Modified-site
(B) LOCATION: 11
(D) OTHER INFORMATION: /note= "N0.50;D0.50"

(ix) FEATURE:

(A) NAME/KEY: Modified-site
(B) LOCATION: 14
(D) OTHER INFORMATION: /note= "K0.50;N0.50"

(ix) FEATURE:

(A) NAME/KEY: Modified-site
(B) LOCATION: 15
(D) OTHER INFORMATION: /note= "L0.50;I0.50"

(ix) FEATURE:

(A) NAME/KEY: Modified-site
(B) LOCATION: 16
(D) OTHER INFORMATION: /note= "V0.50;F0.50"

(ix) FEATURE:

(A) NAME/KEY: Modified-site
(B) LOCATION: 19
(D) OTHER INFORMATION: /note= "T0.34;I0.33;A0.33"

(ix) FEATURE:

    (A) NAME/KEY: Modified-site
    (B) LOCATION: 22
    (D) OTHER INFORMATION: /note= "N0.50;D0.50"

(ix) FEATURE:

    (A) NAME/KEY: Modified-site
    (B) LOCATION: 23
    (D) OTHER INFORMATION: /note= "Q0.34;R0.33;E0.33"

(xi) SEQUENCE DESCRIPTION: SEQ ID NO:83:

```
Phe Gly Thr Lys Thr Gln Ser Ser Asn Phe Asn Thr Ala Lys Leu Val
 1               5                  10                  15

Pro Asn Thr Ala Leu Asn Gln Ala Val Val
            20                  25
```

(2) INFORMATION FOR SEQ ID NO:84:

    (i) SEQUENCE CHARACTERISTICS:

        (A) LENGTH: 24 amino acids
        (B) TYPE: amino acid
        (D) TOPOLOGY: linear

    (ii) MOLECULE TYPE: peptide

    (ix) FEATURE:

        (A) NAME/KEY: Modified-site
        (B) LOCATION: 3
        (D) OTHER INFORMATION: /note= "N0.50;D0.50"

    (ix) FEATURE:

        (A) NAME/KEY: Modified-site
        (B) LOCATION: 7
        (D) OTHER INFORMATION: /note= "Q0.50;H0.50"

    (ix) FEATURE:

        (A) NAME/KEY: Modified-site
        (B) LOCATION: 8
        (D) OTHER INFORMATION: /note= "T0.50;A0.50"

    (ix) FEATURE:

        (A) NAME/KEY: Modified-site
        (B) LOCATION: 9
        (D) OTHER INFORMATION: /note= "K0.50;T0.50"

    (ix) FEATURE:

(A) NAME/KEY: Modified-site
(B) LOCATION: 12
(D) OTHER INFORMATION: /note= "N0.50;D0.50"

(ix) FEATURE:

(A) NAME/KEY: Modified-site
(B) LOCATION: 13
(D) OTHER INFORMATION: /note= "S0.50;G0.50"

(ix) FEATURE:

(A) NAME/KEY: Modified-site
(B) LOCATION: 14
(D) OTHER INFORMATION: /note= "A0.34;T0.33;K0.33"

(ix) FEATURE:

(A) NAME/KEY: Modified-site
(B) LOCATION: 15
(D) OTHER INFORMATION: /note= "F0.50;L0.50"

(xi) SEQUENCE DESCRIPTION: SEQ ID NO:84:

```
Phe Gly Asn Asn Glu Asn Gln Thr Lys Val Ser Asn Ser Ala Phe Val Pro
1               5                   10                  15

Asn Met Ser Leu Asp Gln Ser Val Val
                20
```

(2) INFORMATION FOR SEQ ID NO:85:

(i) SEQUENCE CHARACTERISTICS:

(A) LENGTH: 25 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 4
(D) OTHER INFORMATION: /note= "N0.50;G0.50"

(ix) FEATURE:

(A) NAME/KEY: Modified-site
(B) LOCATION: 5
(D) OTHER INFORMATION: /note= "E0.50;V0.50"

(ix) FEATURE:

(A) NAME/KEY: Modified-site
(B) LOCATION: 7
(D) OTHER INFORMATION: /note= "Q0.50;A0.50"

(ix) FEATURE:

    (A) NAME/KEY: Modified-site
    (B) LOCATION: 8
    (D) OTHER INFORMATION: /note= "K0.34;S0.33;T0.33"

(ix) FEATURE:

    (A) NAME/KEY: Modified-site
    (B) LOCATION: 9
    (D) OTHER INFORMATION: /note= "T0.34;K0.33;Q0.33"

(ix) FEATURE:

    (A) NAME/KEY: Modified-site
    (B) LOCATION: 10
    (D) OTHER INFORMATION: /note= "V0.50;P0.50"

(ix) FEATURE:

    (A) NAME/KEY: Modified-site
    (B) LOCATION: 11
    (D) OTHER INFORMATION: /note= "K0.50;A0.50"

(ix) FEATURE:

    (A) NAME/KEY: Modified-site
    (B) LOCATION: 12
    (D) OTHER INFORMATION: /note= "A0.34;T0.33;K0.33"

(ix) FEATURE:

    (A) NAME/KEY: Modified-site
    (B) LOCATION: 13
    (D) OTHER INFORMATION: /note= "E0.25;N0.25;D0.25;T0.25"

(ix) FEATURE:

    (A) NAME/KEY: Modified-site
    (B) LOCATION: 14
    (D) OTHER INFORMATION: /note= "S0.50;A0.50"

(ix) FEATURE:

    (A) NAME/KEY: Modified-site
    (B) LOCATION: 15
    (D) OTHER INFORMATION: /note= "V0.50;I0.50"

(ix) FEATURE:

    (A) NAME/KEY: Modified-site
    (B) LOCATION: 18
    (D) OTHER INFORMATION: /note= "M0.50;V0.50"

(ix) FEATURE:

    (A) NAME/KEY: Modified-site
    (B) LOCATION: 19

(D) OTHER INFORMATION: /note= "S0.50;Q0.50"

(ix) FEATURE:

    (A) NAME/KEY: Modified-site
    (B) LOCATION: 20
    (D) OTHER INFORMATION: /note= "F0.50;L0.50"

(ix) FEATURE:

    (A) NAME/KEY: Modified-site
    (B) LOCATION: 21
    (D) OTHER INFORMATION: /note= "D0.50;N0.50"

(xi) SEQUENCE DESCRIPTION: SEQ ID NO:85:

```
Phe Gly Asp Asn Glu Asn Gln Lys Thr Val Lys Ala Glu Ser Val Pro
 1               5                   10                  15

Asn Met Ser Phe Asp Gln Ser Val Val
            20              25
```

(2) INFORMATION FOR SEQ ID NO:86:

(i) SEQUENCE CHARACTERISTICS:

    (A) LENGTH: 30 amino acids
    (B) TYPE: amino acid
    (D) TOPOLOGY: linear

(ii) MOLECULE TYPE: peptide

(ix) FEATURE:

    (A) NAME/KEY: Modified-site
    (B) LOCATION: 1
    (D) OTHER INFORMATION: /note= "S0.50;L0.50"

(ix) FEATURE:

    (A) NAME/KEY: Modified-site
    (B) LOCATION: 3
    (D) OTHER INFORMATION: /note= "T0.34;E0.33;K0.33"

(ix) FEATURE:

    (A) NAME/KEY: Modified-site
    (B) LOCATION: 4
    (D) OTHER INFORMATION: /note= "A0.34;T0.33;P0.33"

(ix) FEATURE:

    (A) NAME/KEY: Modified-site
    (B) LOCATION: 5
    (D) OTHER INFORMATION: /note= "I0.50;V0.50"

(ix) FEATURE:

    (A) NAME/KEY: Modified-site
    (B) LOCATION: 6
    (D) OTHER INFORMATION: /note= "F0.50;L0.50"

(ix) FEATURE:

    (A) NAME/KEY: Modified-site
    (B) LOCATION: 8
    (D) OTHER INFORMATION: /note= "T0.50;V0.50"

(ix) FEATURE:

    (A) NAME/KEY: Modified-site
    (B) LOCATION: 18
    (D) OTHER INFORMATION: /note= "A0.50;K0.50"

(ix) FEATURE:

    (A) NAME/KEY: Modified-site
    (B) LOCATION: 20
    (D) OTHER INFORMATION: /note= "D0.34;T0.33;E0.33"

(ix) FEATURE:

    (A) NAME/KEY: Modified-site
    (B) LOCATION: 22
    (D) OTHER INFORMATION: /note= "K0.50;V0.50"

(ix) FEATURE:

    (A) NAME/KEY: Modified-site
    (B) LOCATION: 23
    (D) OTHER INFORMATION: /note= "T0.34;A0.33;S0.33"

(ix) FEATURE:

    (A) NAME/KEY: Modified-site
    (B) LOCATION: 24
    (D) OTHER INFORMATION: /note= "S0.34;G0.33;N0.33"

(ix) FEATURE:

    (A) NAME/KEY: Modified-site
    (B) LOCATION: 27
    (D) OTHER INFORMATION: /note= "G0.50;N0.50"

(ix) FEATURE:

    (A) NAME/KEY: Modified-site
    (B) LOCATION: 28
    (D) OTHER INFORMATION: /note= "Q0.50;E0.50"

(ix) FEATURE:

    (A) NAME/KEY: Modified-site
    (B) LOCATION: 30

(D) OTHER INFORMATION: /note= "G0.50;A0.50"

(xi) SEQUENCE DESCRIPTION: SEQ ID NO:86:

Ser Ala Thr Ala Ile Phe Asp Thr Thr Thr Leu Asn Pro Thr Ile Ala
1               5                   10                  15

Gly Ala Gly Asp Val Lys Thr Ser Ala Glu Gly Gln Leu Gly
                20              25                  30


(2) INFORMATION FOR SEQ ID NO:87:

(i) SEQUENCE CHARACTERISTICS:

(A) LENGTH: 31 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 3
(D) OTHER INFORMATION: /note= "E0.34;T0.33;K0.33"

(ix) FEATURE:

(A) NAME/KEY: Modified-site
(B) LOCATION: 4
(D) OTHER INFORMATION: /note= "A0.50;P0.50"

(ix) FEATURE:

(A) NAME/KEY: Modified-site
(B) LOCATION: 5
(D) OTHER INFORMATION: /note= "I0.50;V0.50"

(ix) FEATURE:

(A) NAME/KEY: Modified-site
(B) LOCATION: 6
(D) OTHER INFORMATION: /note= "L0.50;V0.50"

(ix) FEATURE:

(A) NAME/KEY: Modified-site
(B) LOCATION: 8
(D) OTHER INFORMATION: /note= "V0.50;I0.50"

(ix) FEATURE:

(A) NAME/KEY: Modified-site
(B) LOCATION: 16
(D) OTHER INFORMATION: /note= "A0.50;T0.50"

(ix) FEATURE:

(A) NAME/KEY: Modified-site
(B) LOCATION: 18
(D) OTHER INFORMATION: /note= "K0.50;C0.50"

(ix) FEATURE:

(A) NAME/KEY: Modified-site
(B) LOCATION: 20
(D) OTHER INFORMATION: /note= "S0.34;T0.33;A0.33"

(ix) FEATURE:

(A) NAME/KEY: Modified-site
(B) LOCATION: 22
(D) OTHER INFORMATION: /note= "V0.50;A0.50"

(ix) FEATURE:

(A) NAME/KEY: Modified-site
(B) LOCATION: 23
(D) OTHER INFORMATION: /note= "A0.34;S0.33;G0.33"

(ix) FEATURE:

(A) NAME/KEY: Modified-site
(B) LOCATION: 24
(D) OTHER INFORMATION: /note= "S0.50;A0.50"

(ix) FEATURE:

(A) NAME/KEY: Modified-site
(B) LOCATION: 25
(D) OTHER INFORMATION: /note= "G0.50;NO.50"

(ix) FEATURE:

(A) NAME/KEY: Modified-site
(B) LOCATION: 26
(D) OTHER INFORMATION: /note= "S0.50;T0.50"

(ix) FEATURE:

(A) NAME/KEY: Modified-site
(B) LOCATION: 27
(D) OTHER INFORMATION: /note= "E0.50;D0.50"

(ix) FEATURE:

(A) NAME/KEY: Modified-site
(B) LOCATION: 28
(D) OTHER INFORMATION: /note= "N0.50;G0.50"

(ix) FEATURE:

(A) NAME/KEY: Modified-site
(B) LOCATION: 29

(D) OTHER INFORMATION: /note= "E0.34;D0.33;Q0.33"

(ix) FEATURE:

(A) NAME/KEY: Modified-site
(B) LOCATION: 30
(D) OTHER INFORMATION: /note= "L0.50;I0.50"

(ix) FEATURE:

(A) NAME/KEY: Modified-site
(B) LOCATION: 31
(D) OTHER INFORMATION: /note= "A0.50;S0.50"

(xi) SEQUENCE DESCRIPTION: SEQ ID NO:87:


```
Leu Ala Glu Ala Ile Leu Asp Val Thr Thr Leu Asn Pro Thr Ile Ala
 1               5                  10                  15

Gly Lys Gly Ser Val Val Ala Ser Gly Ser Glu Asn Glu Leu Ala
             20                  25                  30
```


 (2) INFORMATION FOR SEQ ID NO:88:

(i) SEQUENCE CHARACTERISTICS:

(A) LENGTH: 24 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 1
(D) OTHER INFORMATION: /note= "K0.50;T0.50"

(ix) FEATURE:

(A) NAME/KEY: Modified-site
(B) LOCATION: 6
(D) OTHER INFORMATION: /note= "A0.34;K0.33;Q0.33"

(ix) FEATURE:

(A) NAME/KEY: Modified-site
(B) LOCATION: 8
(D) OTHER INFORMATION: /note= "F0.50;L0.50"

(ix) FEATURE:

(A) NAME/KEY: Modified-site
(B) LOCATION: 11
(D) OTHER INFORMATION: /note= "D0.34;A0.33;N0.33"

(ix) FEATURE:

(A) NAME/KEY: Modified-site
(B) LOCATION: 12
(D) OTHER INFORMATION: /note= "I0.50;L0.50"

(ix) FEATURE:

(A) NAME/KEY: Modified-site
(B) LOCATION: 13
(D) OTHER INFORMATION: /note= "T0.50;I0.50"

(ix) FEATURE:

(A) NAME/KEY: Modified-site
(B) LOCATION: 14
(D) OTHER INFORMATION: /note= "A0.50;S0.50"

(ix) FEATURE:

(A) NAME/KEY: Modified-site
(B) LOCATION: 17
(D) OTHER INFORMATION: /note= "E0.50;D0.50"

(ix) FEATURE:

(A) NAME/KEY: Modified-site
(B) LOCATION: 24
(D) OTHER INFORMATION: /note= "D0.50;A0.50"

(xi) SEQUENCE DESCRIPTION: SEQ ID NO:88:


```
Lys Gly Tyr Val Gly Ala Glu Phe Pro Leu Asp Ile Thr Ala Gly Thr
 1               5                  10                  15

Glu Ala Ala Thr Gly Thr Lys Asp
                20
```


(2) INFORMATION FOR SEQ ID NO:89:

(i) SEQUENCE CHARACTERISTICS:

(A) LENGTH: 24 amino acids
(B) TYPE: amino acid
(D) TOPOLOGY: linear

(ii) MOLECULE TYPE: peptide

(xi) SEQUENCE DESCRIPTION: SEQ ID NO:89:


```
Lys Gly Tyr Val Gly Ala Glu Phe Pro Leu Asp Leu Lys Ala Gly Thr
 1               5                  10                  15

Asp Gly Val Thr Gly Thr Lys Asp
                20
```

(2) INFORMATION FOR SEQ ID NO:90:

    (i) SEQUENCE CHARACTERISTICS:

        (A) LENGTH: 32 amino acids
        (B) TYPE: amino acid
        (D) TOPOLOGY: linear

    (ii) MOLECULE TYPE: peptide

    (xi) SEQUENCE DESCRIPTION: SEQ ID NO:90:

```
Glu Ser Val Gln Ile Asn Cys Thr Arg Pro Asn Tyr Asn Lys Arg Lys
 1               5                  10                  15

Arg Ile His Ile Gly Pro Gly Arg Ala Phe Tyr Thr Thr Lys Asn Met
               20                  25                  30
```

(2) INFORMATION FOR SEQ ID NO:91:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide

    (xi) SEQUENCE DESCRIPTION: SEQ ID NO:91:

```
Asn Asn Asn Asp Asp Ser Tyr Ile Pro Ser Ala Glu Lys Ile Leu Glu
 1               5                  10                  15

Phe Val Lys Gln
               20
```

(2) INFORMATION FOR SEQ ID NO:92:

    (i) SEQUENCE CHARACTERISTICS:

        (A) LENGTH: 55 amino acids
        (B) TYPE: amino acid
        (D) TOPOLOGY: linear

    (ii) MOLECULE TYPE: peptide

    (xi) SEQUENCE DESCRIPTION: SEQ ID NO:92:

```
Lys Cys Asn Thr Ala Thr Cys Ala Thr Gln Arg Leu Ala Asn Phe Leu
 1               5                   10                  15

Val His Ser Ser Asn Asn Phe Gly Ala Ile Leu Ser Ser Thr Asn Val Gly
             20                  25                  30

Ser Asn Thr Tyr Gly Gly Phe Phe Leu Leu Thr Arg Ile Leu Thr Ile Pro
    35                  40                  45                  50

Gln Ser Leu Asp Gly Gly Thr Ala Lys Ser Lys Lys Phe Pro Ser Tyr Thr
            55                  60                  65

Ala Thr Tyr Gln Phe
            70
```

(2) INFORMATION FOR SEQ ID NO:93:

  (i) SEQUENCE CHARACTERISTICS:

    (A) LENGTH: 55 amino acids
    (B) TYPE: amino acid
    (D) TOPOLOGY: linear

  (ii) MOLECULE TYPE: peptide

  (xi) SEQUENCE DESCRIPTION: SEQ ID NO:93:

```
Lys Cys Asn Thr Ala Thr Cys Ala Thr Gln Arg Leu Ala Asn Phe Leu
 1               5                   10                  15

Val His Ser Ser Gly Gly Phe Phe Leu Leu Thr Arg Ile Leu Thr Ile
             20                  25                  30

Pro Gln Ser Leu Asp Gly Gly Thr Ala Lys Ser Lys Lys Phe Pro Ser
        35                  40                  45

Tyr Thr Ala Thr Tyr Gln Phe
                50                  55
```

(2) INFORMATION FOR SEQ ID NO:94:

  (i) SEQUENCE CHARACTERISTICS:

    (A) LENGTH: 33 amino acids
    (B) TYPE: amino acid
    (D) TOPOLOGY: linear

  (ii) MOLECULE TYPE: peptide

  (xi) SEQUENCE DESCRIPTION: SEQ ID NO:94:

```

```
Thr Ala Lys Ser Lys Lys Phe Pro Ser Tyr Thr Ala Thr Tyr Gln Phe
1               5                   10                  15

Gly Gly Phe Phe Leu Leu Thr Arg Ile Leu Thr Ile Pro Gln Ser Leu
            20                  25                  30

Asp Gly Gly His Ser Ser Asn Asn Phe Gly Ala Ile Leu Ser Ser Thr
            35                  40                  45

Asn Val Gly Ser Asn Thr Tyr
        50                  55
```

(2) INFORMATION FOR SEQ ID NO:95:

    (i) SEQUENCE CHARACTERISTICS:

        (A) LENGTH: 63 amino acids
        (B) TYPE: amino acid
        (D) TOPOLOGY: linear

    (ii) MOLECULE TYPE: peptide

    (xi) SEQUENCE DESCRIPTION: SEQ ID NO:95:

```
Gln Leu Gly Pro Gln Gly Pro Pro His Leu Val Ala Asp Pro Ser Lys Lys
1               5                   10                  15

Gln Gly Pro Trp Leu Glu Glu Glu Glu Glu Ala Tyr Gly Trp Met Asp Phe
            20                  25                  30

Gly Gly Phe Phe Leu Leu Thr Arg Ile Leu Thr Ile Pro Gln Ser Leu Asp
            35                  40                  45

Gly Gly Thr Ala Lys Ser Lys Lys Phe Pro Ser Tyr Thr Ala Thr Tyr Gln
        50                  55                  60                  65

                            Phe
```

(2) INFORMATION FOR SEQ ID NO:96:

    (i) SEQUENCE CHARACTERISTICS:

        (A) LENGTH: 57 amino acids
        (B) TYPE: amino acid
        (D) TOPOLOGY: linear

    (ii) MOLECULE TYPE: peptide

    (xi) SEQUENCE DESCRIPTION: SEQ ID NO:96:

```
Gln Leu Gly Pro Gln Gly Pro Pro His Leu Val Ala Asp Pro Ser Lys
 1               5               10                  15
Lys Gln Gly Pro Trp Leu Gly Gly Phe Phe Leu Leu Thr Arg Ile Leu
            20              25                  30
Thr Ile Pro Gln Ser Leu Asp Gly Gly Thr Ala Lys Ser Lys Lys Phe
            35              40                  45
Pro Ser Tyr Thr Ala Thr Tyr Gln Phe
    50                  55
```

(2) INFORMATION FOR SEQ ID NO:97:

    (i) SEQUENCE CHARACTERISTICS:

        (A) LENGTH: 53 amino acids
        (B) TYPE: amino acid
        (D) TOPOLOGY: linear

    (ii) MOLECULE TYPE: peptide

    (xi) SEQUENCE DESCRIPTION: SEQ ID NO:97:

```
Gln Leu Gly Pro Gln Gly Pro Pro His Leu Val Ala Asp Pro Ser Lys
 1               5               10                  15
Lys Gln Gly Gly Phe Phe Leu Leu Thr Arg Ile Leu Thr Ile Pro Gln
            20              25                  30
Ser Leu Asp Gly Gly Thr Ala Lys Ser Lys Lys Phe Pro Ser Tyr Thr
            35                  40                  45
Ala Thr Tyr Gln Phe
    50
```

(2) INFORMATION FOR SEQ ID NO:98:

    (i) SEQUENCE CHARACTERISTICS:

        (A) LENGTH: 44 amino acids
        (B) TYPE: amino acid
        (D) TOPOLOGY: linear

    (ii) MOLECULE TYPE: peptide

    (xi) SEQUENCE DESCRIPTION: SEQ ID NO:98:

```

```
Gln Leu Gly Pro Gln Gly Pro Pro His Gly Gly Phe Phe Leu Leu Thr
1               5               10              15
Arg Ile Leu Thr Ile Pro Gln Ser Leu Asp Gly Gly Thr Ala Lys Ser
                20              25              30
Lys Lys Phe Pro Ser Tyr Thr Ala Thr Tyr Gln Phe
        35              40
```

(2) INFORMATION FOR SEQ ID NO:99:

    (i) SEQUENCE CHARACTERISTICS:

        (A) LENGTH: 50 amino acids
        (B) TYPE: amino acid
        (D) TOPOLOGY: linear

    (ii) MOLECULE TYPE: peptide

    (xi) SEQUENCE DESCRIPTION: SEQ ID NO:99:

```
Thr Ala Lys Ser Lys Lys Phe Pro Ser Tyr Thr Ala Thr Tyr Gln Phe
1               5               10              15
Gly Gly Phe Phe Leu Leu Thr Arg Ile Leu Thr Ile Pro Gln Ser Leu
                20              25              30
Asp Gly Gly Gln Gly Pro Trp Leu Glu Glu Glu Glu Ala Tyr Gly Trp
        35              40              45
Met Asp Phe
    50
```

(2) INFORMATION FOR SEQ ID NO:100:

    (i) SEQUENCE CHARACTERISTICS:

        (A) LENGTH: 45 amino acids
        (B) TYPE: amino acid
        (D) TOPOLOGY: linear

    (ii) MOLECULE TYPE: peptide

    (xi) SEQUENCE DESCRIPTION: SEQ ID NO:100:

```
Thr Ala Lys Ser Lys Lys Phe Pro Ser Tyr Thr Ala Thr Tyr Gln Phe
1               5               10              15
Gly Gly Phe Phe Leu Leu Thr Arg Ile Leu Thr Ile Pro Gln Ser Leu
                20              25              30
Asp Gly Gly Gln Gly Pro Trp Leu Glu Glu Glu Glu Glu Ala Tyr
        35              40              45
```

(2) INFORMATION FOR SEQ ID NO:101:

(i) SEQUENCE CHARACTERISTICS:

    (A) LENGTH: 62 amino acids
    (B) TYPE: amino acid
    (D) TOPOLOGY: linear

(ii) MOLECULE TYPE: peptide

(xi) SEQUENCE DESCRIPTION: SEQ ID NO:101:

```
Val Pro Leu Pro Ala Gly Gly Gly Thr Val Leu Thr Lys Met Tyr Pro
 1               5                   10                  15
Arg Gly Asn His Trp Ala Val Gly His Leu Met Gly Gly Phe Phe Leu
                20                  25                  30
Leu Thr Arg Ile Leu Thr Ile Pro Gln Ser Leu Asp Gly Gly Thr Ala
            35                  40                  45
Lys Ser Lys Lys Phe Pro Ser Tyr Thr Ala Thr Tyr Gln Phe
        50                  55                  60
```

(2) INFORMATION FOR SEQ ID NO:102:

(i) SEQUENCE CHARACTERISTICS:

    (A) LENGTH: 43 amino acids
    (B) TYPE: amino acid
    (D) TOPOLOGY: linear

(ii) MOLECULE TYPE: peptide

(xi) SEQUENCE DESCRIPTION: SEQ ID NO:102:

```
Thr Ala Lys Ser Lys Lys Phe Pro Ser Tyr Thr Ala Thr Tyr Gln Phe
 1               5                   10                  15
Gly Gly Phe Phe Leu Leu Thr Arg Ile Leu Thr Ile Pro Gln Ser Leu
                20                  25                  30
Asp Gly Gly Gly Asn His Trp Ala Val Gly His Leu Met
            35                  40                  45
```

(2) INFORMATION FOR SEQ ID NO:103:

(i) SEQUENCE CHARACTERISTICS:

    (A) LENGTH: 45 amino acids
    (B) TYPE: amino acid
    (D) TOPOLOGY: linear

(ii) MOLECULE TYPE: peptide

(xi) SEQUENCE DESCRIPTION: SEQ ID NO:103:

```
Lys Thr Lys Gly Ser Gly Phe Phe Val Phe Gly Gly Phe Phe Leu Leu
 1               5               10              15

Thr Arg Ile Leu Thr Ile Pro Gln Ser Leu Asp Gly Gly Thr Ala Lys
             20              25              30

Ser Lys Lys Phe Pro Ser Tyr Thr Ala Thr Tyr Gln Phe
         35              40              45
```

(2) INFORMATION FOR SEQ ID NO:104:

    (i) SEQUENCE CHARACTERISTICS:

        (A) LENGTH: 33 amino acids
        (B) TYPE: amino acid
        (D) TOPOLOGY: linear

    (ii) MOLECULE TYPE: peptide

    (xi) SEQUENCE DESCRIPTION: SEQ ID NO:104:

```
Thr Ala Lys Ser Lys Lys Phe Pro Ser Tyr Thr Ala Thr Tyr Gln Phe
 1               5               10              15

Gly Gly Phe Phe Leu Leu Thr Arg Ile Leu Thr Ile Pro Gln Ser Leu
             20              25              30

Asp Gly Gly Lys Thr Lys Gly Ser Gly Phe Phe Val Phe
         35              40              45
```

(2) INFORMATION FOR SEQ ID NO:105:

    (i) SEQUENCE CHARACTERISTICS:

        (A) LENGTH: 63 amino acids
        (B) TYPE: amino acid
        (D) TOPOLOGY: linear

    (ii) MOLECULE TYPE: peptide

    (ix) FEATURE:

        (A) NAME/KEY: Modified-site
        (B) LOCATION: 7
        (D) OTHER INFORMATION: /note= "A0.50;E0.50"

    (ix) FEATURE:

        (A) NAME/KEY: Modified-site
        (B) LOCATION: 11
        (D) OTHER INFORMATION: /note= "S0.25;N0.25;K0.25;R0.25"

    (ix) FEATURE:

(A) NAME/KEY: Modified-site
(B) LOCATION: 13
(D) OTHER INFORMATION: /note= "T0.34;V0.33;A0.33"

(ix) FEATURE:

(A) NAME/KEY: Modified-site
(B) LOCATION: 14
(D) OTHER INFORMATION: /note= "A0.50;E0.50"

(ix) FEATURE:

(A) NAME/KEY: Modified-site
(B) LOCATION: 15
(D) OTHER INFORMATION: /note= "G0.50;D0.50"

(ix) FEATURE:

(A) NAME/KEY: Modified-site
(B) LOCATION: 17
(D) OTHER INFORMATION: /note= "Q0.34;E0.33;S0.33"

(ix) FEATURE:

(A) NAME/KEY: Modified-site
(B) LOCATION: 18
(D) OTHER INFORMATION: /note= "N0.50;K0.50"

(ix) FEATURE:

(A) NAME/KEY: Modified-site
(B) LOCATION: 21
(D) OTHER INFORMATION: /note= "T0.34;V0.33;K0.33"

(ix) FEATURE:

(A) NAME/KEY: Modified-site
(B) LOCATION: 22
(D) OTHER INFORMATION: /note= "T0.34;A0.33;V0.33"

(xi) SEQUENCE DESCRIPTION: SEQ ID NO:105:

```
Glu Phe Gln Met Gly Ala Ala Pro Thr Thr Ser Asp Thr Ala Gly Leu
 1               5                   10                  15

Gln Asn Asp Pro Thr Thr Asn Val Ala Arg Gly Gly Phe Phe Leu Leu
             20                  25                  30

Thr Arg Ile Leu Thr Gly Gly Ile Pro Gln Ser Leu Asp Gly Gly Thr
             35                  40                  45

Ala Lys Ser Lys Lys Phe Pro Ser Tyr Thr Ala Thr Tyr Gln Phe
         50                  55                  60
```

(2) INFORMATION FOR SEQ ID NO:106:

(i) SEQUENCE CHARACTERISTICS:

(A) LENGTH: 59 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 6
(D) OTHER INFORMATION: /note= "A0.50;D0.50"

(ix) FEATURE:

(A) NAME/KEY: Modified-site
(B) LOCATION: 11
(D) OTHER INFORMATION: /note= "T0.34;A0.33;S0.33"

(ix) FEATURE:

(A) NAME/KEY: Modified-site
(B) LOCATION: 12
(D) OTHER INFORMATION: /note= "T0.34;D0.33;S0.33"

(ix) FEATURE:

(A) NAME/KEY: Modified-site
(B) LOCATION: 15
(D) OTHER INFORMATION: /note= "A0.50;S0.50"

(ix) FEATURE:

(A) NAME/KEY: Modified-site
(B) LOCATION: 16
(D) OTHER INFORMATION: /note= "A0.34;T0.33;V0.33"

(ix) FEATURE:

(A) NAME/KEY: Modified-site
(B) LOCATION: 19
(D) OTHER INFORMATION: /note= "S0.50;T0.50"

(ix) FEATURE:

(A) NAME/KEY: Modified-site
(B) LOCATION: 21
(D) OTHER INFORMATION: /note= "L0.50;C0.50"

(xi) SEQUENCE DESCRIPTION: SEQ ID NO:106:

```
Glu Phe Gln Met Gly Ala Lys Pro Thr Thr Thr Thr Gly Asn Ala Ala
1               5                   10              15

Ala Pro Ser Thr Leu Thr Ala Arg Gly Gly Phe Phe Leu Leu Thr Arg
            20              25                  30

Ile Leu Thr Ile Pro Gln Ser Leu Asp Gly Gly Thr Ala Lys Ser Lys
            35              40              45

Lys Phe Pro Ser Tyr Thr Ala Thr Tyr Gln Phe
    50              55
```

(2) INFORMATION FOR SEQ ID NO:107:

(i) SEQUENCE CHARACTERISTICS:

(A) LENGTH: 60 amino acids
(B) TYPE: amino acid
(D) TOPOLOGY: linear

(ii) MOLECULE TYPE: peptide

(xi) SEQUENCE DESCRIPTION: SEQ ID NO:107:

```
Glu Phe Glu Met Gly Glu Ala Leu Ala Gly Ala Ser Gly Asn Thr Thr
1               5                   10              15

Ser Thr Leu Ser Lys Leu Val Glu Arg Gly Gly Phe Phe Leu Leu Thr
            20              25                  30

Arg Ile Leu Thr Ile Pro Gln Ser Leu Asp Gly Gly Thr Ala Lys Ser
            35              40              45

Lys Lys Phe Pro Ser Tyr Thr Ala Thr Tyr Gln Phe
    50              55                  60
```

(2) INFORMATION FOR SEQ ID NO:108:

(i) SEQUENCE CHARACTERISTICS:

(A) LENGTH: 61 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 6
(D) OTHER INFORMATION: /note= "Q0.50;K0.50"

(ix) FEATURE:

(A) NAME/KEY: Modified-site
(B) LOCATION: 7

(D) OTHER INFORMATION: /note= "S0.34;A0.33;Y0.33"

(ix) FEATURE:

    (A) NAME/KEY: Modified-site
    (B) LOCATION: 8
    (D) OTHER INFORMATION: /note= "S0.50;T0.50"

(ix) FEATURE:

    (A) NAME/KEY: Modified-site
    (B) LOCATION: 9
    (D) OTHER INFORMATION: /note= "N0.20;S0.20;G0.20;D0.20;K0.20"

(ix) FEATURE:

    (A) NAME/KEY: Modified-site
    (B) LOCATION: 11
    (D) OTHER INFORMATION: /note= "N0.50;D0.50"

(ix) FEATURE:

    (A) NAME/KEY: Modified-site
    (B) LOCATION: 14
    (D) OTHER INFORMATION: /note= "K0.50;N0.50"

(ix) FEATURE:

    (A) NAME/KEY: Modified-site
    (B) LOCATION: 15
    (D) OTHER INFORMATION: /note= "L0.50;I0.50"

(ix) FEATURE:

    (A) NAME/KEY: Modified-site
    (B) LOCATION: 16
    (D) OTHER INFORMATION: /note= "V0.50;F0.50"

(ix) FEATURE:

    (A) NAME/KEY: Modified-site
    (B) LOCATION: 19
    (D) OTHER INFORMATION: /note= "T0.34;I0.33;A0.33"

(ix) FEATURE:

    (A) NAME/KEY: Modified-site
    (B) LOCATION: 22
    (D) OTHER INFORMATION: /note= "N0.50;D0.50"

(ix) FEATURE:

    (A) NAME/KEY: Modified-site
    (B) LOCATION: 23
    (D) OTHER INFORMATION: /note= "Q0.34;R0.33;E0.33"

(xi) SEQUENCE DESCRIPTION: SEQ ID NO:108:

```
Phe Gly Thr Lys Thr Gln Ser Ser Asn Phe Asn Thr Ala Lys Leu Val
1               5                   10                  15

Pro Asn Thr Ala Leu Asn Gln Ala Val Val Gly Gly Phe Phe Leu Leu
            20                  25                  30

Thr Arg Ile Leu Thr Ile Pro Gln Ser Leu Asp Gly Gly Thr Ala Lys
        35                  40                  45

Ser Lys Lys Phe Pro Ser Tyr Thr Ala Thr Tyr Gln Phe
    50                  55                  60
```

(2) INFORMATION FOR SEQ ID NO:109:

    (i) SEQUENCE CHARACTERISTICS:

        (A) LENGTH: 57 amino acids
        (B) TYPE: amino acid
        (D) TOPOLOGY: linear

    (ii) MOLECULE TYPE: peptide

    (ix) FEATURE:

        (A) NAME/KEY: Modified-site
        (B) LOCATION: 3
        (D) OTHER INFORMATION: /note= "N0.50;D0.50"

    (ix) FEATURE:

        (A) NAME/KEY: Modified-site
        (B) LOCATION: 7
        (D) OTHER INFORMATION: /note= "Q0.50;H0.50"

    (ix) FEATURE:

        (A) NAME/KEY: Modified-site
        (B) LOCATION: 8
        (D) OTHER INFORMATION: /note= "T0.50;A0.50"

    (ix) FEATURE:

        (A) NAME/KEY: Modified-site
        (B) LOCATION: 9
        (D) OTHER INFORMATION: /note= "K0.50;T0.50"

    (ix) FEATURE:

        (A) NAME/KEY: Modified-site
        (B) LOCATION: 12
        (D) OTHER INFORMATION: /note= "N0.50;D0.50"

    (ix) FEATURE:

        (A) NAME/KEY: Modified-site
        (B) LOCATION: 13
        (D) OTHER INFORMATION: /note= "S0.50;G0.50"

(ix) FEATURE:

    (A) NAME/KEY: Modified-site
    (B) LOCATION: 14
    (D) OTHER INFORMATION: /note= "A0.34;T0.33;K0.33"

(ix) FEATURE:

    (A) NAME/KEY: Modified-site
    (B) LOCATION: 15
    (D) OTHER INFORMATION: /note= "F0.50;L0.50"

(xi) SEQUENCE DESCRIPTION: SEQ ID NO:109:

```
Phe Gly Asn Asn Glu Asn Gln Thr Lys Val Ser Asn Ser Ala Phe Val
 1               5                  10                  15

Pro Asn Met Ser Leu Asp Gln Ser Val Val Gly Gly Phe Phe Leu Leu
            20                  25                  30

Thr Arg Ile Leu Thr Ile Pro Gln Ser Leu Asp Gly Gly Thr Ala Lys
            35                  40                  45

Ser Lys Lys Phe Pro Ser Tyr Thr Ala Gln Phe
        50                  55
```

(2) INFORMATION FOR SEQ ID NO:110:

    (i) SEQUENCE CHARACTERISTICS:

        (A) LENGTH: 60 amino acids
        (B) TYPE: amino acid
        (D) TOPOLOGY: linear

    (ii) MOLECULE TYPE: peptide

    (ix) FEATURE:

        (A) NAME/KEY: Modified-site
        (B) LOCATION: 4
        (D) OTHER INFORMATION: /note= "N0.50;G0.50"

    (ix) FEATURE:

        (A) NAME/KEY: Modified-site
        (B) LOCATION: 5
        (D) OTHER INFORMATION: /note= "E0.50;V0.50"

    (ix) FEATURE:

        (A) NAME/KEY: Modified-site
        (B) LOCATION: 7
        (D) OTHER INFORMATION: /note= "Q0.50;A0.50"

    (ix) FEATURE:

        (A) NAME/KEY: Modified-site

(B) LOCATION: 8
(D) OTHER INFORMATION: /note= "K0.34;S0.33;T0.33"

(ix) FEATURE:

(A) NAME/KEY: Modified-site
(B) LOCATION: 9
(D) OTHER INFORMATION: /note= "T0.34;K0.33;Q0.33"

(ix) FEATURE:

(A) NAME/KEY: Modified-site
(B) LOCATION: 10
(D) OTHER INFORMATION: /note= "V0.50;P0.50"

(ix) FEATURE:

(A) NAME/KEY: Modified-site
(B) LOCATION: 11
(D) OTHER INFORMATION: /note= "K0.50;A0.50"

(ix) FEATURE:

(A) NAME/KEY: Modified-site
(B) LOCATION: 12
(D) OTHER INFORMATION: /note= "A0.34;T0.33;K0.33"

(ix) FEATURE:

(A) NAME/KEY: Modified-site
(B) LOCATION: 13
(D) OTHER INFORMATION: /note= "E0.25;N0.25;D0.25;T0.25"

(ix) FEATURE:

(A) NAME/KEY: Modified-site
(B) LOCATION: 14
(D) OTHER INFORMATION: /note= "S0.50;A0.50"

(ix) FEATURE:

(A) NAME/KEY: Modified-site
(B) LOCATION: 15
(D) OTHER INFORMATION: /note= "V0.50;I0.50"

(ix) FEATURE:

(A) NAME/KEY: Modified-site
(B) LOCATION: 18
(D) OTHER INFORMATION: /note= "M0.50;V0.50"

(ix) FEATURE:

(A) NAME/KEY: Modified-site
(B) LOCATION: 19
(D) OTHER INFORMATION: /note= "S0.50;Q0.50"

(ix) FEATURE:

(A) NAME/KEY: Modified-site
(B) LOCATION: 20
(D) OTHER INFORMATION: /note= "F0.50;L0.50"

(ix) FEATURE:

(A) NAME/KEY: Modified-site
(B) LOCATION: 21
(D) OTHER INFORMATION: /note= "D0.50;N0.50"

(xi) SEQUENCE DESCRIPTION: SEQ ID NO:110:

```
Phe Gly Asp Asn Glu Asn Gln Lys Thr Val Lys Ala Glu Ser Val Pro
1               5                   10                  15

Asn Met Ser Phe Asp Gln Ser Val Val Gly Gly Phe Phe Leu Leu Thr
            20                  25                  30

Arg Ile Leu Thr Ile Pro Gln Ser Leu Asp Gly Gly Thr Ala Lys Ser
            35                  40                  45

Lys Lys Phe Pro Ser Tyr Thr Ala Thr Tyr Gln Phe
        50                  55                  60
```

(2) INFORMATION FOR SEQ ID NO:111:

(i) SEQUENCE CHARACTERISTICS:

(A) LENGTH: 65 amino acids
(B) TYPE: amino acid
(D) TOPOLOGY: linear

(ii) MOLECULE TYPE: peptide

(ix) FEATURE:

(A) NAME/KEY: Modified-site
(B) LOCATION: 1
(D) OTHER INFORMATION: /note= "S0.50;L0.50"

(ix) FEATURE:

(A) NAME/KEY: Modified-site
(B) LOCATION: 3
(D) OTHER INFORMATION: /note= "T0.34;E0.33;K0.33"

(ix) FEATURE:

(A) NAME/KEY: Modified-site
(B) LOCATION: 4
(D) OTHER INFORMATION: /note= "A0.34;T0.33;P0.33"

(ix) FEATURE:

(A) NAME/KEY: Modified-site
(B) LOCATION: 5
(D) OTHER INFORMATION: /note= "I0.50;V0.50"

(ix) FEATURE:

    (A) NAME/KEY: Modified-site
    (B) LOCATION: 6
    (D) OTHER INFORMATION: /note= "F0.50;L0.50"

(ix) FEATURE:

    (A) NAME/KEY: Modified-site
    (B) LOCATION: 8
    (D) OTHER INFORMATION: /note= "T0.50;V0.50"

(ix) FEATURE:

    (A) NAME/KEY: Modified-site
    (B) LOCATION: 18
    (D) OTHER INFORMATION: /note= "A0.50;K0.50"

(ix) FEATURE:

    (A) NAME/KEY: Modified-site
    (B) LOCATION: 20
    (D) OTHER INFORMATION: /note= "D0.34;T0.33;E0.33"

(ix) FEATURE:

    (A) NAME/KEY: Modified-site
    (B) LOCATION: 22
    (D) OTHER INFORMATION: /note= "K0.50;V0.50"

(ix) FEATURE:

    (A) NAME/KEY: Modified-site
    (B) LOCATION: 23
    (D) OTHER INFORMATION: /note= "T0.34;A0.33;S0.33"

(ix) FEATURE:

    (A) NAME/KEY: Modified-site
    (B) LOCATION: 24
    (D) OTHER INFORMATION: /note= "S0.34;G0.33;N0.33"

(ix) FEATURE:

    (A) NAME/KEY: Modified-site
    (B) LOCATION: 27
    (D) OTHER INFORMATION: /note= "G0.50;N0.50"

(ix) FEATURE:

    (A) NAME/KEY: Modified-site
    (B) LOCATION: 28
    (D) OTHER INFORMATION: /note= "Q0.50;E0.50"

(ix) FEATURE:

    (A) NAME/KEY: Modified-site
    (B) LOCATION: 30

(D) OTHER INFORMATION: /note= "G0.50;A0.50"

(xi) SEQUENCE DESCRIPTION: SEQ ID NO:111:

```
Ser Ala Thr Ala Ile Phe Asp Thr Thr Thr Leu Asn Pro Thr Ile Ala
1               5                   10                  15

Gly Ala Gly Asp Val Lys Thr Ser Ala Glu Gly Gln Leu Gly Gly Gly
            20                  25                  30

Phe Phe Leu Leu Thr Arg Ile Leu Thr Ile Pro Gln Ser Leu Asp Gly
        35                  40                  45

Gly Thr Ala Lys Ser Lys Lys Phe Pro Ser Tyr Thr Ala Thr Tyr Gln
        50                  55                  60

                            Phe
                            65
```

(2) INFORMATION FOR SEQ ID NO:112:

(i) SEQUENCE CHARACTERISTICS:

(A) LENGTH: 66 amino acids
(B) TYPE: amino acid
(D) TOPOLOGY: linear

(ii) MOLECULE TYPE: peptide

(A) NAME/KEY: Modified-site
(B) LOCATION: 2
(C) OTHER INFORMATION: /note= A0.50;V0.50

(ix) FEATURE:

(A) NAME/KEY: Modified-site
(B) LOCATION: 3
(D) OTHER INFORMATION: /note= "E0.34;T0.33;K0.33"

(ix) FEATURE:

(A) NAME/KEY: Modified-site
(B) LOCATION: 4
(D) OTHER INFORMATION: /note= "A0.50;P0.50"

(ix) FEATURE:

(A) NAME/KEY: Modified-site
(B) LOCATION: 5
(D) OTHER INFORMATION: /note= "I0.50;V0.50"

(ix) FEATURE:

(A) NAME/KEY: Modified-site

EP 0 708 656 B1

(B) LOCATION: 6
(D) OTHER INFORMATION: /note= "L0.50;V0.50"

(ix) FEATURE:

(A) NAME/KEY: Modified-site
(B) LOCATION: 8
(D) OTHER INFORMATION: /note= "V0.50;I0.50"

(ix) FEATURE:

(A) NAME/KEY: Modified-site
(B) LOCATION: 16
(D) OTHER INFORMATION: /note= "A0.50;T0.50"

(ix) FEATURE:

(A) NAME/KEY: Modified-site
(B) LOCATION: 18
(D) OTHER INFORMATION: /note= "K0.50;C0.50"

(ix) FEATURE:

(A) NAME/KEY: Modified-site
(B) LOCATION: 20
(D) OTHER INFORMATION: /note= "S0.34;T0.33;A0.33"

(ix) FEATURE:

(A) NAME/KEY: Modified-site
(B) LOCATION: 22
(D) OTHER INFORMATION: /note= "V0.50;A0.50"

(ix) FEATURE:

(A) NAME/KEY: Modified-site
(B) LOCATION: 23
(D) OTHER INFORMATION: /note= "A0.34;S0.33;G0.33"

(ix) FEATURE:

(A) NAME/KEY: Modified-site
(B) LOCATION: 24
(D) OTHER INFORMATION: /note= "S0.50;A0.50"

(ix) FEATURE:

(A) NAME/KEY: Modified-site
(B) LOCATION: 25
(D) OTHER INFORMATION: /note= "G0.50;N0.50"

(ix) FEATURE:

(A) NAME/KEY: Modified-site
(B) LOCATION: 26
(D) OTHER INFORMATION: /note= "S0.50;T0.50"

(ix) FEATURE:

       (A) NAME/KEY: Modified-site
       (B) LOCATION: 27
       (D) OTHER INFORMATION: /note= "E0.50;D0.50"

   (ix) FEATURE:

       (A) NAME/KEY: Modified-site
       (B) LOCATION: 28
       (D) OTHER INFORMATION: /note= "N0.50;G0.50"

   (ix) FEATURE:

       (A) NAME/KEY: Modified-site
       (B) LOCATION: 29
       (D) OTHER INFORMATION: /note= "E0.34;D0.33;Q0.33"

   (ix) FEATURE:

       (A) NAME/KEY: Modified-site
       (B) LOCATION: 30
       (D) OTHER INFORMATION: /note= "L0.50;I0.50"

   (ix) FEATURE:

       (A) NAME/KEY: Modified-site
       (B) LOCATION: 31
       (D) OTHER INFORMATION: /note= "A0.50;S0.50"

   (xi) SEQUENCE DESCRIPTION: SEQ ID NO:112:


```
Leu Ala Glu Ala Ile Leu Asp Val Thr Thr Leu Asn Pro Thr Ile Ala


 1               5               10              15

Gly Lys Gly Ser Val Val Ala Ser Gly Ser Glu Asn Glu Leu Ala Gly
         20              25              30

Gly Phe Phe Leu Leu Thr Arg Ile Leu Thr Ile Pro Gln Ser Leu Asp
         35              40              45

Gly Gly Thr Ala Lys Ser Lys Lys Phe Pro Ser Tyr Thr Ala Thr Tyr
     50              55              60

Gln Phe
 65
```

    (2) INFORMATION FOR SEQ ID NO:113:

       (i) SEQUENCE CHARACTERISTICS:

           (A) LENGTH: 59 amino acids
           (B) TYPE: amino acid
           (D) TOPOLOGY: linear

       (ii) MOLECULE TYPE: peptide

(ix) FEATURE:

  (A) NAME/KEY: Modified-site
  (B) LOCATION: 1
  (D) OTHER INFORMATION: /note= "K0.50;T0.50"

(ix) FEATURE:

  (A) NAME/KEY: Modified-site
  (B) LOCATION: 6
  (D) OTHER INFORMATION: /note= "A0.34;K0.33;Q0.33"

(ix) FEATURE:

  (A) NAME/KEY: Modified-site
  (B) LOCATION: 8
  (D) OTHER INFORMATION: /note= "F0.50;L0.50"

(ix) FEATURE:

  (A) NAME/KEY: Modified-site
  (B) LOCATION: 11
  (D) OTHER INFORMATION: /note= "D0.34;A0.33;N0.33"

(ix) FEATURE:

  (A) NAME/KEY: Modified-site
  (B) LOCATION: 12
  (D) OTHER INFORMATION: /note= "I0.50;L0.50"

(ix) FEATURE:

  (A) NAME/KEY: Modified-site
  (B) LOCATION: 13
  (D) OTHER INFORMATION: /note= "T0.50;I0.50"

(ix) FEATURE:

  (A) NAME/KEY: Modified-site
  (B) LOCATION: 14
  (D) OTHER INFORMATION: /note= "A0.50;S0.50"

(ix) FEATURE:

  (A) NAME/KEY: Modified-site
  (B) LOCATION: 17
  (D) OTHER INFORMATION: /note= "E0.50;D0.50"

(ix) FEATURE:

  (A) NAME/KEY: Modified-site
  (B) LOCATION: 24
  (D) OTHER INFORMATION: /note= "D0.50;A0.50"

(xi) SEQUENCE DESCRIPTION: SEQ ID NO:113:

```
Lys Gly Tyr Val Gly Ala Glu Phe Pro Leu Asp Ile Thr Ala Gly Thr
1               5                   10                  15

Glu Ala Ala Thr Gly Thr Lys Asp Gly Gly Phe Phe Leu Leu Thr Arg
            20                  25                  30

Ile Leu Thr Ile Pro Gln Ser Leu Asp Gly Gly Thr Ala Lys Ser Lys
        35                  40                  45

Lys Phe Pro Ser Tyr Thr Ala Thr Tyr Gln Phe
        50                  55
```

(2) INFORMATION FOR SEQ ID NO:114:

(i) SEQUENCE CHARACTERISTICS:

(A) LENGTH: 59 amino acids
(B) TYPE: amino acid
(D) TOPOLOGY: linear

(ii) MOLECULE TYPE: peptide

(xi) SEQUENCE DESCRIPTION: SEQ ID NO:114:

```
Lys Gly Tyr Val Gly Ala Glu Phe Pro Leu Asp Leu Lys Ala Gly Thr
1               5                   10                  15

Asp Gly Val Thr Gly Thr Lys Asp Gly Gly Phe Phe Leu Leu Thr Arg
            20                  25                  30

Ile Leu Thr Ile Pro Gln Ser Leu Asp Gly Gly Thr Ala Lys Ser Lys
        35                  40                  45

Lys Phe Pro Ser Tyr Thr Ala Thr Tyr Gln Phe
        50                  55
```